# EUROPÄISCHE PATENTSCHRIFT

(11) **EP 1 578 817 B1**
(45) Veröffentlichungstag und Bekanntmachung des Hinweises auf die Patenterteilung: **06.09.2006**
(21) Anmeldenummer: 03789288.2
(22) Anmeldetag: 16.12.2003
(51) Int. Cl.: C08F 26/02, C08F 20/54, A61Q 5/00

(54) **WÄSSRIGE POLYMERDISPERSION**
AQUEOUS POLYMER DISPERSIONS
DISPERSION POLYMERE AQUEUSE

(30) Priorität: 20.12.2002 DE 10261197
(43) Veröffentlichungstag der Anmeldung: 28.09.2005
(73) Patentinhaber: BASF Aktiengesellschaft, 67056 Ludwigshafen (DE)
(72) Erfinder: CHRISSTOFFELS, Lysander, 67117 Limburgerhof (DE); HÖSSEL, Peter, 67105 Schifferstadt (DE); LEDUC, Marc, 67346 Speyer (DE); WOOD, Claudia, 69469 Weinheim (DE); ANGEL, Maximilian, 67105 Schifferstadt (DE); MATHAUER, Klemens, 69115 Heidelberg (DE); MUKHERJEE, Pulakesh, 68309 Mannheim (DE); GAUWEILER, Werner, 67363 Lustadt (DE); BÖLTER, Henning, 67319 Wattenheim (DE)
(86) Internationale Anmeldenummer: PCT/EP2003/014275
(87) Internationale Veröffentlichungsnummer: WO 2004/058831

(56) Entgegenhaltungen:
- WO-A-98/54234
- FR-A- 2 429 225
- US-B1- 6 231 876

## Beschreibung

Die vorliegende Erfindung betrifft wässrige Dispersionen vernetzter wasserlöslicher oder wasserquellbarer Polymerisate auf Basis vom monoethylenisch ungesättigten Monomeren, die ein quaterniertes oder quatemisierbares Stickstoffatom oder (Meth)acrylamidgruppen enthalten, durch radikalische Polymerisation in einer wässrigen Lösung in Gegenwart eines oder mehrere polymerer Dispergiermittel und eines oder mehrerer polymerer Fällungsagenzien, ein Verfahren zu ihrer Herstellung sowie die Verwendung in kosmetischen Formulierungen, insbesondere in haarkosmetischen Formulierungen.

Kationische Polymere werden als Konditioniermittel in kosmetischen Formulierungen eingesetzt. Anforderungen an Haarkonditioniermittel sind z.B. eine starke Reduktion der erforderlichen Kämmkraft im nassen wie auch im trockenen Haar, gute Entwirrung beim ersten Durchkämmen (engl. "Detangling") und gute Verträglichkeit mit weiteren Formulierungskomponenten. Außerdem verhindern kationische Polymere die elektrostatische Aufladung des Haares.

In Shampoos werden vor allem kationische Zellulose-Derivate (Polyquaternium-10) oder Guar-Gum Derivate eingesetzt. Allerdings beobachtet man bei diesen Verbindungen einen build-up Effekt, d.h. das Haar wird bei mehrfacher Anwendung mit dem Conditioner belegt und fühlt sich beschwert an.

Für die Konditionierung und Festigung von keratinösen Substanzen wie Haar, Nägel und Haut werden seit Jahren auch synthetische Polymere eingesetzt. Zudem werden synthetische Polymere in kosmetischen Formulierungen, die Pigmente oder kosmetisch wirksame Aktivkomponenten enthalten, als Verträglichkeitsvermittler zur Erreichung einer homogenen, stabilen Formulierung eingesetzt.

Zum Beispiel finden Copolymere aus Acrylamid und Dimethyldiallylammoniumchlorid (Polyquatemium 7) Verwendung. Diese haben allerdings den Nachteil hoher Restmonomerengehalte, da Acrylamid und Dimethyldiallylammoniumchlorid ungünstige Copolymerisationsparameter aufweisen.

Trotz der umfangreichen Bemühungen besteht nach wie vor Verbesserungsbedarf bei Polymeren zur Erzeugung elastischer Frisuren bei gleichzeitig starker Festigung auch bei hoher Luftfeuchtigkeit, guter Auswaschbarkeit und gutem Griff des Haares. Der Verbesserungsbedarf besteht ebenso bei Polymeren zur Erzeugung von gut kämmbarem, entwirrbarem Haar und zur Konditionierung von Haut und Haar in ihren sensorisch erfassbaren Eigenschaften wie Griff, Volumen, Handhabbarkeit usw. Ferner sind klare wässrige Zubereitungen dieser Polymere wünschenswert, die sich demnach durch eine gute Verträglichkeit mit anderen Formulierungesbestandteilen auszeichnen.

Weiterhin besteht Bedarf nach Polymeren, die als Konditioniermittel für kosmetische Zubereitungen geeignet sind und die mit einem hohen Feststoffgehalt hergestellt werden können. Von besonderem Interesse sind Polymere, die einen hohen Feststoffgehalt haben, eine geringe Viskosität aufweisen unter gleichzeitigem Erhalt der anwendungstechnischen Eigenschaften (wie beispielsweise Kämmbarkeit).

Aufgabe der vorliegenden Erfindung war es, ein Konditioniermittel für kosmetische Zubereitungen, insbesondere Shampoos zu finden, welche die genannten Nachteile nicht aufweist.

WO 02/15854 A1 beschreibt die Verwendung hydrophiler Pfropfcopolymere mit N-Vinylamind und/oder offenkettigen N-Vinylamideinheiten in kosmetischen Formulierungen. Keines der Beispiele offenbart Polymerisate, welche in Gegenwart von mindestens 2 Dispergiermitteln und einem Vernetzer hergestellt wurden.

EP 929 285 B1 beschriebt die Verwendung wasserlöslicher Copolymere als Wirkstoffe in kosmetischen Formulierungen. Diese Copolymere enthalten als charakteristische Strukturelemente Vinylcarbonsäureamid-Einheiten (z.B. N-Vinylformamid) sowie Imidazol- und imidazolimum-Monomere. Die beschriebenen Copolymere können in Gegenwart eines Vernetzers hergestellt werden. EP 929 285 B1 beschreibt keine Copolymere, welche in Gegenwart von polymeren Dispergiermitten hergestellt werden.

WO 98/54234 A1 beschreibt die Herstellung wasserlöslicher Polymerdispersionen aus Vinylamidmonomeren. Die dort beschriebenen Polymerdispersionen werden ohne Vernetzer herstellt.

WO 00/27893 A1 beschreibt wässrige Dispersionen von unvernetzten wasserlöslichen Polymeren enthaltend N-Vinylformamid und/oder N-Vinylacetamid.

WO 96/03969 A1 (EP 0 774 952) beschreibt die Herstellung und Verwendung von unvernetzten Vinylformamidhaltigen Polymerisate in haarkosmetische Zubereitungen. Die Herstellung diese Polymerisate erfolgt in Wasser als Lösungspolymerisat und als Fällungspolymerisat in organische Lösemittel.

US 4,713,236 beschreibt Polymere enthaltend Vinylamingruppen als Konditioniermittel in der Haarkosmetik. Die Polymerisate werden erhalten durch Hydrolyse der Vinylacetamid oder Vinylformamid enthaltende Polymere die als Lösungspolymerisat oder als Fällungspolymerisat hergestellt wurden. Vernetzte Polymere werden nicht beschrieben.

WO 98/04596 A1 (EP 915 915) beschreibt wasserlösliche Polymerisate enthaltend Vinylamin Einheiten und deren kosmetische Verwendung.

WO 02/34796 A1 beschreibt ein Verfahren zur Herstellung wässrige Dispersionen von wasserlöslichen unvernetzten Polymeren.

WO 02/083085 A1 beschreibt die Verwendung von Dispersionen von kationischen, anionischen oder nichtionischen Polymeren in einer wässrigen Salzlösung in der Kosmetik. Vernetzte Polymere werden nicht beschrieben.

DE 29 24 663 beschreibt ein Verfahren zur Herstellung von wässrigen Dispersionen aus wasserlöslichen Polymermassen. Die Dispersionen werden durch Polymerisation der Monomere in einer wässrigen Lösung eines wasserlöslichen Polymers erhalten, wobei unbedingt auf die Beachtung des Gleichgewichtsverhältnisses zwischen der Monomermenge und der Menge des wasserlöslichen Polymeren geachtet werden muss. Vernetzte Polymere werden nicht beschrieben.

Es wurde gefunden, dass die erfindungsgemäße Aufgabe gelöst wird durch wässrige Dispersionen, welche erhältlich sind durch radikalische Polymerisation von
a) mindestens einem N-Vinylhaltigen Monomer und/oder mindestens einem (Meth)acrylamidmonomer
b) mindestens einem polymeren Dispergiermittel
c) mindestens einem polymeren Fällungsagens
d) mindestens einem Vernetzer
e) gegebenenfalls weiteren Monomeren
f) gegebenenfalls mindestens einem Regler
wobei das Gewichtsverhältnis von b) zu c) im Bereich von 1:50 bis 1:0,02 liegt

Der Begriff (Meth)acrylamidmonomere bezeichnet sowohl acrylamidgruppen- als auch methacrylamidgruppen-beinhaltende Monomere.

In einer bevorzugten Ausführungsform der Erfindung ist als weitere Komponente g) ein Puffer während der Polymerisation vorhanden. Als Komponente g) geeignet ist hierbei mindestens eine Substanz, die in der Lage ist, den pH-Wert während der Polymerisation zu puffern, d.h. den pH-Wert während der Polymerisation bei 5,0 bis 10, insbesondere 6,0 bis 8,0, bevorzugt 6,5 bis 7,5 zu halten. Alternativ durch ständiges Messen des pH-Wertes und parallele Zugabe einer Säure oder Base der pH-Wert im bevorzugten pH Bereich gehalten werden.

Solche Puffereigenschaften findet man beispielsweise bei Salzen schwacher Säuren (siehe CD Römpp Chemie Lexikon-Version 1,0, Stuttgart/New York: Georg Thieme Verlag 1995).

Als Puffersubstanzen können zur pH-Wert Einstellung prinzipiell alle anorganischen oder organischen Basen verwendet werden, insbesondere solche Basen, die wasserlöslich sind.

In einer bevorzugten Ausführungsform ist die Puffersubstanz ein Salz einer Säure, ausgewählt aus der Gruppe, bestehend aus Kohlensäure, Borsäure, Essigsäure, Zitronensäure und Phosphorsäure, und/oder eine Base, ausgewählt aus der Gruppe, bestehend aus Alkali- und Erdalkalihydroxide, Ammoniak sowie primäre, sekundäre und tertiäre Amine.

Als Salze der oben genannten schwachen Säuren sind bevorzugt Alkali- und Erdalkalisalze zu nennen, besonders bevorzugt Natrium-, Kalium- oder Magnesiumsalze. Ganz besonders bevorzugte Puffersubstanzen sind Natriumacetat, Natriumcitrat, Natriumpyrophosphat, Kaliumpyrophosphat, Natriumdihydrogenphosphat, Dinatriumhyrogenphosphat, Natriumhydrogencarbonat und/oder Natriumborat. Zusätzlich können auch noch Salze von ungesättigten schwachen Carbonsäuren wie z.B. Acrylsäure oder Methacrylsäure mitverwendet werden.

Beispiele für Alkali- und Erdalkalihydroxide sind u.a. Natriumhydroxid, Kaliumhydroxid, Magnesiumhydroxid, Calciumhydroxid.

Beispiele für primäre, sekundäre und tertiäre Amine sind u.a. Ethylamin, Diethylamin, Triethylamin, n-Propylamin, Di-n-propylamin, Tri-n-propylamin, Ethylendiamin, Triethanolamin, Anilin.

Die neben den oben genannten Puffersubstanzen bevorzugt verwendeten, basisch wirkenden Reagenzien sind Natriumhydroxid, Kaliumhydroxid, Calciumhydroxid und/oder Ammoniak, besonders bevorzugt ist Natriumhydroxid.

Die genannten Puffersubstanzen können sowohl einzeln als auch in Mischungen eingesetzt werden. Die Puffersubstanzen können zur Einstellung des pH-Wertes gemeinsam oder auch jeweils einzeln zugegeben werden.

Die erfindungsgemäß erhältlichen Dispersionen weisen in einer bevorzugten Ausführungsform einen LD-Wert kleiner gleich 30 %, insbesondere kleiner gleich 20 %, bevorzugt kleiner gleich 10 %, insbesondere kleiner gleich 5 %.

Die Bestimmung des LD-Wertes (Lichtdurchlässigkeit) bei wässrigen Polymerdispersionen bei einem definierten Feststoffgehalt dient der Beurteilung der Klarheit oder Farbstärke. Die Lichtdurchlässigkeit der wässrigen Dispersionen wird im Vergleich zu reinem Wasser bei einer Küvettenlänge von 2,5 cm bei 600 nm gemessen. Das Spektrophotometer (Hach: Spektrophotometer DR/2000, Messart "Transmission") wird zuerst mit reinem Wasser auf 100% eingestellt werden. Danach spült man die Küvette mehrmals mit der Dispersion, füllt die Dispersion in die Küvette und liest die Lichtdurchlässigkeit in % ab.

In einer bevorzugten Ausführungsform werden die Komponenten a) bis d) in folgenden Mengen eingesetzt. Die Angaben der einzelnen Gewichtsprozent beziehen sich dabei immer auf die Gesamtsumme der Komponenten a) bis d), die als 100 % gesetzt wird. Sind weitere mögliche Komponenten vorhanden (z.B. Komponenten e) und f)), so berechnen sich die Gewichtsangaben dieser weiteren Komponenten bezogen auf die Summe von a) bis d), die als 100% gesetzt wird.

Komponente a) wird bevorzugt in einer Menge von 10 bis 90 Gew. %, insbesondere 20 bis 70 Gew. %, bevorzugt 30 bis 60 Gew. % eingesetzt.

Komponente b) wird bevorzugt in einer Menge von 1 bis 50 Gew. %, insbesondere 2 bis 30 Gew. %, bevorzugt 3 bis 20 Gew. % eingesetzt.

Komponente c) wird bevorzugt in einer Menge von 10 bis 90 Gew. %, insbesondere 20 bis 70 Gew. %, bevorzugt 30 bis 60 Gew. % eingesetzt.

Komponente d) wird bevorzugt in einer Menge von 0,01 bis 10 Gew. %, insbesondere 0,05 bis 5 Gew. %, bevorzugt 0,1 bis 1,5 Gew. % eingesetzt.

Besonders bevorzugt sind wässrige Dispersionen, die erhältlich sind durch radikalische Polymerisation von
10 bis 90 Gew. %, insbesondere 20 bis 70 Gew. %, bevorzugt 30 bis 60 Gew. % Komponente a)
1 bis 50 Gew. %, insbesondere 2 bis 30 Gew. %, bevorzugt 3 bis 20 Gew. % Komponente b)
10 bis 90 Gew. %, insbesondere 20 bis 70 Gew. %, bevorzugt 30 bis 60 Gew. % Komponente c)
0,01 bis 10 Gew. %, insbesondere 0,05 bis 5 Gew. %, bevorzugt 0,1 bis 1,5 Gew. % Komponente d)
mit der Maßgabe, dass sich die Summe von a) bis d) zu 100 % addiert.

Sind weitere Komponenten vorhanden, sind diese bevorzugt in folgenden Mengen vorhanden (bezogen auf die 100 % der Summe von a) bis d)
- 0 - 40 Gew.-%, bevorzugt 0 - 25 Gew.-%, besonders bevorzugt 0 - 15 Gew.-%. Komponente d)
- 0 - 5 Gew.-%, bevorzugt 0 - 2,5 Gew.-%, besonders bevorzugt 0 -1,5 Gew.-% Komponente f)
- 0 - 5 Gew.-%, bevorzugt 0 - 3 Gew.-% Komponente g)

Bei der Herstellung der wässrigen Dispersionen wird üblicherweise 400 bis 25 % Wasser, insbesondere 150 bis 50% Wasser, bezogen auf die Summe aller Komponenten (d.h. a) bis d) sowie gegebenenfalls e), f) und g) sowie weiterer möglicher Bestandteile eingesetzt.

Ein weiterer Gegenstand der Erfindung betrifft ein Verfahren zur Herstellung von wässrigen Dispersionen wobei
a) mindestens ein N-Vinylhaltiges Monomer und/oder mindestens ein (Meth)acrylamidmonomer
b) mindestens ein polymeres Dispergiermittel
c) mindestens ein polymeres Fällungsagens
d) mindestens ein Vernetzer
e) gegebenenfalls weitere Monomere
g) gegebenfalls einer Puffersubstanz
in Gegenwart mindestens eines Reglers umgesetzt werden und das Gewichtsverhältnis von b) zu c) im Bereich von 1:50 bis 1:0,02 liegt.

### Monomer a)

Als N-vinylhaltiges Monomer a) eignen sich beispielsweise N-Vinylamide und/oder N-Vinyllactame.

Als N-vinylhaltiges Monomer a) eignen sich beispielsweise N-Vinylamide der allgemeinen Formel (la) 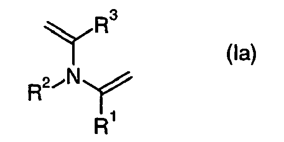 wobei R¹, R², R³ = H oder C₁- bis C₆-Alkyl bedeuten

Zur Herstellung der erfindungsgemäß verwendeten Polymerisate werden als offenkettige N-Vinylamidverbindung a) der allgemeinen Formel (Ia) beispielsweise folgende Monomere eingesetzt: N-Vinylformamid, N-Vinyl-N-methylformamid, N-Vinylacetamid, N-Vinyl-N-methylacetamid, N-Vinyl-N-ethylacetamid, N-Vinylpropionamid, N-Vinyl-N-methylpropionamid und N-Vinyl-butyramid.

Als N-vinylhaltige Monomere a) eignen sich weiterhin N-Vinyllactame der allgemeinen Formel (IIa) 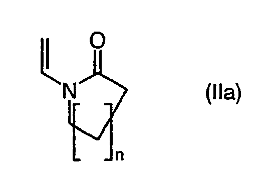 wobei n = 1,2,3

Beispiele für Monomere der allgemeinen Formel (IIa) sind N-Vinylpyrrolidon (n=1) und N-Vinylcaprolactam (n=3).

Als N-vinylhaltige Monomere a) eignen sich weiterhin N-Vinylpiperidon, N-Vinyloxazolidon und N-Vinyltriazol.

In einer bevorzugten Ausführungsform der Erfindung wird als Monomer a) ein N-Vinylamid, insbesonderen N-Vinylformamid, eingesetzt.

Als Monomere a) eignen sich auch (Meth)acrylamidmonomere der allgemeinen Formel X 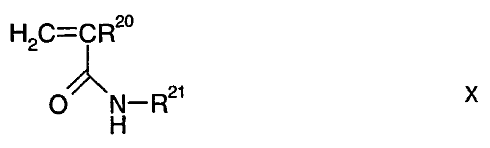 wobei R²⁰ Wasserstoff oder Methyl und R²¹ linear oder verzweigt C₁ bis C₆-Alkyl, linear oder verzweigt C₁-bis C₆-Alkyloxyalkyl bedeuten, wobei die Reste ein- oder mehrfach durch Hydroxyl- und/oder Carboxyl- und/oder Sulfonsäuregruppen substituiert sein können. Dabei bedeuten C₁- bis C₆-Alkyl Methyl, Ethyl, Propyl, 1-Methylethyl, Butyl, 1-Methylpropyl, 2-Methylpropyl, 1,1-Dimethylethyl, Pentyl, 1-Methylbutyl, 2-Methylbutyl, 3-Methylbutyl, 1,1-Dimethylpropyl, 1,2-Dimethylpropyl, 2,2-Dimethylpropyl, 1-Ethylpropyl, Hexyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 1,1,2-Trimethylpropyl, 1,2,2-Trimethylpropyl, 1-Ethyl-1-Methylpropyl, 1-Ethyl-2-Methylpropyl.

Bevorzugte (Meth)acrylamidmonomere der Formel X definiert durch die Kombinationen der Reste R²⁰ und R²¹ sind in Tabelle 1 wiedergegeben:

**Tabelle 2**

| **R**^{**20**} | **R**^{**21**} |
|---|---|
| H | H |
| H | CH(OH)COOH |
| H | C(CH₂OH)₃ |
| H | CH₂OH |
| H | CH₂OCH(CH₃)(C₂H₅) |
| H | CH(CH₃)₂ |
| H | CH₃ |
| H | C(CH₃)₂CH₂SO₃H bzw. C(CH₃)₂CH₂SO₃ |
| H | CH₂CHOHCH₃ |
| H | CH₂OCH₃ |
| H | C₂H₅ |
| CH₃ | H |
| CH₃ | CH₂OH |
| CH₃ | CH₃ |
| CH₃ | CH₂CHOHCH₃ |
| CH₃ | CH₂OCH₃ |
| CH₃ | C₂H₅ |
| CH₃ | CH(OH)COOH |
| CH₃ | C(CH₂OH)₃ |
| CH₃ | CH₂OCH(CH₃)(C₂H₅) |
| CH₃ | CH(CH₃)₂ |
| CH₃ | C(CH₃)₂CH₂SO₃H bzw. C(CH₃)₂CH₂SO₃⁻ |

Besonders bevorzugte (Meth)acrylamide als Monomere a) sind Acrylamid, 2-Acrylamidoglykolsäure, N-(Tris(hydroxymethyl)-methyl)acrylamid, N-Hydroxymethylacrylamid, N-Methylacrylamid, N-Isopropylacrylamid, 2-Acrylamido-2-methyl-1-propansulfonsäure Methacrylamid, N-Ethyl-methacrylamid, N-Hydroxymethylmethacrylamid, N-(2-hydroxypropyl)-methacrylamid, N-Methyl-methacrylamid, N-Isobutoxy-methylacrylamid, N-Methoxymethylmethacrylamid.
In einer ganz besonders bevorzugten Ausführungsform werden als Monomere a) Acrylamid, Methacrylamid, N-Hydroxymethylacrylamid, N-(2-hydroxypropyl)-methacrylamid, N-Hydroxymethylmethacrylamid und N-Isopropylacrylamid eingesetzt.

Selbstverständlich können auch Mischungen der jeweiligen Monomeren aus der Gruppe a) wie z.B. Mischungen aus N-Vinylformamid und N-Vinylacetamid oder Mischungen aus unterschiedlichen (Meth)acrylamidmonomeren oder Mischungen aus N-Vinylhaltigen Monomeren und (Meth)acrylamidmonomeren copolymerisiert werden.

### Polymeres Dispergiermittel b)

Die als Komponente b) in den wässrigen Dispersionen enthaltenen polymeren Dispergiermittel unterscheiden sich in der Zusammensetzung von den durch die radikalische Polymerisation der Monomere erhältlichen, wasserlöslichen Polymerisate. Das polymere Dispergiermittel b) dient als Dispergiermittel des entstehenden wasserlöslichen Polymerisates. Als polymeres Dispergiermittel b) eignen sich somit alle Verbindungen, die in der Lage sind das entstehende wasserlösliche Polymerisat zu dispergieren.

Die mittleren Molmassen der polymeren Dispergiermittel liegen vorzugsweise in dem Bereich von 500 bis 20000 000, insbesondere bei 1000 bis 90 0000, bevorzugt über 10 000 bis 700 000.

Die polymeren Dispergiermittel enthalten wenigstens eine funktionelle Gruppe ausgewählt aus Ether-, Hydroxyl-, Carboxyl-, Sulfon-, Sulfatester-, Amino-, Imino-, tert.-Amino-, und/oder quaternären Ammoniumgruppen. Beispiele für solche Verbindungen sind: Polyvinylacetat, Polyalkylenglykole, insbesonderen Polyethylenglykole, Polyvinylalkohol, Polyvinylpyridin, Polyethylenimin, Polyvinylimidazol, Polyvinylsuccinimid und Polydiallyldimethylammoniumchlorid, Polyvinylpyrrolidon, Polymerisaten, die mindestens 5 Gew.% an Vinylpyrrolidon-Einheiten enthalten, Polymerisaten, die mindestens 50 Gew.% an Vinylalkohol-Einheiten enthalten, Oligosacchariden, Polysacchariden, oxidativ, hydrolytisch oder enzymatisch abgebauten Polysacchariden, chemisch modifizierten Oligo- oder Polysacchariden wie beispielsweise Carboxymethylcellulose, wasserlösliche Stärke und Stärkederivate, Stärkeester, Stärkexanthanogenate, Stärkeacetate, Dextran, und deren Mischungen.

Werden als polymere Dispergiermittel Polyalkylenglykole, insbesondere Polyethylenglykole und Polypropylenglykole, eingesetzt, hat es sich als vorteilhaft erwiesen Verbindungen mit einem Molekulargewicht von über 10 000 einzusetzen.

Geeignet als polymeres Dispergiermittel b) sind Polymerisate, die mindestens 50 Gew.-% an Vinylalkohol-Einheiten enthalten. Bevorzugt enthalten diese Polymerisate mindestens 70 Gew.-%, ganz besonders bevorzugt 80 Gew.-% Polyvinylalkoholeinheiten. Solche Polymerisate werden überlicherweise durch Polymerisation eines Vinylesters und anschließender zumindest teilweiser Alkoholyse, Aminolyse oder Hydrolyse hergestellt. Bevorzugt sind Vinylester linearer und verzweigter C₁-C₁₂-Carbonsäuren, ganz besonders bevorzugt ist Vinylacetat. Die Vinylester können selbstverständlich auch im Gemisch eingesetzt werden.

Als Comonomere des Vinylesters kommen beispielsweise N-Vinylcaprolactam, N-Vinylpyrrolidon, N-Vinylimidazol, N-Vinyl-2-methylimidazol, N-Vinyl-4-methylimidazol, 3-Methyl-1-vinylimidazoliumchlorid, 3-Methyl-1-vinylimidazoliummethylsulfat, Diallylammoniumchlorid, Styrol, Alkylstyrole in Frage.

Weitere geeignete Comonomere sind beispielsweise monoethylenisch ungesättigten C₃-C₆₋Carbonsäuren wie z.B. Acrylsäure, Methacrylsäure, Crotonsäure, Fumarsäure, sowie deren Ester, Amide und Nitrile wie z.B. Acrylsäuremethylester, Acrylsäureethylester, Methacrylsäuremethylester, Methacrylsäureethylester, Methacrylsäurestearylester, Hydroxyethylacrylat, Hydroxypropylacrylat, Hydroxybutylacrylat, Hydroxyethylmethacrylat, Hydroxypropylmethacrylat, Hydroxyisobutylacrylat, Hydroxyisobutylmethacrylat, Maleinsäuremonomethylester, Maleinsäuredimethylester, Maleinsäuremonoethylester, Maleinsäurediethylester, 2-Ethylhexylacrylat, 2-Ethylhexylmethacrylat, Maleinsäureanhydrid sowie dessen Halbester, Alkylenglykol(meth)acrylate, Acrylamid, Methacrylamid, N-Dimethylacrylamid, N-tert.-butylacrylamid, Acrylnitril, Methacrylnitril, Vinylether wie z.B. Methyl-, Ethyl-, Butyl oder Dodecylvinylether, kationische Monomere wie Dialkylaminoalkyl(meth)acrylate und Dialkylaminoalkyl(meth)acrylamide wie Dimethylaminoethylacrylat, Diethylaminoethylacrylat, Diethylaminoethylmethacrylat, sowie die Salze der zuletzt genannten Monomeren mit Carbonsäuren oder Mineralsäuren sowie die quarternierten Produkte.

Bevorzugte polymere Dispergiermittel sind Polymerisate, die durch Homopolymerisation von Vinylacetat und anschließender zumindest teilweiser Hydrolyse, Alkoholyse oder Aminolyse hergestellt werden.

Die Herstellung dieser polymeren Dispergiermittel erfolgt nach bekannten Verfahren, zum Beispiel der Lösungs-, Fällungs-, Suspensions- oder Emulsionspolymerisation unter Verwendung von Verbindungen, die unter den Polymerisationsbedingungen Radikale bilden. Die Polymerisationstemperaturen liegen üblicherweise in dem Bereich von 30 bis 200°C, vorzugsweise 40 bis 110°C. Geeignete Initiatoren sind beispielsweise Azo- und Peroxyverbindungen sowie die üblichen Redoxinitiatorsysteme, wie Kombinationen aus Wasserstoffperoxid und reduzierend wirkenden Verbindungen, zum Beispiel Natriumsulfit, Natriumbisulfit, Natriumformaldehydsulfoxilat und Hydrazin. Diese Systeme können gegebenenfalls zusätzlich noch geringe Mengen eines Schwermetallsalzes enthalten.

Zur Herstellung dieser polymeren Dispergiermittel werden die Estergruppen der ursprünglichen Monomere und gegebenenfalls weiterer Monomere nach der Polymerisation durch Hydrolyse, Alkoholyse oder Aminolyse zumindest teilweise gespalten. Im nachfolgenden wird dieser Verfahrensschritt allgemein als Verseifung bezeichnet. Die Verseifung erfolgt in an sich bekannter Weise durch Zugabe einer Base oder Säure, bevorzugt durch Zugabe einer Natrium- oder Kaliumhydroxidlösung in Wasser und/oder Alkohol. Besonders bevorzugt werden methanolische Natrium- oder Kaliumhydroxidlösungen eingesetzt, Die Verseifung wird bei Temperaturen im Bereich von 10 bis 80°C, bevorzugt im Bereich von 20 bis 60°C, durchgeführt. Der Verseifungsgrad hängt ab von der Menge der eingesetzten Base bzw. Säure, von der Verseifungstemperatur, der Verseifungszeit und dem Wassergehalt der Lösung.

Besonders bevorzugte polymere Dispergiermittel sind Polymerisate, die durch Homopolymerisation von Vinylacetat und anschließender zumindest teilweiser Verseifung hergestellt werden. Solche Polyvinylalkoholeinheiten enthaltenden Polymere sind unter dem Namen Mowiol® erhältlich.

Weitere besonders bevorzugte polymere Dispergiermittel b) sind Polymerisate, die mindestens 5 Gew.-% an Vinylpyrrolidon-Einheiten enthalten. Bevorzugt enthalten diese Polymerisate einen Vinylpyrrolidon-Anteil von mindestens 10 Gew.-%, ganz besonders bevorzugt von mindestens 30 Gew.-%, insbesondere mindestens 50 Gew.-%.

Als Comonomere des Vinylpyrrolidons zur Synthese dieser Polymerisate kommen beispielsweise N-Vinylacetat, N-Vinylcaprolactam, N-Vinylimidazol, N-Vinyl-2-methylimidazol, N-Vinyl-4-methylimidazol, 3-Methyl-1-vinylimidazoliumchlorid, 3-Methyl-1-vinylimidazoliummethylsulfat, Diallylammoniumchlorid, Styrol, Alkylstyrole in Frage. Weitere geeignete Comonomere sind beispielsweise monoethylenisch ungesättigten C₃-C₆-Carbonsäuren wie z.B. Acrylsäure, Methacrylsäure, Crotonsäure, Fumarsäure, sowie deren Ester, Amide und Nitrile wie z.B. Acrylsäuremethylester, Acrylsäureethylester, Methacrylsäuremethylester, Methacrylsäureethylester, Methacrylsäurestearylester, Hydroxyethylacrylat, Hydroxypropylacrylat, Hydroxybutylacrylat, Hydroxyethylmethacrylat, Hydroxypropylmethacrylat, Hydroxyisobutylacrylat, Hydroxyisobutylmethacrylat, Maleinsäuremonomethylester, Maleinsäuredimethylester, Maleinsäuremonoethylester, Maleinsäurediethylester, 2-Ethylhexylacrylat, 2-Ethylhexylmethacrylat, Maleinsäureanhydrid sowie dessen Halbester, Alkylenglykol(meth)acrylate, Acrylamid, Methacrylamid, N-Dimethylacrylamid, N-tert.-butylacrylamid, Acrylnitril, Methacrylnitril, Vinylether wie z.B. Methyl-, Ethyl-, Butyl oder Dodecylvinylether, kationische Monomere wie Dialkylaminoalkyl(meth)acrylate und Dialkylaminoalkyl(meth)acrylamide wie Dimethylaminoethylacrylat, Diethylaminoethylacrylat, Diethylaminoethylmethacrylat, sowie die Salze der zuletzt genannten Monomeren mit Carbonsäuren oder Mineralsäuren sowie die quarternierten Produkte.

Besonders bevorzugt als Comonomere des Vinylpyrrolidons sind Vinylacetat, N-Vinylcaprolactam, N-Vinylimidazol, 3-Methyl-1-vinylimidazoliumchlorid, 3-Methyl-1-vinylimidazoliummethylsulfat und/oder Styrol.

Die Herstellung dieser polymeren Dispergiermittel erfolgt nach bekannten Verfahren, zum Beispiel der Lösungs-, Fällungs-, Suspensions- oder Emulsionspolymerisation unter Verwendung von Verbindungen, die unter den Polymerisationsbedingungen Radikale bilden. Die Polymerisationstemperaturen liegen üblicherweise in dem Bereich von 30 bis 200°C, vorzugsweise 40 bis 110°C. Geeignete Initiatoren sind beispielsweise Azo- und Peroxyverbindungen sowie die üblichen Redoxinitiatorsysteme, wie Kombinationen aus Wasserstoffperoxid und reduzierend wirkenden Verbindungen, zum Beispiel Natriumsulfit, Natriumbisulfit, Natriumformaldehydsulfoxilat und Hydrazin. Diese Systeme können gegebenenfalls zusätzlich noch geringe Mengen eines Schwermetallsalzes enthalten.

Besonders bevorzugt als polymeres Dispergiermittel b) ist Polyvinylpyrrolidon.

In einer bevorzugten Ausführungsform wird als polymeres Dispergiermittel b) Polyvinylpyrrolidion mit einem Molekulargewicht von 1 000 bis 10 x 10⁶, inbesondere 10 000 bis 5 x 10⁶, bevorzugt 10 000 bis 7 x 10 ⁵eingesetzt.

Alle genannten polymeren Dispergiermittel können auch in beliebigen Mischungen untereinander eingesetzt werden. Besonders bevorzugt als polymeres Dispergiermittel ist eine Mischung aus Polymerisaten, die mindestens 5 Gew.% an Vinylpyrrolidon-Einheiten und Polyvinylpyrrolidon enthalten.

Als polymere Dispergiermittel können auch natürliche Substanzen, die Saccharid-Strukturen enthalten, eingesetzt werden. Solche natürlichen Substanzen sind beispielsweise Saccharide pflanzlicher oder tierischer Herkunft oder Produkte, die durch Metabolisierung durch Mikroorganismen entstanden sind, sowie deren Abbauprodukte. Geeignete Verbindungen sind beispielsweise Oligosaccharide, Polysaccharide, oxidativ, enzymatisch oder hydrolytisch abgebaute Polysaccharide, oxidativ hydrolytisch abgebaute oder oxidativ enzymatisch abgebaute Polysaccharide, chemisch modifizierte Oligo- oder Polysaccharide und Mischungen davon.

Bevorzugte Produkte sind die in US 5,334,287 auf Spalte 4, Zeile 20 bis Spalte 5, Zeile 45 genannten Verbindungen.

### Polymeres Fällungsagens c)

Das als Komponente c) in den wässrigen Dispersionen enthaltene Fällungsagens unterscheidet sich in der Zusammensetzung von den durch die radikalische Polymerisation der Monomere erhältlichen, wasserlöslichen Polymerisate. Das polymere Fällungsagenz c) verringert die Solvatisierungseigenschaften der wässrigen Phase und es kommt zu einer Verdrängung der entstehenden wasserlöslichen Polymerisate aus der wässrigen Phase in die disperse Phase. Das polymere Fällungsagenz c) ist somit mit dem wasserlöslichen Polymerisat unverträglich.

Als polymere Fällungsagenz c) eignen sich somit alle Verbindungen, welche die Solvatisierungseigenschaften der wässrigen Phase verringern und zu einer Verdrängung der entstehenden wasserlöslichen Polymerisate aus der wässrigen Phase in die disperse Phase führen.

Unter wasserlöslich sind Verbindungen zu verstehen, die bei 25°C bis zu einer Konzentration von 20 Gew.-% in Wasser, bevorzugt bis zu einer Konzentration von 50 Gew.-% in Wasser und besonders bevorzugt bis zu einer Konzentration von 70 Gew.-% in Wasser klar löslich sind.

Das polymere Fällungsagens c) steht nicht maßgeblich als Reaktionspartner zur Verfügung, d.h. es kommt nicht in maßgeblichen Anteilen zu einer kovalenten Bindung zwischen dem polymeren Fällungsagens c) und den übrigen Monomeren. Dem Fachmann sind übliche Reaktionsbedingungen bekannt, um dies zu erzielen. Beispielsweise seien genannt die Temperaturwahl bei der Polymerisation. Diese wird bevorzugt unter 100°C, insbesondere unter 80°C, bevorzugt unter 70°C gewählt. Eine weitere mögliche Reaktionsbedingung ist die Wahl des Wasseranteils in der Polymerisationsreaktion. Vorteilhaft sind Wassergehalte von ≥ 20 Gew.-%, insbesondere ≥ 30 %, bevorzugt ≥ 40 %, Die Angabe des Wassergehalt erfolgt bezogen auf die Summe aller Bestandteile (d.h. auf die Summe von a) bis d) sowie gegebenenfalls e), f) und g) sowie weiteren Bestandteilen), die als 100 gesetzt wird.

Die mittlere Molmasse des polymeren Fällungsagens liegt vorzugsweise in dem Bereich von 300 bis 100 000, insbesondere bei 1000 bis 30 000, bevorzugt bei 1000 bis 10 000.

Als polymeres Fällungsagenz c) eignen sich insbesondere wasserlösliche polyetherhaltige Verbindungen. Hierbei können sowohl Polyalkylenoxide auf Basis von Ethylenoxid, Propylenoxid, Butylenoxid und weiteren Alkylenoxiden als auch Polyglycerin verwendet werden. Dabei kann es sich bei den Struktureinheiten sowohl um Homopolymere als auch um statistische Copolymere und Blockcopolymere handeln.

Bevorzugt werden als polymere Fällungsagentien Verbindungen der folgenden Formel (Ib) eingesetzt. 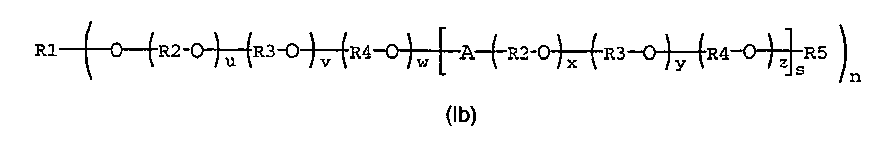 in der die Variablen unabhängig voneinander folgende Bedeutung haben:
R¹ Wasserstoff, C₁-C₂₄-Alkyl, R⁶-C(=O)-, R⁶-NH-C(=O)-, Polyalkoholrest;
R⁵ Wasserstoff, C₁-C₂₄-Alkyl, R⁶-C(=O)-, R⁶-NH-C(=O)-;
R² bis R⁴-(CH₂)₂-, -(CH₂)₃-, -(CH₂)₄-, -CH₂-CH(R⁶)-, -CH₂-CHOR⁷-CH₂-;
R⁶ C₁-C₂₄-Alkyl;
R⁷ Wasserstoff, C₁-C₂₄-Alkyl, R⁶-C(=O)-, R⁶-NH-C(=O)-;
A-C(=O)-O, -C(=O)-B-C(=O)-O, -CH₂-CH(-OH)-B-CH(-OH)-CH₂-O, -C(=O)-NH-B-NH-C(=O)-O; 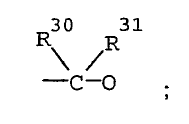
B-(CH₂)_{f}-, Arylen, ggf. substituiert;
R³⁰, R³¹ Wasserstoff, C₁-C₂₄-Alkyl, C₁-C₂₄-Hydroxyalkyl, Benzyl oder Phenyl;
n 1 wenn R¹ kein Polyakoholrest ist oder
n 1 bis 1000 wenn R¹ ein Polyakoholrest ist
s = 0 bis 1000; t =1 bis 12; u =1 bis 5000;v = 0 bis 5000; w =0 bis 5000;x= 0 bis 5000;
y= 0 bis 5000; z= 0 bis 5000.

Als Alkylreste für R⁶ und R³⁰ und R³¹ seien verzweigte oder unverzweigte C₁-C₂₄-Alkylketten, bevorzugt Methyl, Ethyl, n-Propyl, 1-Methylethyl, n-Butyl, 1-Methylpropyl-, 2-Methylpropyl, 1,1-Dimethylethyl, n-Pentyl, 1-Methylbutyl, 2-Methylbutyl, 3-Methylbutyl, 2,2-Dimethylpropyl, 1-Ethylpropyl, n-Hexyl, 1,1-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl, n-Heptyl, 2-Ethylhexyl, n-Octyl, n-Nonyl, n-Decyl, n-Undecyl, n-Dodecyl, n-Tridecyl, n-Tetradecyl, n-Pentadecyl, n-Hexadecyl, n-Heptadecyl, n-0ctadecyl, n-Nonadecyl oder n-Eicosyl genannt.

Als bevorzugte Vertreter der oben genannten Alkylreste seien verzweigte oder unverzweigte C₁₋C₁₂-, besonders bevorzugt C₁-C₆-Alkylketten genannt.

Bevorzugt als polymere Fällungsagens c) sind Polyalkylenglykole, wie beispielsweise Polyethylenglykole und Polypropylenglykole. Besonders bevorzugt sind Polyethylenglykole.

Als polymeres Fällungsagenz c) können auch Silikonderivate eingesetzt werden. Geeignete Silikonderivate sind die unter dem INCI Namen Dimethicone Copolyole oder Silikontenside bekannten Verbindungen wie zum Beispiel die unter den Markennamen Abil® (der Fa. T. Goldschmidt), Alkasil® (der Fa. Rhöne-Poulenc), Silicone Polyol Copolymer® (der Fa Genesee), Belsil® (der Fa. Wacker), Silwet® (der Fa. Witco, Greenwich, CT, USA) oder Dow Corning (der Fa. Dow Corning) erhältlich. Diese beinhalten Verbindungen mit den CAS-Nummern 64365-23-7; 68937-54-2; 68938-54-5; 68937-55-3.

Bevorzugte Vertreter solcher polyetherhaltigen Silikonderivaten sind solche, die folgende Strukturelemente enthalten: 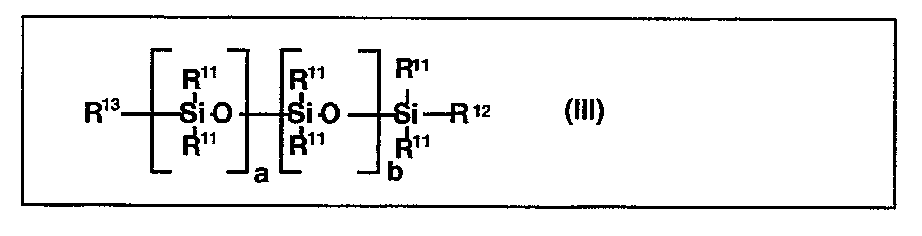 wobei: 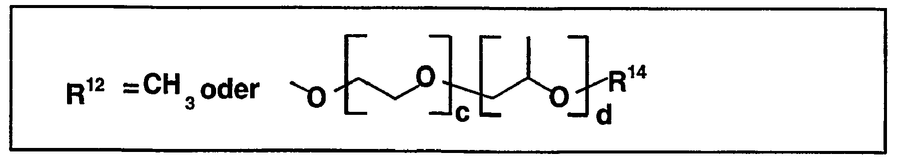 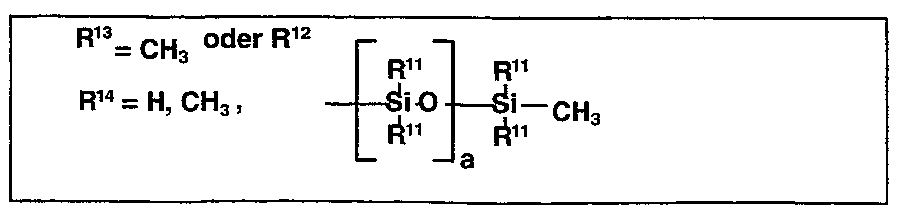 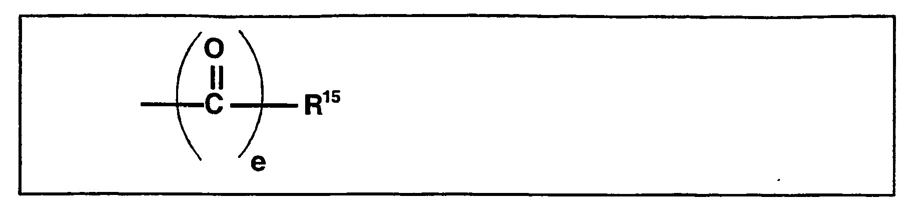 R¹⁵ ein organischer Rest aus 1 bis 40 Kohlenstoffatomen, der Amino-, Carbonsäure- oder Sulfonatgruppen enthalten kann oder für den Fall e=0, auch das Anion einer anorganischen Säure bedeutet,
und wobei die Reste R¹¹ identisch oder unterschiedlich sein können, und entweder aus der Gruppe der aliphatischen Kohlenwasserstoffe mit 1 bis 20 Kohlenstoffatomen stammen, cyclische aliphatische Kohlenwasserstoffe mit 3 bis 20 C-Atomen sind, aromatischer Natur oder gleich R¹⁶ sind, wobei: 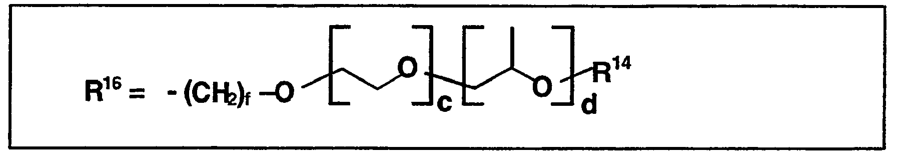 mit der Maßgabe, daß mindestens einer der Reste R¹¹, R¹² oder R¹³ ein polyalkylenoxidhaltiger Rest nach obengenannter Definition ist,
und f eine ganze Zahl von 1 bis 6 ist,
a und b ganze Zahlen derart sind, daß das Molekulargewicht des Polysiloxan-Blocks zwischen 300 und 30000 liegt,
c und d ganze Zahlen zwischen 0 und 50 sein können mit der Maßgabe, daß die Summe aus c und d größer als 0 ist, und e 0 oder 1 ist.

Bevorzugte Reste R¹² und R¹⁶ sind solche, bei denen die Summe aus c+d zwischen 5 und 30 beträgt.

Bevorzugt werden die Gruppen R¹¹ aus der folgenden Gruppe ausgewählt: Methyl, Ethyl, Propyl, Butyl, Isobutyl, Pentyl, Isopentyl, Hexyl, Octyl, Decyl, Dodecyl und Octadecyl, cycloaliphatische Reste, speziell Cyclohexyl, aromatische Gruppen, speziell Phenyl oder Naphthyl, gemischt aromatisch-aliphatische Reste wie Benzyl oder Phenylethyl sowie Tolyl und Xylyl und R¹⁶.

Besonders geeignete Reste R¹⁴ sind solche, bei denen im Falle von R¹⁴ = -(CO)ₑ-R¹⁵ R¹⁵ ein beliebiger Alkyl-, Cycloalkyl oder Arylrest bedeutet, der zwischen 1 und 40 C-Atomen besitzt und der weitere ionogene Gruppen wie NH₂, COOH, SO₃H tragen kann.

Bevorzugte anorganische Reste R¹⁵ sind, für den Fall e=0, Phosphat und Sulfat.

Besonders bevorzugte polyetherhaltige Silikonderivate sind solche der allgemeinen Struktur:

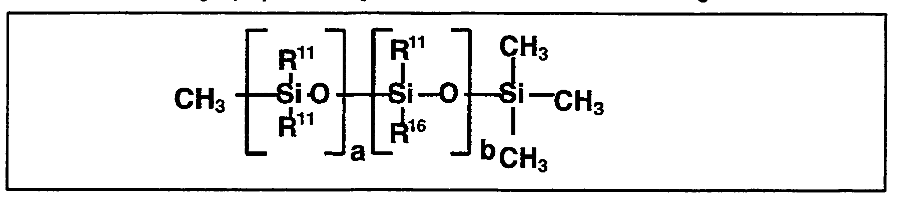

### Verhältnis b) zu c)

Das Gewichtsverhältnis der Summe der polymeren Dispergiermittel b) zur Summe der polymeren Fällungsagentien c) liegt im Bereich von 1:50 bis 1:0.02, insbesondere von 1:20 bis 1: 0,05, besonders bevorzugt im Bereich von 1:10 bis 1: 0.1, insbesondere 1:10 bis 1:0.5, bevorzugt 1:10 bis 1:1.

In einer bevorzugten Ausführungsform der Erfindung werden als polymere Dispergiermittel b) Polymerisate, die mindestens 5 Gew.% an Vinylpyrrolidon-Einheiten enthalten und/oder Polyvinylpyrrolidon eingesetzt und als polymeres Fällungsagens c) Polyethylenglykole eingesetzt.

In einer bevorzugten Ausführungsform der Erfindung wird das Gewichtsverhältnis der Mengen an polymerem Dispergierungsmittel b) und polymeres Fällungsagens c) zur Summe der restlichen Monomeren so gewählt, das das Verhältnis im Bereich von 10 : 1 bis 1 : 0,1 , insbesondere im Bereich von 5 : 1 bis 1 : 0,5 liegt. Die Summe der restlichen Monomere ergibt sich aus der Summe von a) und d) sowie gegebenenfalls e) und f). Sie entspricht der Summe aller weiteren Bestandteile ohne zugefügtes Wasser.

### Vernetzer d)

Monomere d), die eine vernetzende Funktion besitzen, sind Verbindungen mit mindestens 2 ethylenisch ungesättigten, nichtkonjugierten Doppelbindungen im Molekül.

Geeignete Vernetzer d) sind zum Beispiel Acrylester, Methacrylester, Allylether oder Vinylether von mindestens zweiwertigen Alkoholen. Die OH-Gruppen der zugrundeliegenden Alkohole können dabei ganz oder teilweise verethert oder verestert sein; die Vernetzer enthalten aber mindestens zwei ethylenisch ungesättigte Gruppen.

Beispiele für die zugrundeliegenden Alkohole sind zweiwertige Alkohole wie 1,2-Ethandiol, 1,2-Propandiol, 1,3-Propandiol, 1,2-Butandiol, 1,3-Butandiol, 2,3-Butandiol, 1,4-Butandiol, But-2-en-1,4-diol, 1,2-Pentandiol, 1,5-Pentandiol, 1,2-Hexandiol, 1,6-Hexandiol, 1,10-Decandiol, 1,2-Dodecandiol, 1,12-Dodecandiol, Neopentylglykol, 3-Methylpentan-1,5-diol, 2,5-Dimethyl-1,3-hexandiol, 2,2,4-Trimethyl--1,3-pentandiol, 1,2-Cyclohexandiol, 1,4-Cyclohexandiol, 1,4-Bis(hydroxymethyl)cyclohexan, Hydroxypivalinsäure-neopentylglykolmonoester, 2,2-Bis(4-hydroxyphenyl)-propan, 2,2-Bis[4-(2-hydroxypropyl)phenyl]propan, Diethylenglykol, Triethylenglykol, Tetraethylenglykol, Dipropylenglykol, Tripropylenglykol, Tetrapropylenglykol, 3-Thiopentan-1,5-diol, sowie Polyethylenglykole, Polypropylenglykole und Polytetrahydrofurane mit Molekulargewichten von jeweils 200 bis 10000. Außer den Homopolymerisaten des Ethylenoxids bzw. Propylenoxids können auch Blockcopolymerisate aus Ethylenoxid oder Propylenoxid oder Copolymerisate, die Ethylenoxid- und Propylenoxid-Gruppen eingebaut enthalten, eingesetzt werden. Beispiele für zugrundeliegende Alkohole mit mehr als zwei OH-Gruppen sind Trimethylolpropan, Glycerin, Pentaerythrit, 1,2,5-Pentantriol, 1,2,6-Hexantriol, Triethoxycyanursäure, Sorbitan, Zucker wie Saccharose, Glucose, Mannose. Selbstverständlich können die mehrwertigen Alkohole auch nach Umsetzung mit Ethylenoxid oder Propylenoxid als die entsprechenden Ethoxylate bzw. Propoxylate eingesetzt werden. Die mehrwertigen Alkohole können auch zunächst durch Umsetzung mit Epichlorhydrin in die entsprechenden Glycidylether überführt werden.

Weitere geeignete Vernetzer sind die Vinylester oder die Ester einwertiger, ungesättigter Alkohole mit ethylenisch ungesättigten C₃- bis C₆-Carbonsäuren, beispielsweise Acrylsäure, Methacrylsäure, Itaconsäure, Maleinsäure oder Furnarsäure. Beispiele für solche Alkohole sind Allylalkohol, 1-Buten-3-ol, 5-Hexen-1-ol, 1-Octen-3-ol, 9-Decen-1-ol, Dicyclopentenylalkohol, 10-Undecen-1-ol, Zimtalkohol, Citronellol, Crotylalkohol oder cis-9-Octadecen-1-ol. Man kann aber auch die einwertigen, ungesättigten Alkohole mit mehrwertigen Carbonsäuren verestern, beispielsweise Malonsäure, Weinsäure, Trimellitsäure, Phthalsäure, Terephthalsäure, Citronensäure oder Bernsteinsäure.

Weitere geeignete Vernetzer sind Ester ungesättigter Carbonsäuren mit den oben beschriebenen mehrwertigen Alkoholen, beispielsweise der Ölsäure, Crotonsäure, Zimtsäure oder 10-Undecensäure.

Geeignet als Monomere d) sind außerdem geradkettige oder verzweigte, lineare oder cyclische, aliphatische oder aromatische Kohlenwasserstoffe, die über mindestens zwei Doppelbindungen verfügen, die bei aliphatischen Kohlenwasserstoffen nicht konjugiert sein dürfen, z.B. Divinylbenzol, Divinyltoluol, 1,7-Octadien, 1,9-Decadien, 4-Vinyl-1-cyclohexen, Trivinylcyclohexan oder Polybutadiene mit Molekulargewichten von 200 bis 20000.

Als Vernetzer sind ferner geeignet die Acrylsäureamide, Methacrylsäureamide und N-Allylamine von mindestens zweiwertigen Aminen. Solche Amine sind zum Beispiel 1,2-Diaminomethan, 1,2-Diaminoethan, 1,3-Diaminopropan, 1,4-Diaminobutan, 1,6-Diaminohexan, 1,12-Dodecandiamin, Piperazin, Diethylentriamin oder Isophorondiamin. Ebenfalls geeignet sind die Amide aus Allylamin und ungesättigten Carbonsäuren wie Acrylsäure, Methacrylsäure, Itaconsäure, Maleinsäure, oder mindestens zweiwertigen Carbonsäuren, wie sie oben beschrieben wurden.

Ferner sind Triallylamin und Triallylmonoalkylammoniumsalze, z.B. Triallylmethylammoniumchlorid oder -methylsulfat, als Vernetzer geeignet.

Geeignet sind auch N-Vinyl-Verbindungen von Harnstoffderivaten, mindestens zweiwertigen Amiden, Cyanuraten oder Urethanen, beispielsweise von Harnstoff, Ethylenharnstoff, Propylenhamstoff oder Weinsäurediamid, z.B. N,N'-Divinylethylenharnstoff oder N,N'-Divinylpropylenharnstoff.

Weitere geeignete Vernetzer sind Divinyldioxan, Tetraallylsilan oder Tetravinylsilan.

Selbstverständlich können auch Mischungen der vorgenannten Verbindungen eingesetzt werden. Vorzugsweise werden solche Vernetzer eingesetzt, die in der Monomermischung löslich sind.

Besonders bevorzugt eingesetzte Vernetzer sind beispielsweise Methylenbisacrylamid, Triallylamin und Triallylalkylammoniumsalze, Divinylimidazol, Pentaerythrittriallylether, N,N'-Divinylethylenharnstoff, Umsetzungsprodukte mehrwertiger Alkohole mit Acrylsäure oder Methacrylsäure, Methacrylsäureester und Acrylsäureester von Polyalkylenoxiden oder mehrwertigen Alkoholen, die mit Ethylenoxid und/oder Propylenoxid und/oder Epichlorhydrin umgesetzt worden sind.

Ganz besonders bevorzugt als Vernetzer sind Pentaerythrittriallylether, Methylenbisacrylamid, N,N'-Divinylethylenharnstoff, Triallylamin und Triallylmonoalkylammoniumsalze, und Acrylsäureester von Glykol, Butandiol, Trimethylolpropan oder Glycerin oder Acrylsäureester von mit Ethylenoxid und/oder Epichlorhydrin umgesetzten Glykol, Butandiol, Trimethylolpropan oder Glycerin.

### Weitere Monomere e)

In einer bevorzugten Ausführungsform der vorliegenden Erfindung können weitere Monomere bei der radikalischen Polymerisationsreaktion vorhanden sein.

Geeignete von a) verschiedene weitere Monomere e) sind N-Vinyllactame, z.B. N-Vinylpiperidon, N-Vinylpyrrolidon und N-Vinylcaprolactam, N-Vinylacetamid, N-Methyl-N-vinylacetamid, N-Vinylformamid, von a) verschiedene (Meth)acrylamidmonomere wie Acrylamid, Methacrylamid, N,N-Dimethylacrylamid, N-Methylolmethacrylamid, N-Vinyloxazolidon, N-Vinyltriazol, Hydroxyalkyl(meth)acrylate, z.B. Hydroxyethyl(meth)acrylat und Hydroxypropyl(meth)-acrylate, oder Alkylethylenglykol(meth)-acrylate mit 1 bis 50 Ethylenglykoleinheiten im Molekül. Besonders bevorzugt werden als Monomere e) N-Vinyllactame eingesetzt. Ganz besonders bevorzugt ist N-Vinylpyrrolidon.

Außerdem eignen sich von Monomer a) verschiedene N-Vinylimidazole der allgemeinen Formel (I) 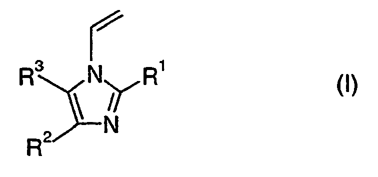 worin R¹ bis R³ für Wasserstoff, C₁-C₄-Alkyl oder Phenyl steht, Diallylamine der allgemeinen Formel (II), sowie Dialkylaminoalkyl(meth)acrylate und Dialkylaminoalkyl(meth)acrylamide der allgemeinen Formel (IIIa), z.B. Dimethylaminoethylmethacrylat oder Dimethylaminopropylmethacrylamid.

Ferner eignen sich ungesättigte Carbonsäuren und ungesättigte Anhydride, z.B. Acrylsäure, Methacrylsäure, Crotonsäure, Itaconsäure, Maleinsäure, Fumarsäure oder ihre entsprechenden Anhydride sowie von Monomer a) verschiedene ungesättigte Sulfonsäuren, wie z.B. Acrylamidomethylpropansulfonsäure, sowie die Salze der ungesättigten Säuren, wie z.B. die Alkali- oder Ammoniumsalze.

Als weitere Monomere e) seien genannt C₁-C₄₀-Alkylester der (Meth)acrylsäure, wobei die Ester abgeleitet werden von linearen, verzweigtkettigen oder carbocyclischen Alkoholen, z.B. Methyl-(meth)acrylat, Ethyl(meth)-acrylat, tert.-Butyl(meth)acrylat, Isobutyl(meth)acrylat, n-Butyl(meth)-acrylat, Stearyl(meth)acrylat, oder Ester von alkoxylierten Fettalkoholen, z.B. C₁-C₄₀-Fettalkoholen, umgesetzt mit Ethylenoxid, Propylenoxid oder Butylenoxid, insbesondere C₁₀-C₁₈₋Fettalkohole, umgesetzt mit 3 bis 150 Ethylenoxideinheiten. Weiterhin eignen sich von Monomer a) verschiedene N-Alkyl-substituierte Acrylamide mit linearen, verzweigtkettigen oder carbocyclischen Alkylresten wie N-tert.-Butylacrylamid, N-Butylacrylamid, N-Octylacrylamid, N-tert.-Octylacrylam id.

Ferner eignen sich Styrol, Vinyl- und Allylester von C₁-C₄₀-Carbonsäuren, die linear, verzweigtkettig oder carbocyclisch sein können, z.B. Vinylacetat, Vinylpropionat, Vinylneononanoat, Vinylneoundekansäure, t-Butyl-benzoesäurevinylester, Alkylvinylether, beispielsweise Methylvinylether, Ethylvinylether, Butylvinylether, Stearylvinylether.

Ferner eignen sich von Monomer a) verschiedene (Meth)Acrylamide, wie N-tert.-Butyl(meth)-acrylamid, N-Butyl(meth)acrylamid, N-Octyl(meth)acrylamid, N-tert.-Octyl(meth)acrylamid und von Monomer a) verschiedene N-Alkyl-substituierte Acrylamide mit linearen, verzweigtkettigen oder carbocyclischen Alkylresten, wobei der Alkylrest die oben für R⁴ angegebenen Bedeutungen besitzen kann.

Als Monomere (e) eignen sich insbesondere C₁ bis C₂₄-, ganz besonders C₁ bis C₁₀-Alkylester der (Meth)acrylsäure, z.B. Methyl(meth)acrylat, Ethyl(meth)acrylat, tert.-Butyl(meth)-acrylat, Isobutyl(meth)acrylat, n-Butyl(meth)acrylat und von Monomer a) verschiedene (Meth)acrylamide wie N-tert.-Butylacrylamid oder N-tert.-Octylacrylamid.

In einer bevorzugten Ausführungsform der Erfindung werden als weitere Monomere e) kationische und/oder quartemisierbare Monomere eingesetzt. Geeignete weitere Monomere sind die N-Vinylimidazol-Derivate der allgemeinen Formel (I), worin R¹ bis R³ für Wasserstoff, C₁-C₄₋Alkyl oder Phenyl steht.

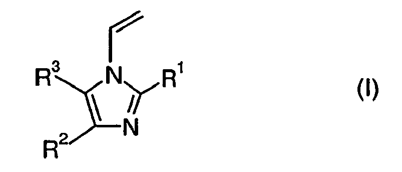

Weiterhin eignen sich Diallylamine der allgemeinen Formel (II), worin R⁴ für C₁-C₂₄-Alkyl steht

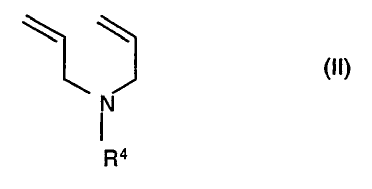

Weiterhin eignen sich N,N-Dialkylaminoalkylacrylate und -methacrylate und N,N-Dialkylaminoalkylacrylamide und -methacrylamide der allgemeinen Formel (IIIa), 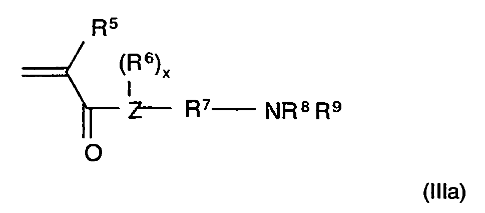 wobei R⁵, R⁶ unabhängig für ein Wasserstoffatom oder einen Methylrest stehen, R⁷ für ein Alkylenrest mit 1 bis 24 C-Atomen, optional substituiert durch Alkylreste und R⁸, R⁹ für C₁-C₂₄ Alkylreste. Z steht für ein Stickstoffatom zusammen mit x =1 oder für ein Sauerstoffatom zusammen mit x = 0.

Beispiele für Verbindungen der allgemeinen Formel (I) sind folgender Tabelle 2 zu entnehmen:

**Tabelle 2**

| R¹ | R² | R³ |
|---|---|---|
| H | H | H |
| Me | H | H |
| H | Me | H |
| H | H | Me |
| Me | Me | H |
| H | Me | Me |
| Me | H | Me |
| Ph | H | H |
| H | Ph | H |
| H | H | Ph |
| Ph | Me | H |
| Ph | H | Me |
| Me | Ph | H |
| H | Ph | Me |
| H | Me | Ph |
| Me | H | Ph |

| | | |
|---|---|---|
| Me = Methyl; Ph = Phenyl | | |

Weitere brauchbare Monomere der Formel (I) sind die Ethyl-, Propyl- oder Butyl-Analoga der in Tabelle 2 aufgelisteten Methyl-substituierten 1-Vinylimidazole.

Beispiele für Verbindungen der allgemeinen Formel (II) sind Diallylamine, worin R⁴ für Methyl, Ethyl, iso- oder n-Propyl, iso-, n- oder tert.-Butyl, Pentyl, Hexyl, Heptyl, Octyl, Nonyl oder Decyl steht. Beispiele für längerkettige Reste R⁴ sind Undecyl, Dodecyl, Tridecyl, Pentadecyl, Octadecyl und Icosayl.

Beispiele für Verbindungen der allgemeinen Formel (IIIa) sind N,N-Dimethylaminomethyl(meth)-acrylat; N,N-Diethylaminomethyl(meth)acrylat;
N,N-Dimethylaminoethyl(meth)acrylat; N,N-Diethylaminoethyl(meth)acrylat;
N,N-Dimethylaminobutyl(meth)acrylat; N,N-Diethylaminobutyl(meth)acrylat,
N,N-Dimethylaminohexyl(meth)acrylat;N,N-Dimethylaminooctyl(meth)acrylat,
N,N-Dimethylaminododecyl(meth)acrylat; N-[3-(dimethylamino)propyl]methacrylamid,
N-[3-(dimethylamino)propyl]acrylamid; N-[3-(dimethylamino)butyl]methacrylamid,
N-[8-(dimethylamino)octyl]methacrylamid; N-[12-(dimethylamino)dodecyl]methacrylamid,
N-[3-(diethylamino)propyl]methacrylamid; N-[3-(diethylamino)propyl]acrylamid.

Bevorzugte Beispiele für weitere Monomere sind 3-Methyl-1-vinylimidazoliumchlorid und -methosulfat, Dimethyldiallylammoniumchlorid sowie N,N-Dimethylaminoethylmethacrylat und N-[3-(dimethylamino)propyl]methacrylamid, die durch Methylchlorid, Dimethylsulfat oder Diethylsulfat quaternisiert wurden.

Besonders bevorzugte Monomere sind 3-Methyl-1-vinylimidazoliumchlorid und -methosulfat und Dimethyldiallylammoniumchlorid, ganz besonders bevorzugt sind 3-Methyl-1-vinylimidazoliumchlorid und -methosulfat.

Die weiteren Monomere können entweder in quaternisierter Form als Monomere eingesetzt werden oder nicht-quatemisiert polymerisiert werden, wobei man im letzteren Fall das erhaltene Polymer entweder quaternisiert oder protoniert.

Zur Quaternisierung der Verbindungen der allgemeinen Formel (I) bis (IIIa) eignen sich beispielsweise Alkylhalogenide mit 1 bis 24 C-Atomen in der Alkylgruppe, z.B. Methylchlorid, Methylbromid, Methyliodid, Ethylchlorid, Ethylbromid, Propylchlorid, Hexylchlorid, Dodecylchlorid, Laurylchlorid und Benzylhalogenide, insbesondere Benzylchlorid und Benzylbromid. Weitere geeignete Quaternierungsmittel sind Dialkylsulfate, insbesondere Dimethylsulfat oder Diethylsulfat. Die Quaternisierung der basischen Monomere der allgemeinen Formel (I) bis (IIIa) kann auch mit Alkylenoxiden wie Ethylenoxid oder Propylenoxid in Gegenwart von Säuren durchgeführt werden.

Die Quaternisierung des Monomeren oder eines Polymeren mit einem der genannten Quaternisierungsmittel kann nach allgemein bekannten Methoden erfolgen.

Bevorzugte Quaternierungsmittel sind: Methylchlorid, Dimethylsulfat oder Diethylsulfat.

Die Quaternisierung des Polymeren kann vollständig oder auch nur teilweise erfolgen. Der Anteil quaternisierter Monomere im Polymeren kann über einen weiten Bereich variieren und liegt z.B. bei etwa 20 bis 100 Mol.-%.

Zur Protonierung eignen sich beispielsweise Mineralsäuren wie HCl, H₂SO₄, H₃PO₄, sowie Monocarbonsäuren, wie z.B. Ameisensäure und Essigsäure, Dicarbonsäuren und mehrfunktionelle Carbonsäuren, wie z.B. Oxalsäure, Milchsäure und Zitronensäure, sowie alle anderen protonenabgebenden Verbindungen und Substanzen, die in der Lage sind, das entsprechende Vinylimidazol oder Diallylamin zu protonieren. Insbesondere eignen sich wasserlösliche Säuren zur Protonierung.

Die Protonierung des Polymers kann entweder im Anschluss an die Polymerisation erfolgen oder bei der Formulierung der kosmetischen Zusammenstellung, bei der in der Regel ein physiologisch verträglicher pH-Wert eingestellt wird.

Unter Protonierung ist zu verstehen, dass mindestens ein Teil der protonierbaren Gruppen des Polymers, bevorzugt 20 bis 100 Mol-%, protoniert wird, so dass eine kationische Gesamtladung des Polymers resultiert.

### Regler f)

Die radikalische Polymerisation kann in Gegenwart mindestens eines Reglers f) erfolgen. Als Regler (Polymerisationsregler) werden Verbindungen mit hohen Übertragungskonstanten bezeichnet. Regler beschleunigen Kettenübertragungsreaktionen und bewirken damit eine Herabsetzung des Polymerisationsgrades der resultierenden Polymeren, ohne die Bruttoreaktions-Geschwindigkeit zu beeinflussen.

Bei den Reglern kann man zwischen mono-, bi- oder polyfunktionalen Regler unterscheiden je nach Anzahl der funktionellen Gruppen im Molekül, die zu einen oder mehreren Kettenübertragungsreaktionen führen können. Geeignete Regler werden beispielsweise ausführlich beschrieben von K.C. Berger und G. Brandrup in J. Brandrup, E.H. Immergut, Polymer Handbook, 3. Aufl., John Wiley & Sons, New York, 1989, S. II/81 -II/141.

Als Regler eignen sich beispielsweise Aldehyde wie Formaldehyd, Acetaldehyd, Propionaldehyd, n-Butyraldehyd, Isobutyraldehyd.

Ferner können auch als Regler eingesetzt werden: Ameisensäure, ihre Salze oder Ester, 2,5-Diphenyl-1-hexen, Ammoniumformiat, Hydroxylammoniumsulfat, und Hydroxylammoniumphosphat.

Weitere geeignete Regler sind Halogenverbindungen wie Alkylhalogenide, wie Tetrachlormethan, Chloroform, Bromtrichlormethan, Bromoform, Allylbromid, und Benzylverbindungen, wie Benzylchlorid oder Benzylbromid.

Weitere geeignete Regler sind Allylverbindungen, wie z.B. Allylalkohol, funktionale Allylether, wie Allyl Ethoxylate, alkyl allyl ether, oder Glycerin Monoallylether.

Bevorzugt werden als Regler Verbindungen eingesetzt, die Schwefel in gebundener Form enthalten.

Verbindungen dieser Art sind beispielsweise anorganische Hydrogensulfite, Disulfite und Dithionite oder organische Sulfide, Disulfide, Polysulfide, Sulfoxide, Sulfone. Folgende Regler werden beispielhaft genannt: Di-n-butylsulfid, Di-n-octylsulfid, Diphenylsulfid, Thiodiglykol, Ethylthioethanol, Diisopropyldisulfid, Di-n-butyldisulfid, Di-n-hexyldisulfid, Diacetyldisulfid, Diethanolsulfid, Di-t-butyltrisulfid, Dimethylsulfoxid, Dialkylsulfid, Dialkyldisulfid und/oder Diarylsulfid.

Besonders bevorzugt sind organische Verbindungen, die Schwefel in gebundener Form enthalten.

Bevorzugt als Polymerisationsregler eingesetzte Verbindungen sind Thiole (Verbindungen, die Schwefel in Form von SH-Gruppen enthalten, auch als Mercaptane bezeichnet). Bevorzugt sind als Regler mono-, bi- und polyfunktionale Mercaptane, Mercaptoalkohole und/oder Mercaptocarbonsäuren.

Beispiele für diese Verbindungen sind Allylthioglykolate, Ethylthioglykolat, Cystein, 2-Mercaptoethanol, 1,3-Mercaptopropanol, 3-Mercaptopropan-1,2-diol, 1,4-Mercaptobutanol, Mercaptoessigsäure, 3-Mercaptopropionsäure, Mercaptobemsteinsäure, Thioglycerin, Thioessigsäure, Thioharnstoff und Alkylmercaptane wie n-Butylmercaptan, n-Hexylmercaptan oder n-Dodecylmercaptan.

Besonders bevorzugte Thiole sind Cystein, 2-Mercaptoethanol, 1,3-Mercaptopropanol, 3-Mercaptopropan-1,2-diol, Thioglycerin, Thioharnstoff.

Beispiele für bifunktionale Regler, die zwei Schwefel in gebundener Form enthalten sind bifunktionale Thiole wie z.B. Dimercaptopropansulfonsäure (Natrium Salz), Dimercaptobemsteinsäure, Dimercapto-1-propanol, Dimercaptoethan, Dimercaptopropan, Dimercaptobutan, Dimercaptopentan, Dimercaptohexan, Ethylenglykol-bis-thioglykolate und Butandiol-bis-thioglykolat.

Beispiele für polyfunktionale Regler sind Verbindungen, die mehr als zwei Schwefel in gebundener Form enthalten. Beispiele hierfür sind trifunktionale und/oder tetrafunktionale Mercaptane.

Bevorzugte trifunktionale Regler sind trifunktionale Mercaptane, wie z.B. Trimethylolpropan tris(2-mercaptoethanat, Trimethylolpropan tris(3-mercaptopropionat), Trimethylolpropan tris(4-mercaptobutanat), Trimethylolpropan tris(5-mercaptopentanat), Trimethylolpropan tris(6-mercaptohexanat), Trimethylolpropan tris(2-mercaptoacetat).

Glyceryl thioglycolat, glyceryl thiopropionat, glyceryl thioethylat, glycerylthiobutanat. 1,1,1-Propanetriyl tris-(Mercaptoacetat), 1,1,1-Propanetriyl tris-(Mercaptoethanat), 1,1,1-Propanetriyl tris-(Mercaptoproprionat), 1,1,1-Propanetriyl tris-(Mercaptobutanat) 2-hydroxymethyl-2-methyl-1,3-propandiol tris-(Mercaptoacetat), 2-hydroxymethyl-2-methyl-1,3-propandiol tris-(Mercaptoethanat), 2-hydroxymethyl-2-methyl-1,3-propandiol tris-(Mercaptopropionat), 2-hydroxymethyl-2-methyl-1,3-propandiol tris-(Mercaptobutanat).

Besonders bevorzugte trifunktionale Regler sind Glyceryl thioglycolat, Trimethylolpropan tris(2-mercaptoacetat, 2-hydroxmethyl-2-methyl-1,3-propandiol tris-(Mercaptoacetat).

Bevorzugte tetrafunktionale Mercaptane sind Pentaerythritol tetraquis (2-mercaptoacetat), Pentaeryhtritol tetraquis(2-mercaptoethanat), Pentaerythritol tetraquis(3-mercaptopropionat), Pentaerythritol tetraquis-(4-mercaptobutanat), Pentaerythritol tetraquis(5-mercaptopentanat), Pentaerythritol tetraquis(6-mercaptohexanat).

Als weitere polyfunktionale Regler eignen sich Si-Verbindungen, die durch Umsetzung von Verbindungen der Formel (IVa) entstehen. Weiterhin eignen sich als polyfunktionale Regler Si-Verbindungen der Formel (IVb). 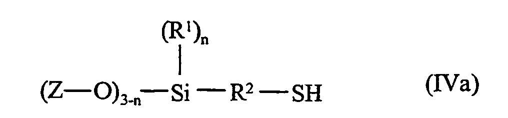 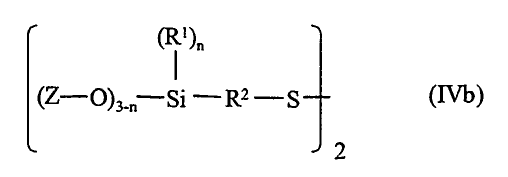 in der
n ein Wert von 0 bis 2 ist,
R¹ eine C₁-C₁₆-Alkylgruppe oder Phenylgruppe bedeutet
R² eine C₁-C₁₈-Alkylgruppe, die Cyclohexyl-oder Phenylgruppe bezeichnet,
Z für eine C₁-C₁₈ Alkylgruppe, C₂-C₁₈-Alkylengruppe oder C₂-C₁₈-Alkinylgruppe steht, deren Kohlenstoffatome durch nichtbenachbarte Sauerstoff- oder Halogenatome ersetzt sein können, oder für eine der Gruppen 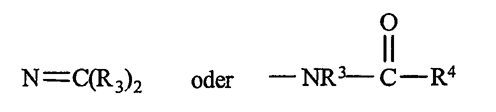 in denen
R₃ eine C₁-C₁₂-Alkylgruppe bedeutet und
R₄ eine C₁-C₁₈-Alkylgruppe bezeichnet.

Besonders bevorzugt sind die Verbindungen IVa, darunter vor allem Mercaptopropyltrimethoxysilan und Mercaptopropyltriethoxysilan.

Alle genannten Regler können einzeln oder in Kombination miteinander eingesetzt werden.

In einer bevorzugten Ausführungsform des Verfahrens werden multifunktionelle Regler eingesetzt.

Ein weiterer Gegenstand der Erfindung betrifft ein Verfahren zur Herstellung von wässrigen Dispersionen wobei a) mindestens ein N-Vinylhaltiges Monomer und/oder mindestens ein
a) (Meth)acrylamidmonomer
b) mindestens ein polymeres Dispergiermittel
c) mindestens ein polymeres Fällungsagens
d) mindestens ein Vernetzer
e) gegebenenfalls weitere Monomere
g) gegebenenfalls in Gegenwart einer Puffersubstanz
in Gegenwart mindestens eines Reglers umgesetzt werden und das Gewichtsverhältnis von b) zu c) im Bereich von 1:50 bis 1:0,02 liegt.

Als Initiatoren für die radikalische Polymerisation können wasserlösliche und wasserunlösliche Peroxo- und/oder Azo-Verbindungen eingesetzt werden, beispielsweise Alkali- oder Ammoniumperoxidisulfate, Wasserstoffperoxid, Dibenzoylperoxid, tert.-Butylperpivalat, 2,2'-Azobis-(2,4-dimethylvaleronitril), tert.-Butylperoxineodecanoat, tert.-Butyl-per-2-ethylhexanoat, Di-tert.-butylperoxid, tert.-Butylhydroperoxid, Azo-bis-isobutyronitril, Azo-bis-(2-amidinopropan)dihydrochlorid oder 2,2'-Azo-bis-(2-methylbutyronitril). Geeignet sind auch Initiatormischungen oder Redox-Initiator Systeme, wie z.B. Ascorbinsäure/Eisen(II)sulfat/-Natriumperoxodisulfat, tert.-Butylhydroperoxid/Natriumdisulfit, tert.-Butylhydroperoxid/Natriumhydroxymethansulfanat, Wasserstoffperoxid/Ascorbinsäure. Die Initiatoren können in den üblichen Mengen eingesetzt werden, beispielsweise 0,05 bis 7 Gew.-%, bezogen auf die Menge der zu polymerisierenden Monomeren.

Durch die Mitverwendung von Redox-Coinitiatoren, beispielsweise Benzoin, Dimethylanilin sowie organisch löslicher Komplexe und Salze von Schwermetallen, wie Kupfer, Kobalt, Mangan, Nickel und Chrom oder insbesondere Eisen, können die Halbwertzeiten der genannten Peroxide, besonders der Hydroperoxide, verringert werden, so daß beispielsweise tert.-Buthylhydroperoxid in Gegenwart von 5 ppm Kupfer-II-Acetylacetonat bereits bei 100°C wirksam ist.

Bevorzugt werden wasserlösliche Initiatoren eingesetzt, wie Hydroperoxide, Peroxide und/oder Hydrochloride. Besonders bevorzugt wird das Verfahrenmit den Initiatoren durchgeführt, die ausgewählt sind aus der Gruppe, die gebildet wird von Wasserstoffperoxid, tert.-Butylhydroperoxid, Azo-bis-(2-amidinopropan)dihydrochlorid und/oder Wasserstoffperoxid/Ascorbinsäure.

In einer Ausführungsform der Erfindung wird das Verfahren in Gegenwart eines Puffers g) durchgeführt. Bevorzugt ist auch eine pH-Regelung der Polymerisation durch eine dosierte/automatisierte Zugabe von Säuren oder Basen, wodurch der bevorzugte pH-Bereich während der gesamten Polymerisation eingehalten werden kann.

Die Polymerisationsreaktion wird mit Hilfe von in Radikale zerfallende Polymerisationsinitiatoren gestartet. Es können sämtliche Initiatoren eingesetzt werden, die für die Polymerisation der Monomeren bekannt sind. Geeignet sind beispielsweise in Radikale zerfallende Initiatoren, die bei den jeweils gewählten Temperaturen Halbwertzeiten von weniger als 3 Stunden besitzen. Falls die Polymerisation bei unterschiedlichen Temperaturen durchgeführt wird, indem man die Monomeren zunächst bei einer niedrigeren Temperatur anpolymerisiert und anschließend bei einer deutlich höheren Temperatur auspolymerisiert, so verwendet man zweckmäßigerweise mindestens zwei unterschiedliche Initiatoren, die in dem jeweils gewählten Temperaturbereich eine ausreichende Zerfallsgeschwindigkeit haben.

Die Polymerisation wird üblicherweise bei Temperaturen zwischen 20 und 200°C, bevorzugt zwischen 30 und 90°C, ganz bevorzugt zwischen 40 und 80°C durchgeführt bei Normaldruck oder unter Eigendruck. Bevorzugt ist die Polymerisation unter Stickstoff-Atmosphäre, insbesondere bei leicht erhöhtem Druck (z.B. 0,5 bis 1 bar Stickstoff-Überdruck vor Beginn der Polymerisation bei T = 25°C einstellen).

Übliche Verfahrenshilfsmittel wie Komplexierer (beispielsweise Ethylendiamintetraessigsäure, EDTA), Geruchsstoffe können gegebenenfalls zugesetzt werden. Viskositätsveränderer wie Glycerol, Methanol, Ethanol, t-Butanol, Glycol, etc können ebenfalls in der wässrigen Dispersion zugegeben sein.

Die Polymerisation wird in einer bevorzugten Ausführungsform als Batchfahrweise durchgeführt. Hierbei ist bevorzugt, die Komponenten (a-g) in der Vorlage vorzulegen.

In einer weiteren bevorzugten Ausführungsform wird das erfindungsgemäße Verfahren als Zulauffahrweise durchgeführt. Dabei werden einzelne oder alle Reaktionsteilnehmer ganz oder teilweise, absatzweise oder kontinuierlich, gemeinsam oder in getrennten Zuläufen zu einer Reaktionsmischung gegeben. Es ist jedoch auch möglich, den Initiator zu der auf Polymerisationstemperatur erwärmten Vorlage enthaltend die polymere Dispergiermittel, polymeren Fällungsagenz(ien), und monomere Komponenten (a), (d) und gegebenenfalls Monomer (e) und Regler (f) und Puffer (g) zu zudosieren. In einer weiteren Variante werden zu einem Gemisch aus Monomeren (a) und (d) und gegebenenfalls Monomer (e) einem Lösemittel der Initiator und einem Lösemittel der Regler (f) nach Erreichen der Polymerisations-Temperatur über längere Zeit kontinuierlich zu einer Vorlage enthaltend (g) zugegeben. Man kann auch die Vorlage enthaltend die polymeren Fällungsagenzien (c) und polymeren Dispergiermittel b) und Monomer (d) und gegebenenfalls Puffer (g) auf Polymerisationstemperatur erwärmen und Initiator Lösung und Monomere (a) und gegebenenfalls (e) in getrennten Zuläufen zugeben.

Selbstverständlich können auch Initiator, Monomere d) und Monomere a) und gegebenenfalls Monomere e) zu einer auf Polymerisationstemperatur erwärmten Vorlage enthaltend ein Gemisch polymere Fällungsagenzien c) und polymerer Dispergiermittel b) und Puffer (g) gegeben werden.

Vorzugsweise verwendet man ein Gemisch aus polymeren Fällungsagenzien c) und polymeren Dispergiermitteln b) in Wasser und mindestens einem Teil der Monomeren a), d) und gegebenenfalls e) und gegebenenfalls Regler f) und gegebenenfalls Puffer (g) sowie gegebenenfalls weitere Komponenten als Vorlage.

Die Dispersionen sind üblicherweise milchig weiss und haben in der Regel eine Viskosität von 100 bis 50,000 m Pas, bevorzugt von 200 bis 20,000 m Pas, besonders bevorzugt von 300 bis 15,000 m Pas.

Die bei der Polymerisation entstandenen Dispersionen können im Anschluss an den Polymerisationsprozess einer physikalischen oder chemischen Nachbehandlung unterworfen werden. Solche Verfahren sind beispielsweise die bekannten Verfahren zur Restmonomerenreduzierung wie zB. die Nachbehandlung durch Zusatz von Polymerisationsinitiatoren oder Mischungen mehrerer Polymerisationsinitiatoren bei geeigneten Temperaturen oder Erhitzen der Polymerisationslösung auf Temperaturen oberhalb der Polymerisationstemperatur, eine Nachbehandlung der Polymerlösung mittels Wasserdampf oder Strippen mit Stickstoff oder Behandeln der Reaktionsmischung mit oxidierenden oder reduzierenden Reagenzien, Adsorptionsverfahren wie die Adsorption von Verunreinigung an ausgewählten Medien wie z.B. Aktivkohle oder eine Ultrafiltration. Es können sich auch die bekannten Aufarbeitungsschritte anschließen, beispielsweise geeignete Trockenverfahren wie Sprüh-, Gefrier- oder Walzentrocknung oder an die Trocknung anschließende Agglomerationsverfahren. Die nach dem erfindungsgemäßen Verfahren erhaltenen restmonomerenarmen Dispersionen können auch direkt in den Handel gebracht werden.

In einer bevorzugten Ausführungsform werden die wässrigen Dispersionen einer Behandlung unterzogen mit dem Ziel, die im Polymer enthaltene Komponente (a) in das entsprechende Amin umzuwandeln, so dass der Anteil der entstehenden Amine im Polymer < 20 mol-%, bevorzugt < 15 mol-%, insbesondere unter 10 mol-%, besonders bevorzugt unter 5 mol-% bezogen auf die Komponente (a) liegt. Als geeignet Methode sei die Hydrolyse genannt.

Durch Abspaltung von Formylgruppen aus N-Vinylformamideinheiten enthaltenden Polymerisaten und durch Abspaltung der Gruppe CH₃-CO- aus N-Vinylacetamideinheiten enthaltenden Polymerisaten entstehen jeweils Vinylamin-Einheiten enthaltende Polymerisate. Die Abspaltung kann partiell oder vollständig durchgeführt werden. Sofern die Hydrolyse in Gegenwart von Säuren vorgenommen wird, liegen die Vinylamin-Einheiten der Polymeren als Ammoniumsalze vor. Die Hydrolyse kann jedoch auch mit Hilfe von Basen vorgenommen werden, z.B. von Metallhydroxiden, insbesondere von Alkalimetall- und Erdalkalimetallhydroxiden. Vorzugsweise verwendet man Natriumhydroxid oder Kaliumhydroxid. In besonderen Fällen kann die Hydrolyse auch mit Hilfe von Ammoniak oder Aminen durchgeführt werden. Bei der Hydrolyse in Gegenwart von Basen liegen die Vinylamin-Einheiten in Form der freien Basen vor.

Als Hydrolysemittel eignen sich vorzugsweise Mineralsäuren, wie Halogenwasserstoffe, die gasförmig oder als wäßrige Lösung eingesetzt werden können. Vorzugsweise verwendet man konzentrierte Salzsäure, Schwefelsäure, Salpetersäure oder Phosphorsäure sowie organische Säuren, wie C₁- bis C₅-Carbonsäuren, sowie aliphatische oder aromatische Sulfonsäuren. Beispielsweise benötigt man pro Formylgruppenäquivalent in den N-Vinylformamideinheiten ein polymerisiert enthaltenden Polymeren 0,05 bis 2, insbesondere 1 bis 1,5 Moläquivalente einer Säure. Die Hydrolyse der N-Vinylformamind-Einheiten verläuft bedeutend schneller als die der N-Vinylacetamid-Einheiten aufweisenden Polymerisate. Sofern man Copolymerisate der in Betracht kommenden Vinylcarbonsäureamide mit anderen Comonomeren der Hydrolyse unterwirft, so können auch die im Copolymerisat enthaltenen Comonomer-Einheiten chemisch verändert werden. So entstehen beispielsweise aus Vinylacetat-Einheiten Vinylalkohol-Einheiten. Aus Acrylsäuremethylester-Einheiten entstehen bei der Hydrolyse Acrylsäure-Einheiten und aus Acrylnitril-Einheiten werden Acrylamid- bzw. Acrylsäure-Einheiten gebildet. Die Hydrolyse der N-Vinylformamid- und/oder Vinylacetamid-Einheiten der Polymerisate (A) kann zu 5 bis 100, vorzugsweise 10 bis 40 % durchgeführt werden. Obwohl die wäßrigen Dispersionen von wasserlöslichen N-Vinylcarbonsäureamiden beim Verdünnen mit Wasser in Lösung gehen, wird die Dispersion bei der Hydrolyse überraschenderweise nicht zerstört.
Für die im folgenden genannten erfindungsgemäßen Verwendungen können neben den erfindungsgemäßen Dispersionen auch solche wässrigen Dispersionen eingesetzt werden, die erhältlich sind durch radikalische Polymerisation von
a) mindestens einem (Meth)acrylamidmonomer und gegebenenfalls einem N-vinylhaltigen Monomer
b) mindestens einem polymeren Dispergiermittel
c) mindestens einem polymeren Fällungsagens
e) gegebenenfalls weiteren Monomeren
f) gegebenenfalls mindestens einem Regler
g) gegebenenfalls in Gegenwart einer Puffersubstanz
wobei das Gewichtsverhältnis von b) zu c) im Bereich von 1:50 bis 1:0,02 liegt.
In diesen ebenfalls zur erfindungsgemäßen Verwendung geeigneten Dispersionen entsprechen die Monomere a) und e), die polymeren Dispergiermittel b), die polymeren Fällungsagentien c), die Regler f) und die Puffersubstanzen g) sowie die jeweiligen Mengenverhältnisse den wie vorstehend beschriebenen Definitionen.
Beispielsweise werden die erfindungsgemäßen Dispersionen in kosmetischen Mitteln zur Reinigung der Haut verwendet. Solche kosmetischen Reinigungsmittel sind ausgewählt aus Stückseifen, wie Toilettenseifen, Kemseifen, Transparentseifen, Luxusseifen, Deoseifen, Cremeseifen, Babyseifen, Hautschutzseifen, Abrasiveseifen und Syndets, flüssigen Seifen, wie pastöse Seifen, Schmierseifen und Waschpasten, und flüssigen Wasch-, Dusch- und Badepräparaten, wie Waschlotionen, Duschbädern und -gelen, Schaumbädern, Ölbädern und Scrub-Präparaten.

Bevorzugt werden die erfindungsgemäßen Dispersionen in kosmetischen Mitteln zur Pflege und zum Schutz der Haut, in Nagelpflegemitteln sowie in Zubereitungen für die dekorative Kosmetik angewendet.

Besonders bevorzugt ist die Verwendung in Hautpflegemitteln, Intimpflegemitteln, Fußpflegemitteln, Deodorantien, Lichtschutzmitteln, Repellents, Rasiermitteln, Haarentfernungsmitteln, Antiaknemitteln, Make-up, Maskara, Lippenstifte, Lidschatten, Kajalstiften, Eyelinern, Rouges, Pudern und Augenbrauenstiften.

Die Hautpflegemittel liegen insbesondere als W/O- oder O/W-Hautcremes, Tag- und Nachtcremes, Augencremes, Gesichtscremes, Antifaltencremes, Feuchthaltecremes, Bleichcremes, Vitamincremes, Hautlotionen, Pflegelotionen und Feuchthaltelotionen vor.

In den kosmetischen Zubereitungen können die erfindungsgemäßen Dispersionen besondere Wirkungen entfalten. Die Dispersionen können unter anderem zur Feuchthaltung und Konditionierung der Haut und zur Verbesserung des Hautgefühls beitragen. Die Dispersionen können auch als Verdicker in den Formulierungen wirken. Durch Zusatz der erfindungsgemäßen Dispersioen kann in bestimmten Formulierungen eine erhebliche Verbesserung der Hautverträglichkeit erreicht werden.

Die erfindungsgemäßen Dispersionen sind in den hautkosmetischen Zubereitungen in einem Anteil von etwa 0,001 bis 20 Gew.-%, vorzugsweise 0,01 bis 10 Gew.-%, ganz besonders bevorzugt 0,1 bis 5 Gew.-%, bezogen auf das Gesamtgewicht des Mittels, enthalten.

Je nach Anwendungsgebiet können die erfindungsgemäßen Mittel in einer zur Hautpflege geeigneten Form, wie z.B. als Creme, Schaum, Gel, Stift, Pulver, Mousse, Milch oder Lotion appliziert werden.

Die hautkosmetischen Zubereitungen können neben den erfindungsgemäßen Dispersionen und geeigneten Lösungsmitteln noch in der Kosmetik übliche Zusätze, wie Emulgatoren, Konservierungsmittel, Parfümöle, kosmetische Wirkstoffe wie Phytantriol, Vitamin A, E und C, Retinol, Bisabolol, Panthenol, Lichtschutzmittel, Bleichmittel, Färbemittel, Tönungsmittel, Bräunungsmittel (zB. Dihydroxyaceton), Collagen, Eiweißhydrolysate, Stabilisatoren, pH-Wert-Regulatoren, Farbstoffe, Salze, Verdicker, Gelbildner, Konsistenzgeber, Silikone, Feuchthaltemittel, Rückfetter und weitere übliche Additive enthalten.

Als geeignete Lösungsmittel sind insbesondere zu nennen Wasser und niedrige Monoalkohole oder Polyole mit 1 bis 6 Kohlenstoffatomen oder Mischungen davon; bevorzugte Monoalkohole oder Polyole sind Ethanol, i-Propanol, Propylenglycol, Glycerin und Sorbit.

Als weitere übliche Zusätze können enthalten sein Fettkörper, wie mineralische und synthetische Öle, wie z.B. Paraffine, Siliconöle und aliphatische Kohlenwasserstoffe mit mehr als 8 Kohlenstoff-atomen, tierische und pflanzliche Öle, wie z.B. Sonnenblumenöl, Kokosöl, Avocadoöl, Olivenöl, Lanolin, oder Wachse, Fettsäuren, Fettsäureester, wie z.B. Triglyceride von C₆-C₃₀-Fettsäuren, Wachsester, wie z.B. Jojobaöl, Fettalkohole, Vaseline, hydriertes Lanolin und azetyliertes Lanolin. Selbstverständlich können auch Mischungen derselben verwendet werden.
Übliche Verdickungsmittel in derartigen Formulierungen sind vernetzte Polyacrylsäuren und deren Derivate, Polysaccharide wie Xanthan-Gum, Agar-Agar, Alginate oder Tylosen, Carboxymethylcellulose oder Hydroxycarboxymethylcellulose, Fettalkohole, Monoglyceride und Fettsäuren, Polyvinylakolhol und Polyvinylpyrrolidon.

Man kann die erfindungsgemäßen Dispersionen auch mit herkömmlichen Polymeren abmischen, falls spezielle Eigenschaften eingestellt werden sollen.

Als herkömmliche Polymere eignen sich beispielsweise anionische, kationische, amphotere und neutrale Polymere.

Beispiele für anionische Polymere sind Homo- und Copolymerisate von Acrylsäure und Methacrylsäure oder deren Salze, Copolymere von Acrylsäure und Acrylamid und deren Salze; Natriumsalze von Polyhydroxycarbonsäuren, wasserlösliche oder wasserdispergierbare Polyester, Polyurethane und Polyharnstoffe. Besonders geeignete Polymere sind Copolymere aus t-Butylacrylat, Ethylacrylat, Methacrylsäure (z.B. Luvimer^{™} 100P), Copolymere aus Ethylacrylat und Methacrylsäure (z.B. Luvimer^{™} MAE), Copolymere aus N-tert.-Butyl-acrylamid, Ethylacrylat, Acrylsäure (Ultrahold^{™} 8, strong), Copolymere aus Vinylacetat, Crotonsäure und gegebenenfalls weitere Vinylester (z.B. Luviset^{™} Marken), Maleinsäureanhydridcopolymere, ggf. mit Alkoholen umgesetzt, anionische Polysiloxane, z.B. carboxyfunktionelle, Copolymere aus Vinylpyrrolidon, t-Butylacrylat, Methacrylsäure (z.B Luviskol^{™} VBM), Copolymere von Acrylsäure und Methacrylsäure mit hydrophoben Monomeren, wie z.B. C₄-C₃₀-Alkylester der Meth(acrylsäure), C₄-C₃₀-Alkylvinylester, C₄-C₃₀-Alkylvinylether und Hyaluronsäure. Luviset P.U.R., Luviflex Silk.

Weitere geeignete Polymere sind kationische Polymere mit der Bezeichnung Polyquaternium nach INCl, z.B. Copolymere aus Vinylpyrrolidon/N-Vinylimidazoliumsalzen (Luviquat^{™} FC, Luviquat^{™} HM, Luviquat^{™} MS, Luviquat^{™} Care, Luviquat^{™} Hold, INCl Polyquaternium-16, -44, -46), Copolymere aus Acrylamid und Dimethyldiallylammoniumchlorid (Polyquaternium-7), kationische Cellulosederivate (Polyquaternium-4, -10), kationische Stärkederivate (INCl: Starch Hydroxypropytrimonium Chloride, Corn Starch Modified), kationische Guarderivate (INCl: Hydroxypropyl Guar Hydroxypropyftrimonium Chloride), kationische Sonnenblumenöl-Derivate (INCl: Sunflowerseedamidopropyl Hydroxyethyldimonium Chloride), Copolymere aus N-Vinylpyrrolidon/ Dimethylaminoethylmethacrylat, quaternisiert mit Diethylsulfat (Polyquaternium-11), Copolymere aus Acrylsäure, Acrylamid und Methacrylamidopropyltrimoniumchlorid (Polyquaternium-53), Polyquatemium-32, Polyquaternium-28 und andere.

Als weitere Polymere sind auch neutrale Polymere geeignet wie Polyvinylpyrrolidone, Copolymere aus N-Vinylpyrrolidon und Vinylacetat und/oder Vinylpropionat, Copolymere aus N-Vinylpyrrolidon/Dimethylaminopropylacrylamid oder-methacrylamid, Copolymere aus N-Vinylpyrrolidon und Alkylacrylat- oder -methacrylatmonomeren mit Alkylketten von C1 bis C18, Pfropfcopolymere von Polyvinylalkohol auf Polyalkylenglykole wie z.B. Kollicoat IR (BASF), Pfropfcopolymere von anderen Vinylmonomeren auf Polyalkylenglykole, Polysiloxane, Polyvinylcaprolactam und Copolymere mit N-Vinylpyrrolidon, Polyethylenimine und deren Salze, Polyvinylamine und deren Salze, Cellulosederivate, Chitosan, Polyasparaginsäuresalze und Derivate.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze, Dimethicone, Dimethicone-Derivate oder Dimethicon Copolyole (CTFA) und aminofunktionelle Silikonverbindungen wie Amodimethicone (CTFA).

Die erfindungsgemäßen Dispersionen werden in kosmetischen Zubereitungen eingesetzt, deren Herstellung nach den üblichen dem Fachmann geläufigen Regeln erfolgt.

Solche Formulierungen liegen vorteilhafterweise in Form von Emulsionen bevorzugt als Wasser-in-Öl-(W/O)- oder ÖI-in-Wasser-(O/W)-Emulsionen vor. Es ist aber auch erfindungsgemäß möglich und gegebenenfalls vorteilhaft andere Formulierungsarten zu wählen, beispielsweise Hydrodispersionen, Gele, Öle, Oleogele, multiple Emulsionen, beispielsweise in Form von W/O/W-oder O/W/O-Emulsionen, wasserfreie Salben bzw. Salbengrundlagen usw.

Die Herstellung erfindungsgemäß brauchbarer Emulsionen erfolgt nach bekannten Methoden.

Die Emulsionen enthalten neben der erfindungsgemäßen Dispersion übliche Bestandteile, wie Fettalkohole, Fettsäureester und insbesondere Fettsäuretriglyceride, Fettsäuren, Lanolin und Derivate davon, natürliche oder synthetische Öle oder Wachse und Emulgatoren in Anwesenheit von Wasser.

Die Auswahl der Emulsionstyp-spezifischen Zusätze und die Herstellung geeigneter Emulsionen ist beispielsweise beschrieben in Schrader, Grundlagen und Rezepturen der Kosmetika, Hüthig Buch Verlag, Heidelberg, 2. Auflage, 1989, Dritter Teil, worauf hiermit ausdrücklich Bezug genommen wird.

So kann eine erfindungsgemäß brauchbare Hautcreme z.B. als W/O-Emulsion vorliegen. Eine derartige Emulsion enthält eine wässrige Phase, die mittels eines geeigneten Emulgatorsystems in einer Öl- oder Fettphase emulgiert ist.

Die Konzentration des Emulgatorsystems beträgt in diesem Emulsions-Typ etwa 4 und 35 Gew.-%, bezogen auf das Gesamtgewicht der Emulsion; die Fettphase macht etwa 20 und 60 Gew.-% aus und die wässrige Phasen etwa 20 und 70 Gew.-%, jeweils bezogen auf das Gesamtgewicht der Emulsion. Bei den Emulgatoren handelt es sich um diejenigen, welche in diesem Emulsions-typ üblicherweise verwendet werden. Sie werden z.B. ausgewählt unter: C₁₂-C₁₈-SorbitanFettsäureestern; Estern von Hydroxystearinsäure und C₁₂-C₃₀-Fettalkoholen; Mono- und Diestern von C₁₂-C₁₈-Fettsäuren und Glyzerin oder Polyglyzerin; Kondensaten von Ethylenoxid und Propylenglycolen; oxypropylenierten/oxyethylenierten C₁₂-C₂₀-Fettalkoholen; polycyclischen Alkoholen, wie Sterolen; aliphatischen Alkoholen mit einem hohen Molekulargewicht, wie Lanolin; Mischungen von oxypropylenierten/polyglycerinierten Alkoholen und Magnesiumisostearat; Succinestern von polyoxyethylenierten oder polyoxypropylenierten Fettalkoholen; und Mischungen von Magnesium-, Calcium-, Lithium-, Zink- oder Aluminiumlanolat und hydriertem Lanolin oder Lanolin-alkohol.

Zu geeigneten Fettkomponenten, welche in der Fettphase der Emulsionen enthalten sein können, zählen Kohlenwasserstofföle, wie Paraffinöl, Purcellinöl, Perhydrosqualen und Lösungen mikrokristalliner Wachse in diesen Ölen; tierische oder pflanzliche Öle, wie Süßmandelöl, Avocadoöl, Calophylumöl, Lanolin und Derivate davon, Ricinusöl, Sesamöl, Olivenöl, Jojobaöl, Karité-Öl, Hoplostethus-Öl; mineralische Öle, deren Destillationsbeginn unter Atmosphärendruck bei ca. 250°C und deren Destillationsendpunkt bei 410°C liegt, wie z.B. Vaselinöl; Ester gesättigter oder ungesättigter Fettsäuren, wie Alkylmyristate, z.B. i-Propyl-, Butyl- oder Cetylmyristat, Hexadecylstearat, Ethyl- oder i-Propylpalmitat, Octan- oder Decansäuretriglyceride und Cetylricinoleat.

Die Fettphase kann auch in anderen Ölen lösliche Siliconöle, wie Dimethylpolysiloxan, Methylphenylpolysiloxan und das Silikonglycol-Copolymer, Fettsäuren und Fettalkohole enthalten.

Um die Retention von Ölen zu begünstigen, kann man auch Wachse verwenden, wie z.B. Camauba-Wachs, Candellilawachs, Bienenwachs, mikrokristallines Wachs, Ozokeritwachs und Ca-, Mg- und Al-Oleate, -Myristate, -Linoleate und -Stearate.

Im allgemeinen werden diese Wasser-in-Öl-Emulsionen so hergestellt, dass die Fettphase und der Emulgator in den Ansatzbehälter gegeben werden. Man erwärmt diesen bei einer Temperatur von 70 bis 75°C, gibt dann die in Öl löslichen Ingredienzen zu und fügt unter Rühren Wasser hinzu, welches vorher auf die gleiche Temperatur erwärmt wurde und worin man die wasserlöslichen Ingredienzen vorher gelöst hat; man rührt, bis man eine Emulsion der gewünschten Feinheit hat, lässt sie dann auf Raumtemperatur abkühlen, wobei gegebenenfalls weniger gerührt wird.

Weiterhin kann eine erfindungsgemäße Pflegeemulsion als O/W-Emulsion vorliegen. Eine derartige Emulsion enthält üblicherweise eine Ölphase, Emulgatoren, die die Ölphase in der Wasserphase stabilisieren, und eine wässrige Phase, die üblicherweise verdickt vorliegt.

Die wässrige Phase der O/W-Emulsion der erfindungsgemäßen Zubereitungen enthält gegebenenfalls

- Alkohole, Diole oder Polyole sowie deren Ether, vorzugsweise Ethanol, Isopropanol, Propylenglycol, Glycerin, Ethylenglycolmonoethylether;

- übliche Verdickungsmittel bzw. Gelbildner, wie zB. vernetzte Polyacrylsäuren und deren Derivate, Polysaccharide wie Xanthan Gum oder Alginate, Carboxymethylcellulose oder Hydroxycarboxymethylcellulose, Fettalkohole, Polyvinylalkohol und Polyvinylpyrrolidon.

Die Ölphase enthält in der Kosmetik übliche Ölkomponenten, wie beispielsweise:
- Ester aus gesättigten und/oder ungesättigten, verzweigten und/oder unverzweigten C₃-C₃₀₋Alkancarbonsäuren und gesättigten und/oder ungesättigten, verzweigten und/oder unverzweigten C₃-C₃₀-Alkoholen, aus aromatischen Carbonsäuren und gesättigten und/oder ungesättigten, verzweigten und/oder unverzweigten C₃-C₃₀-Alkoholen, beispielhaft Isopropylmyristat, Isopropylstearat, Hexyldecylstearat, Oleyloleat; außerdem synthetische, halbsynthetische und natürliche Gemische solcher Ester, wie Jojobaöl;
- verzweigte und/oder unverzweigte Kohlenwasserstoffe und -wachse;
- Silikonöle wie Cyclomethicon, Dimethylpolysiloxan, Diethylpolysiloxan, Octamethylcyclotetrasiloxan sowie Mischungen daraus;
- Dialkylether;
- Mineralöle und Mineralwachse;
- Triglyceride gesättigter und/oder ungesättigter, verzweigter und/oder unverzweigter C₈-C₂₄₋Alkancarbonsäuren; sie können ausgewählt werden aus synthetischen, halbsynthetischen oder natürlichen Ölen, wie Olivenöl, Palmöl, Mandelöl oder Mischungen.

Als Emulgatoren kommen vorzugsweise O/W-Emulgatoren, wie Polyglycerinester, Sorbitanester oder teilveresterte Glyceride, in Betracht.

Die Herstellung kann durch Aufschmelzen der Ölphase bei ca. 80°C erfolgen; die wasserlöslichen Bestandteile werden in heißem Wasser gelöst, langsam und unter Rühren zur Ölphase zugegeben; homogenisiert und kaltgerührt.

Die erfindungsgemäßen Dispersionen eignen sich auch zur Verwendung in Wasch- und Duschgel-Formulierungen sowie Badepräparaten.

Solche Formulierungen enthalten neben den erfindungsgemäßen Dispersionen üblicherweise anionische Tenside als Basistenside und amphotere und nichtionische Tenside als Cotenside, sowie Lipide, Parfümöle, Farbstoffe, organische Säuren, Konservierungsstoffe und Antioxidantien sowie Verdicker/Gelbildner, Hautkonditioniermittel und Feuchthaltemittel.

In den Wasch, Dusch- und Badepräparaten können alle in Körperreinigungsmitteln üblicherweise eingesetzte anionische, neutrale, amphotere oder kationische Tenside verwendet werden.

Die Formulierungen enthalten 2 bis 50 Gew.-% Tenside, bevorzugt 5 bis 40 Gew.-%, besonders bevorzugt 8 bis 30 Gew.-%.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Alkylglykolalkoxylate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxideinheiten im Molekül aufweisen.

Geeignet sind zum Beispiel Natriumlaurylsulfat, Ammoniumlaurylsulfat, Natriumlaurylethersulfat, Ammoniumlaurylethersulfat, Natriumlaurylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate- oder -propionate, Alkylamphodiacetate, oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60 Mole auf ein Mol Alkohol. Ferner sind Alkylaminoxide, Mono- oder Dialkylalkanolamide, Fettsäure-ester von Polyethylenglykolen, ethoxylierte Fettsäureamide, Alkylpolyglykoside oder Sorbitanetherester geeignet.

Außerdem können die Wasch, Dusch- und Badepräparate übliche kationische Tenside enthalten, wie z.B. quaternäre Ammoniumverbindungen, beispielsweise Cetyltrimethylammoniumchlorid oder-bromid (INCI Cetrimoniumchloride oder -bromide), Hydroxyethylcetyldimoniumphosphat (INCI Quaternium-44), INCI Cocotrimoniummethosulfate, INCI Quaternium-52.

Zusätzlich können auch weitere übliche kationische Polymere eingesetzt werden, so z.B. Copolymere aus Acrylamid und Dimethyldiallylammoniumchlorid (Polyquatemium-7), kationische Cellulosederivate (Polyquaternium-4, -10), kationische Stärkederivate (INCl: Starch Hydroxypropyltrimonium Chloride, Corn Starch Modified), kationische Guarderivate (INCl: Hydroxypropyl Guar Hydroxypropyltrimonium Chloride), kationische Sonnenblumenöl-Derivate (INCl: Sunflowerseedamidopropyl Hydroxyethyldimonium Chloride), Copolymere aus N-Vinylpyrrolidon und quaternisiertem N-Vinylimidazol (Polyquaternium-16, -44, -46), Copolymere aus N-Vinylpyrrolidon/Dimethylaminoethyl-methacrylat, quaternisiert mit Diethylsulfat (Polyquatemium-11), Copolymere aus Acrylsäure, Acrylamid und Methacrylamidopropyltrimoniumchlorid (Polyquaternium-53), Polyquatemium-32, Polyquaternium-28 und andere.

Weiterhin können die Wasch- und Duschgel-Formulierungen und Badepräparate Verdicker, wie z.B. Kochsalz, PEG-55, Propylene Glycol Oleate, PEG-120 Methyl Glucose Dioleate und andere, sowie Konservierungsmittel, weitere Wirk- und Hilfsstoffe und Wasser enthalten.

Haarkosmetische Zubereitungen umfassen insbesondere Stylingmittel und/oder Konditioniermittel in haarkosmetischen Zubereitungen wie Haarkuren, Haarschäume (engl. Mousses), (Haar)gelen oder Haarsprays, Haarlotionen, Haarspülungen, Haarshampoos, Haaremulsionen, Spitzenfluids, Egalisierungsmittel für Dauerwellen, Haarfärbe- und -bleichmittel, "Hot-Oil-Treatment"-Präparate, Conditioner, Festigerlotionen oder Haarsprays. Je nach Anwendungsgebiet können die haarkosmetischen Zubereitungen als (Aerosol-)Spray, (Aerosol-)Schaum, Gel, Gelspray, Creme, Lotion oder Wachs appliziert werden.

Die erfindungsgemäßen haarkosmetischen Formulierungen enthalten in einer bevorzugten Ausführungsform
a) 0,05 bis 20 Gew.-% der erfindungsgemäßen Dispersion
b) 20 bis 99,95 Gew.-% Wasser und/oder Alkohol
c) 0 bis 79,5 Gew.-% weitere Bestandteile

Unter Alkohol sind alle in der Kosmetik üblichen Alkohole zu verstehen, z.B. Ethanol, Isopropanol, n-Propanol.

Unter weiteren Bestandteilen sind die in der Kosmetik üblichen Zusätze zu verstehen, beispielsweise Treibmittel, Entschäumer, grenzflächenaktive Verbindungen, d.h. Tenside, Emulgatoren, Schaumbildner und Solubilisatoren. Die eingesetzten grenzflächenaktiven Verbindungen können anionisch, kationisch, amphoter oder neutral sein. Weitere übliche Bestandteile können ferner sein z.B. Konservierungsmittel, Parfümöle, Weichmacher, Effektstoffe, Trübungsmittel, Wirkstoffe, Antioxidantien, Peroxidzersetzer, UV-Filter, Pflegestoffe wie Panthenol, Collagen, Vitamine, Eiweißhydrolysate, Alpha- und Beta-Hydroxycarbonsäuren, Eiweißhydrolysate, Stabilisatoren, pH-Wert-Regulatoren, Farbstoffe, Pigmente, Viskositätsregulierer, Gelbildner, Salze, Feuchthaltemittel, Rückfetter, Komplexbildner und weitere übliche Additive.

Weiterhin zählen hierzu alle in der Kosmetik bekannten Styling- und Conditionerpolymere, die in Kombination mit den erfindungsgemäßen Polymerisaten eingesetzt werden können, falls ganz spezielle Eigenschaften eingestellt werden sollen.

Als herkömmliche Haarkosmetik-Polymere eignen sich beispielsweise anionische Polymere. Solche anionischen Polymere sind Homo- und Copolymerisate von Acrylsäure und Methacrylsäure oder deren Salze, Copolymere von Acrylsäure und Acrylamid und deren Salze; Natriumsalze von Polyhydroxycarbonsäuren, wasserlösliche oder wasserdispergierbare Polyester, Polyurethane (Luviset^{™} P.U.R.) und Polyharnstoffe. Besonders geeignete Polymere sind Copolymere aus t-Butylacrylat, Ethylacrylat, Methacrylsäure (z.B. Luvimer^{™} 100P), Copolymere aus N-tert.-Butylacrylamid, Ethylacrylat, Acrylsäure (z.B. Ultrahold^{™} 8, Strong), Copolymere aus Vinylacetat, Crotonsäure und gegebenenfalls weiteren Vinylestern (z.B. Luviset^{™} Marken, INCl: VA/Crotonates Copolymer), Maleinsäureanhydridcopolymere, ggf. mit Alkoholen umgesetzt, anionische Polysiloxane, z.B. carboxyfunktionelle, Copolymere aus Vinylpyrrolidon, t-Butylacrylat, Methacrylsäure (z.B Luviskol^{™} VBM).

Weiterhin umfasst die Gruppe der zur Kombination mit den erfindungsgemäßen Polymerisaten geeigneten Polymere beispielhaft Balance CR oder 0/55 (National Starch; Acrylatcopolymer), Balance 47 (National Starch; Octylacrylamid/Acrylat/Butylaminoethylmethacrylate-Copolymer), Aquaflex^{™} FX 64 (ISP; Isobutyfen/Ethytmaleimid/Hydroxyethylmaleimid-Copolymer), Aquaflex^{™} SF-40 (ISP / National Starch; VPNinyl Caprolactam/DMAPA Acrylatcopolymer), Allianz^{™} LT-120 (ISP / Rohm & Haas; Acrylat/C1-2 Succinat/Hydroxyacrylat-Copolymer), Aquarez^{™} HS (Eastman; Polyester-1 ), Diaformer^{™} Z-400 (Clariant; Methacryloylethylbetain/Methacrylat-Copolymer), Diaformer^{™} Z-711 oder Z-712 (Clariant; Methacryloylethyl N-oxid/Methacrylat-Copolymer), , Omnirez^{™} 2000 (ISP; Monoethylester von Poly(Methylvinylether/Maleinsäure in Ethanol), Amphomer^{™} HC oder Resyn XP oder Resyn 28-4961 (National Starch; Acrylat/Octylacrylamide Copolymer), Amphomer^{™} 28-4910 (National Starch; Octylacrylamid/Acrylat/Butylaminoethylmethacrylat-Copolymer), Advantage^{™} HC 37 (ISP; Terpolymer aus Vinylcapro-IactamNinylpyrrolidon/Dimethylaminoethylmethacrylat), Advantage Marken (ISP), Acudyne 258 (Rohm & Haas; Acrylat/ Hydroxyesteracrylat-Copolymer), Luviset^{™} P.U.R. (BASF, Polyurethane-1), Luviflex^{™} Silk (BASF, PEG/PPG-25/25 Dimethicone/Acrylates Copolymer), East-Man^{™} AQ48 (Eastman), Styleze 2000 (ISP; VP/Acrylates/Lauryl Methacrylate Copolymer), Styleze CC-10 (ISP; VP/DMAPA Acrylates Copolymer), Styleze W-20 (ISP), Fixomer A-30 (Ondeo Nalco; Methacrylic Acid/Sodium Acrylamidomethyl Propane Sulfonate Copolymer), Fixate G-100 (Noveon; AMP-Acrylates/Allyl Methacrylate Copolymer).

Ganz besonders bevorzugt werden als anionische Polymere Acrylate mit einer Säurezahl größer gleich 120 und Copolymere aus t-Butylacrylat, Ethylacrylat, Methacrylsäure.

Weitere geeignete Haarkosmetik-Polymere sind kationische Polymere mit der Bezeichnung Polyquaternium nach INCl, z.B. Copolymere aus Vinylpyrrolidon/N-Vinylimidazoliumsalzen (Luviquat^{™} FC, Luviquat^{™} HM, Luviquat^{™} MS, Luviquat^{™} Care, INCl: Polyquatemium-16, Polyquaternium-44), Copolymere aus N-Vinylpyrrolidon/Dimethylaminoethylmethacrylat, quaternisiert mit Diethylsulfat (Luviquat™ PQ 11, INCl: Polyquatemium-11), Copolymere aus N-Vinylcaprolactam N-Vinylpyrrolidon/N-Vinylimidazoliumsalzen (Luviquat™ Hold, INCl: Polyquatemium-46); Copolymere aus Acrylamid und Dimethyldiallylammoniumchlorid (Polyquaternium-7), kationische Cellulosederivate (Polyquaternium-4, -10), kationische Stärkederivate (IN-Cl: Starch Hydraxypropytrimonium Chloride, Corn Starch Modified), kationische Guarderivate (INCl: Hydroxypropyl Guar Hydroxypropyltrimonium Chloride), kationische Sonnenblumenöl-Derivate (INCl: Sunflowerseedamidopropyl Hydroxyethyldimonium Chloride), Copolymere aus Acrylsäure, Acrylamid und Methacrylamidopropyltrimoniumchlorid (INCI: Polyquaternium-53), Polyquaternium-32, Polyquaternium-28 und andere.

Als weitere Haarkosmetik-Polymere sind auch neutrale Polymere geeignet wie Polyvinylpyrrolidone, Copolymere aus N-Vinylpyrrolidon und Vinylacetat und/oder Vinylpropionat, Copolymere aus N-Vinypyrrolidon/Dimethylaminopropylacrylamid oder-methacrylamid, Copolymere aus N-Vinylpyrrolidon und Alkylacrylat- oder -methacrylatmonomeren mit Alkylketten von C1 bis C18, Pfropfcopolymere von Polyvinylalkohol auf Polyalkylenglykole wie z.B. Kollicoat IR (BASF), Pfropfcopolymere von anderen Vinylmonomeren auf Polyalkylenglykole, Polysiloxane, Polyvinylcaprolactam und Copolymere mit N-Vinylpyrrolidon, Polyethylenimine und deren Salze, Polyvinylamine und deren Salze, Cellulosederivate, Chitosan, Polyasparaginsäuresalze und Derivate.

Zur Einstellung bestimmter Eigenschaften können die Zubereitungen zusätzlich auch konditionierende Substanzen auf Basis von Silikonverbindungen enthalten. Geeignete Silikonverbindungen sind beispielsweise Polyalkylsiloxane, Polyarylsiloxane, Polyarylalkylsiloxane, Polyethersiloxane, Silikonharze, Dimethicone, Dimethicone-Derivate oder Dimethicon Copolyole (CTFA) und aminofunktionelle Silikonverbindungen wie Amodimethicone (CTFA).

Die erfindungsgemäßen Polymerisate eignen sich insbesondere als Festigungsmittel in Haarstyling-Zubereitungen, insbesondere Haarsprays (Aerosolsprays und Pumpsprays ohne Treibgas) und Haarschäume (Aerosolschäume und Pumpschäume ohne Treibgas).

In einer bevorzugten Ausführungsform enthalten diese Zubereitungen
a) 0,1 bis 10 Gew.-% der erfindungsgemäßen Dispersion
b) 20 bis 99,9 Gew.-% Wasser und/oder Alkohol
c) 0 bis 70 Gew.-% eines Treibmittel
d) 0 bis 20 Gew.-% weitere Bestandteile

Treibmittel sind die für Haarsprays oder Aerosolschäume üblich verwendeten Treibmittel. Bevorzugt sind Gemische aus Propan/Butan, Pentan, Dimethylether, 1,1-Difluorethan (HFC-152 a), Kohlendioxid, Stickstoff oder Druckluft.
Eine erfindungsgemäß bevorzugte Formulierung für Aerosolhaarschäume enthält
a) 0,1 bis 10 Gew.-% der erfindungsgemäßen Dispersion
b) 55 bis 99,8 Gew.-% Wasser und/oder Alkohol
c) 5 bis 20 Gew.-% eines Treibmittel
d) 0,1 bis 5 Gew.-% eines Emulgators
e) 0 bis 10 Gew.-% weitere Bestandteile

Als Emulgatoren können alle in Haarschäumen üblicherweise eingesetzten Emulgatoren verwendet werden. Geeignete Emulgatoren können nichtionisch, kationisch bzw. anionisch oder amphoter sein.

Beispiele für nichtionische Emulgatoren (INCl-Nomenklatur) sind Laurethe, z.B. Laureth-4; Cetethe, z.B. Cetheth-1, Polyethylenglycolcetylether; Cetearethe, z.B. Cetheareth-25, Polyglycolfettsäureglyceride, hydroxyliertes Lecithin, Lactylester von Fettsäuren, Alkylpolyglycoside.

Beispiele für kationische Emulgatoren sind Cetyltrimethylammoniumchlorid oder -bromid (INCl Cetrimoniumchloride oder -bromide), Hydroxyethyicetyldimoniumphosphat (INCl Quatemium-44), INCl Cocotrimoniummethosulfate, INCl Quaternium-52, Quaternium-1 bis x (INCl).

Anionische Emulgatoren können beispielsweise ausgewählt werden aus der Gruppe der Alkylsulfate, Alkylethersulfate, Alkylsulfonate, Alkylarylsulfonate, Alkylsuccinate, Alkylsulfosuccinate, N-Alkoyisarkosinate, Alkylglykolalkoxylate, Acyltaurate, Acylisethionate, Alkylphosphate, Alkyletherphosphate, Alkylethercarboxylate, Alpha-Olefinsulfonate, insbesondere die Alkali- und Erdalkalimetallsalze, z.B. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Beispiele für Haarschäume siehe Beispiele kosmetische Formulierungen von 38 bis 43.

Eine erfindungsgemäß für Styling-Gele geeignete Zubereitung kann beispielsweise wie folgt zusammengesetzt sein:
a) 0,1 bis 10 Gew.-% der erfindungsgemäßen Dispersion
b) 60 bis 99,85 Gew.-% Wasser und/oder Alkohol
c) 0,05 bis 10 Gew.-% eines Gelbildners
d) 0 bis 20 Gew.-% weitere Bestandteile
Als Gelbildner können alle in der Kosmetik üblichen Gelbildner eingesetzt werden. Hierzu zählen leicht vernetzte Polyacrylsäure, beispielsweise Carbomer (INCI), Cellulosederivate, z.B. Hydroxypropylcellulose, Hydroxyethylcellulose, kationisch modifizierte Cellulosen, Polysaccharide, z.B. Xanthum Gummi, Caprylic/Capric Triglyceride, Sodium acrylates Copolymer, Polyquaternium-32 (and) Paraffinum Liquidum (INCI), Sodium Acrylates Copolymer (and) Paraffinum Liquidum (and) PPG-1 Trideceth-6, Acrylamidopropyl Trimonium Chloride/Acrylamide Copolymer, Steareth-10 Allyl Ether Acrylates Copolymer, Polyquaternium-37 (and) Paraffinum Liquidum (and) PPG-1 Trideceth-6, Polyquaternium 37 (and) Propylene Glycole Dicaprate Dicaprylate (and) PPG-1 Trideceth-6, Polyquatemium-7, Polyquaternium-44.

Beispiele für Styling-Gele sind bei den Beispielen für kosmetische Zubereitungen von 44 bis 52 genannt.

Die erfindungsgemäßen Dispersionen können in kosmetischen Zubereitungen als Konditioniermittel eingesetzt werden. Beispiele für Rinse-off und Leave-on Conditioner sind die Nummern 53 bis 55.

Die erfindungsgemäßen Dispersionen können in kosmetischen Zubereitungen als Verdicker eingesetzt werden.

Die erfindungsgemäßen Dispersionen können auch in Shampooformulierungen als Festigungs- und/oder Konditioniermittel eingesetzt werden. Als Konditioniermittel eignen sich insbesondere Polymere mit kationischer Ladung. Bevorzugte Shampooformulierungen enthalten
a) 0,05 bis 10 Gew.-% der erfindungsgemäßen Dispersion
b) 25 bis 94,95 Gew.-% Wasser
c) 5 - 50 Gew.-% Tenside
c) 0 - 5 Gew.-% eines weiteren Konditioniermittels
d) 0 - 10 Gew.-% weitere kosmetische Bestandteile

In den Shampooformulierungen können alle in Shampoos üblicherweise eingesetzte anionische, neutrale, amphotere oder kationische Tenside verwendet werden.

Geeignete anionische Tenside sind beispielsweise Alkylsulfate, Alkylethersulfate, Alkylsulfonate, Alkylarylsulfonate, Alkylsuccinate, Alkylsulfosuccinate, N-Alkoylsarkosinate, Acyltaurate, Acylisethionate, Alkylglykolalkoxylate, Alkylphosphate, Alkyletherphosphate, Alkylethercarboxylate, Alpha-Olefinsulfonate, insbesondere die Alkali- und Erdalkalimetallsalze, zB. Natrium, Kalium, Magnesium, Calcium, sowie Ammonium- und Triethanolamin-Salze. Die Alkylethersulfate, Alkyletherphosphate und Alkylethercarboxylate können zwischen 1 bis 10 Ethylenoxid oder Propylenoxid-Einheiten, bevorzugt 1 bis 3 Ethylenoxid-Einheiten im Molekül aufweisen.

Geeignet sind zum Beispiel Natriumlaurylsulfat, Ammoniumlaurylsulfat, Natriumlaurylethersulfat, Ammoniumlaurylethersulfat, Natriumlauroylsarkosinat, Natriumoleylsuccinat, Ammoniumlaurylsulfosuccinat, Natriumdodecylbenzolsulfonat, Triethanolamindodecylbenzolsulfonat.

Geeignete amphotere Tenside sind zum Beispiel Alkylbetaine, Alkylamidopropylbetaine, Alkylsulfobetaine, Alkylglycinate, Alkylcarboxyglycinate, Alkylamphoacetate oder -propionate, Alkylamphodiacetate oder -dipropionate.

Beispielsweise können Cocodimethylsulfopropylbetain, Laurylbetain, Cocamidopropylbetain oder Natriumcocamphopropionat eingesetzt werden.

Als nichtionische Tenside sind beispielsweise geeignet die Umsetzungsprodukte von aliphatischen Alkoholen oder Alkylphenolen mit 6 bis 20 C-Atomen in der Alkylkette, die linear oder verzweigt sein kann, mit Ethylenoxid und/oder Propylenoxid. Die Menge Alkylenoxid beträgt ca. 6 bis 60, Mole auf ein Mol Alkohol. Ferner sind Alkylaminoxide, Mono- oder Dialkylalkanolamide, Fettsäureester von Polyethylenglykolen, Alkylpolyglykoside oder Sorbitanetherester geeignet.

Außerdem können die Shampooformulierungen übliche kationische Tenside enthalten, wie z.B. quaternäre Ammoniumverbindungen, beispielsweise Cetyltrimethylammoniumchlorid oder -bromid (INCI Cetrimoniumchloride oder-bromide), Hydroxyethylcetyldimoniumphosphat (INCI Quaternium-44), INCI Cocotrimoniummethosulfate, INCI Quaternium-52.

In den Shampooformulierungen können zur Erzielung bestimmter Effekte übliche Konditioniermittel in Kombination mit den erfindungsgemäßen Polymerisaten eingesetzt werden. Hierzu zählen beispielsweise kationische Polymere mit der Bezeichnung Polyquaternium nach INCI, z.B. Copolymere aus Vinylpyrrolidon/N-Vinylimidazoliumsalzen (Luviquat™ FC, Luviquat™ HM, Luviquat™ MS, Luviquat™ Care, INCI: Polyquaternium-16, Polyquaternium-44), Copolymere aus N-Vinylpyrrolidon/Dimethylaminoethylmethacrylat, quaternisiert mit Diethylsulfat (Luviquat™ PQ 11, INCI: Polyquaternium-11), Copolymere aus N-Vinylcaprolactam N-Vinylpyrrolidon/N-Vinylimidazoliumsalzen (Luviquat™ Hold, INCI: Polyquaternium-46); Copolymere aus Acrylamid und Dimethyldiallylammoniumchlorid (Polyquatemium-7), kationische Cellulosederivate (Polyquaternium-4, -10). Ferner können kationische Stärkederivate (INCI: Starch Hydroxypropyltrimonium Chloride, Corn Starch Modified), kationische Guarderivate (INCI: Hydroxypropyl Guar Hydroxypropyltrimonium Chloride), kationische Sonnenblumenöl-Derivate (INCI: Sunflowerseedamidopropyl Hydroxyethyldimonium Chloride), Copolymere aus Acrylsäure, Acrylamid und Methacrylamidopropyltrimoniumchlorid (INCI: Polyquaternium-53), Polyquaternium-32, Polyquaternium-28 und andere eingesetzt werden.Ferner können Eiweißhydrolysate verwendet werden, sowie konditionierende Substanzen auf Basis von Silikonverbindungen, beispielsweise Polyalkylsiloxane, Polyarylsiloxane, Polyarylalkylsiloxane, Polyethersiloxane oder Silikonharze. Weitere geeignete Silikonverbindungen sind Dimethicone, Dimethicone-Derivate oder Dimethicon Copolyole (CTFA) und aminofunktionelle Silikonverbindungen wie Amodimethicone (CTFA).

Beispiele für Shampoo- und Duschgelformulierungen sind von Nummer 59 bis 68 gegeben.

Herstellungsbeispiele (die Angabe des Feststoffgehaltes erfolgt in allen Beispielen als Gew.-%)

### Beispiel 1

In ein mit Ankerrührer (200 Upm) und Stickstoffeinleitung ausgestattetes 2-I-Glasgefäß wurden 575,7 g Wasser, 2 g Natriumdihydrogenphosphat-Dihydrat, 6 g Polyvinylpyrrolidon (K-Wert 90, bestimmt mit 1%iger wässriger Lösung), 10 g Polyvinylpyrrolidon (K-Wert 17, bestimmt mit 1%iger wässriger Lösung) sowie 180 g Polyethylenglykol der Molmasse 1500 eingewogen und durch Rühren zu einer homogenen Lösung verarbeitet. Man gab 180 g N-Vinylformamid, 44,4 g N-Vinyl-2-methylimmidazolium Methylsulfat (45% wässrige Lösung), und 0,6 g Triallylamin zu und stellte danach durch Zusatz von 25%iger wässriger Natronlauge den pH-Wert der Lösung auf 6,75 ein. Man leitete permanent Stickstoff durch die Reaktionsmischung und gab 1,0 g 2,2'-Azobis-2-(Aminopropan)dihydrochlorid (WakoV50) zu und erhitzte das Reaktionsgemisch zur Polymerisation auf eine Temperatur von 55°C. Die Polymerisationszeit betrug 4 Stunden. Danach wurde 0,24 g 2,2'-Azobis-2-(Aminopropan)dihydrochlorid (WakoVA44) zugegeben und weitere 2 Stunden bei 65°C polymerisiert.

Man erhielt eine wässrige Dispersion von einem Feststoffgehalt von 39,9 %, einer Viskosität von 650 mPas und einem LD-Wert (bei 39,9% Feststoffgehalt gemessen) von < 0,5%.

### Beispiel 2

In ein mit Ankerrührer (200 upm) und Stickstoffeinleitung ausgestattetes 2-1-Glasgefäß wurden 550,9 g Wasser, 2 g Natriumdihydrogenphosphat-Dihydrat, 6 g Polyvinylpyrrolidon (K-Wert 90, bestimmt mit 1%iger wässriger Lösung), 10 g Polyvinylpyrrolidon (K-Wert 17, bestimmt mit 1 %iger wässriger Lösung) sowie 180 g Polyethylenglykol der Molmasse 1500 eingewogen und durch Rühren zu einer homogenen Lösung verarbeitet. Man gab 160 g N-Vinylformamid, 88,9 g N-Vinyl-2-methylimmidazolium Methylsulfat (45% wässrige Lösung), und 1,0 g Triallylamin zu und stellte danach durch Zusatz von 25%iger wässriger Natronlauge den pH-Wert der Lösung auf 6,75 ein. Man leitete permanent Stickstoff durch die Reaktionsmischung und gab 1,0 g 2,2'-Azobis-2-(Aminopropan)dihydrochlorid (WakoV50) zu und erhitzte das Reaktionsgemisch zur Polymerisation auf eine Temperatur von 55°C. Die Polymerisationszeit betrug 4 Stunden. Danach wurde 0,24g 2,2'-Azobis-2-(Aminopropan)dihydrochlorid (WakoVA44) zugegeben und weitere 2 Stunden bei 65°C polymerisiert.

Man erhielt eine wässrige Dispersion von einem Feststoffgehalt von 39,9%, einer Viskosität von 5900 mPas und einem LD-Wert (bei 39,9% Feststoffgehalt gemessen) von < 0,5%.

### Beispiel 3

In ein mit Ankerrührer (200 upm) und Stickstoffeinleitung ausgestattetes 21-Glasgefäß wurden 575,7 g Wasser, 2 g Natriumdihydrogenphosphat-Dihydrat, 12 g Polyvinylpyrrolidon (K-Wert 90, bestimmt mit 1%iger wässriger Lösung), 10 g Polyvinylpyrrolidon (K-Wert 17, bestimmt mit 1 %iger wässriger Lösung) sowie 180 g Polyethylenglykol der Molmasse 1500 eingewogen und durch Rühren zu einer homogenen Lösung verarbeitet. Man gab 180 g N-Vinylformamid, 44,4 g N-Vinyl-2-methylimmidazolium Methylsulfat (45% wässrige Lösung), und 0,4 g Triallylamin zu und stellte danach durch Zusatz von 25%iger wässriger Natronlauge den pH-Wert der Lösung auf 6,75 ein. Man leitete permanent Stickstoff durch die Reaktionsmischung und gab 1,0 g 2,2'-Azobis-2-(Aminopropan)dihydrochlorid (WakoV50) zu und erhitzte das Reaktionsgemisch zur Polymerisation auf eine Temperatur von 55°C. Die Polymerisationszeit betrug 4 Stunden. Danach wurde 0,24g 2,2'-Azobis-2-(Aminopropan)dihydrochlorid (WakoVA44) zugegeben und weitere 2 Stunden bei 65°C polymerisiert.

Man erhielt eine wässrige Dispersion von einem Feststoffgehalt von 39,9%, einer Viskosität von 8600 mPas und einem LD-Wert (bei 39,9% Feststoffgehalt gemessen) von < 0,5%.

### Vergleichsbeispiel 1 (V1)

In ein mit Ankerrührer (200 upm) und Stickstoffeinleitung ausgestattetes 2-1-Glasgefäß wurden 775,7 g Wasser, 2 g Natriumdihydrogenphosphat-Dihydrat gegeben. Man gab 180 g N-Vinylformamid, 44,4 g N-Vinyl-2-methylimmidazolium Methylsulfat (45 % wässrige Lösung), und 0,6 g Triallylamin zu und stellte danach durch Zusatz von 25%iger wässriger Natronlauge den pH-Wert der Lösung auf 6,75 ein. Man leitete permanent Stickstoff durch die Reaktionsmischung und gab 1,0 g 2,2'-Azobis-2-(Aminopropan)dihydrochlorid (WakoV50) zu und erhitzte das Reaktionsgemisch zur Polymerisation auf eine Temperatur von 55°C. Die Polymerisationszeit betrug 4 Stunden. Danach wurde 0,24g 2,2-Azobis-2-(Aminopropan)dihydrochlorid (WakoVA44) zugegeben und weitere 2 Stunden bei 65°C polymerisiert. Man erhielt eine Lösung mit einem Feststoffgehalt 20,1%. Die Lösung hatte eine Viskosität von großer 75000 mPas und einen LD-Wert (bei 20,1% Feststoffgehalt gemessen) von 95 %.

Vergleichsbeispiel 2 (V2)

In ein mit Ankerrührer (200 Upm) und Stickstoffeinleitung ausgestattetes 21-Glasgefäß wurden 746,9 g Wasser, 2 g Natriumdihydrogenphosphat-Dihydrat, zu einer homogenen Lösung verarbeitet. Man gab 160 g N-Vinylformamid, 88,9 g N-Vinyl-2-methylimmidazolium Methylsulfat (45 % wässrige Lösung), und 1,0 g Triallylamin zu und stellte danach durch Zusatz von 25%iger wässriger Natronlauge den pH-Wert der Lösung auf 6,75 ein. Man leitete permanent Stickstoff durch die Reaktionsmischung und gab 1,0 g 2,2'-Azobis-2-(Aminopropan)dihydrochlorid (WakoV50) zu und erhitzte das Reaktionsgemisch zur Polymerisation auf eine Temperatur von 55°C. Die Polymerisationszeit betrug 4 Stunden. Danach wurde 0,24g 2,2'-Azobis-2-(Aminopropan)dihydrochlorid (WakoVA44) zugegeben und weitere 2 Stunden bei 65°C polymerisiert. Man erhielt eine wässrige Lösung mit einem Feststoffgehalt von 20%. Die wässrige Lösung hatte eine Viskosität von großer 75000 mPas und einem LD-Wert (bei 20% Feststoffgehalt gemessen) von 93 %.

### Vergleichsbeispiel 3 (V3)

In ein mit Ankerrührer (200 upm) und Stickstoffeinleitung ausgestattetes 2-1-Glasgefäß wurden 575,7 g Wasser, 2 g Natriumdihydrogenphosphat-Dihydrat, 12 g Polyvinylpyrrolidon (K-Wert 90, bestimmt mit 1%iger wässriger Lösung), 10 g Polyvinylpyrrolidon (K-Wert 17, bestimmt mit 1 %iger wässriger Lösung) sowie 180 g Polyethylenglykol der Molmasse 1500 eingewogen und durch Rühren zu einer homogenen Lösung verarbeitet. Man gab 180 g N-Vinylformamid, 44,4 g N-Vinyl-2-methylimmidazolium Methylsulfat (45% wässrige Lösung), zu und stellte danach durch Zusatz von 25%iger wässriger Natronlauge den pH-Wert der Lösung auf 6,75 ein. Man leitete permanent Stickstoff durch die Reaktionsmischung und gab 1,0 g 2,2'-Azobis-2-(Aminopropan)dihydrochlorid (WakoV50) zu und erhitzte das Reaktionsgemisch zur Polymerisation auf eine Temperatur von 55°C. Die Polymerisationszeit betrug 4 Stunden. Danach wurde 0,24 g 2,2'-Azobis-2-(Aminopropan)dihydrochlorid (WakoVA44) zugegeben und weitere 2 Stunden bei 65°C polymerisiert. Man erhielt eine wässrige Dispersion von einem Feststoffgehalt von 39,9 %, einer Viskosität von 2600 mPas und einem LD-Wert (bei 39,9 % Feststoffgehalt gemessen) von 90%.

### Beispiel 4

In ein mit Ankerrührer (200 Upm) und Stickstoffeinleitung ausgestattetes 2-1-Glasgefäß wurden 583 g Wasser, 2 g Natriumdihydrogenphosphat-Dihydrat, 6 g Polyvinylpyrrolidon (K-Wert 90, bestimmt mit 1%iger wässriger Lösung), 6 g Polyvinylpyrrolidon (K-Wert 17, bestimmt mit 1 %iger wässriger Lösung) sowie 180 g Polyethylenglykol der Molmasse 1500 eingewogen und durch Rühren zu einer homogenen Lösung verarbeitet. Man gab 160 g N-Vinylformamid, 61,5 g Diallyldimethylammoniumchlorid (65% wässrige Lösung), und 0,2 g Triallylamin zu und stellte danach durch Zusatz von 25%iger wässriger Natronlauge den pH-Wert der Lösung auf 6,75 ein. Man leitete permanent Stickstoff durch die Reaktionsmischung und gab 2,0 g 2,2'-Azobis-2-(Aminopropan)dihydrochlorid (WakoV50) zu und erhitzte das Reaktionsgemisch zur Polymerisation auf eine Temperatur von 55°C. Die Polymerisationszeit betrug 4 Stunden. Danach wurde 0,24g 2,2'-Azobis-2-(Aminopropan)dihydrochlorid (WakoVA44) zugegeben und weitere 4 Stunden bei 65°C polymerisiert. Man erhielt eine wässrige Dispersion von einem Feststoffgehalt von 39,5%, einer Viskosität von 950 mPas und einem LD-Wert (bei 39,5% Feststoffgehalt gemessen) von < 0,5.

### Beispiel 5

In ein mit Ankerrührer (200 upm) und Stickstoffeinleitung ausgestattetes 2I-Glasgefäß wurden 575,7 g Wasser, 2 g Natriumdihydrogenphosphat-Dihydrat, 6 g Polyvinylpyrrolidon (K-Wert 90, bestimmt mit 1%iger wässriger Lösung), 10 g Polyvinylpyrrolidon (K-Wert 17, bestimmt mit 1%iger wässriger Lösung) sowie 180 g Polyethylenglykol der Molmasse 1500 eingewogen und durch Rühren zu einer homogenen Lösung verarbeitet. Man gab 180 g N-Vinylformamid, 44,4 g N-Vinyl-2-methylimmidazolium Methylsulfat (45% wässrige Lösung), und 0,6 g Triallylamin zu und stellte danach durch Zusatz von 25%iger wässriger Natronlauge den pH-Wert der Lösung auf 6,75 ein. Man leitete permanent Stickstoff durch die Reaktionsmischung und gab 0,4 g 2,2'-Azobis-2-(Aminopropan)dihydrochlorid (WakoV50) zu und erhitzte das Reaktionsgemisch zur Polymerisation auf eine Temperatur von 50°C. Die Polymerisationszeit betrug 4 Stunden. Danach wurde 0,24 g 2,2'-Azobis-2-(Aminopropan)dihydrochlorid (WakoVA44) zugegeben und weitere 2 Stunden bei 65°C polymerisiert. Man erhielt eine wässrige Dispersion von einem Feststoffgehalt von 39,9 %, einer Viskosität von 1100 m Pas und einem LD-Wert (bei 39,9 % Feststoffgehalt gemessen) von < 0,5 %.

### Beispiel 6

In ein mit Ankerrührer (200 Upm) und Stickstoffeinleitung ausgestattetes 2-I-Glasgefäß wurden 579,9 g Wasser, 2 g Natriumdihydrogenphosphat-Dihydrat, 6 g Polyvinylpyrrolidon (K-Wert 90, bestimmt mit 1%iger wässriger Lösung), 6 g Polyvinylpyrrolidon (K-Wert 17, bestimmt mit 1%iger wässriger Lösung) sowie 180 g Polyethylenglykol der Molmasse 1500 eingewogen und durch Rühren zu einer homogenen Lösung verarbeitet. Man gab 140 g N-Vinylformamid, 40 g Vinylpyrrolidon, 44,4 g N-Vinyl-2-methylimmidazolium Methylsulfat (45 % wässrige Lösung), und 0,4 g Triallylamin zu und stellte danach durch Zusatz von 25%iger wässriger Natronlauge den pH-Wert der Lösung auf 6,75 ein. Man leitete permanent Stickstoff durch die Reaktionsmischung und gab 1,0 g 2,2'-Azobis-2-(Aminopropan)dihydrochlorid (WakoV50) zu und erhitzte das Reaktionsgemisch zur Polymerisation auf eine Temperatur von 55°C. Die Polymerisationszeit betrug 4 Stunden. Danach wurde 0,24g 2,2'-Azobis-2-(Aminopropan)dihydrochlorid (WakoVA44) zugegeben und weitere 2 Stunden bei 65°C polymerisiert.

Man erhielt eine wässrige Dispersion von einem Feststoffgehalt von 39,9 %, einer Viskosität von 1100 mPas und einem LD-Wert (bei 39,9 % Feststoffgehalt gemessen) von < 0,5 %.

### Beispiel 7

In ein mit Ankerrührer (200 Upm) und Stickstoffeinleitung ausgestattetes 2-I-Glasgefäß wurden 583 g Wasser, 2 g Natriumdihydrogenphosphat-Dihydrat, 6 g Polyvinylpyrrolidon (K-Wert 90, bestimmt mit 1%iger wässriger Lösung), 6 g Polyvinylpyrrolidon (K-Wert 17, bestimmt mit 1 %iger wässriger Lösung) sowie 180 g Polyethylenglykol der Molmasse 1500 eingewogen und durch Rühren zu einer homogenen Lösung verarbeitet. Man gab 160 g N-Vinylformamid, 61,5 g Diallyldimethylammonium chlorid (65% wässrige Lösung), und 0,2 g Triallylamin zu und stellte danach durch Zusatz von 25%iger wässriger Natronlauge den pH-Wert der Lösung auf 6,75 ein. Man leitete permanent Stickstoff durch die Reaktionsmischung und gab 1,5g 2,2'-Azobis-2-(Aminopropan)dihydrochlorid (WakoV50) zu und erhitzte das Reaktionsgemisch zur Polymerisation auf eine Temperatur von 55°C. Die Polymerisationszeit betrug 4 Stunden. Danach wurde 0,24g 2,2'-Azobis-2-(Aminopropan)dihydrochlorid (WakoVA44) zugegeben und weitere 4 Stunden bei 65°C polymerisiert.

Man erhielt eine wässrige Dispersion von einem Feststoffgehalt von 39,5 %, einer Viskosität von 2100 mPas und einem LD-Wert (bei 39,5 % Feststoffgehalt gemessen) von < 0,5 %.

### Beispiel 8

In ein mit Ankerrührer (200 Upm) und Stickstoffeinleitung ausgestattetes 2-I-Glasgefäß wurden 550,9 g Wasser, 2 g Natriumdihydrogenphosphat-Dihydrat, 6 g Polyvinylpyrrolidon (K-Wert 90, bestimmt mit 1%iger wässriger Lösung), 10 g Perfectamyl NR (kationische Stärke) sowie 180 g Polyethylenglykol der Molmasse 1500 eingewogen und durch Rühren zu einer homogenen Lösung verarbeitet. Man gab 180 g N-Vinylformamid, 44,4 g N-Vinyl-2-methylimmidazolium Methylsulfat (45% wässrige Lösung), und 0,6 g Triallylamin zu und stellte danach durch Zusatz von 25%iger wässriger Natronlauge den pH-Wert der Lösung auf 6,75 ein. Man leitete permanent Stickstoff durch die Reaktionsmischung und gab 1,0 g 2,2'-Azobis-2-(Aminopropan)dihydrochlorid (WakoV50) zu und erhitzte das Reaktionsgemisch zur Polymerisation auf eine Temperatur von 55°C. Die Polymerisationszeit betrug 4 Stunden. Danach wurde 0,24g 2,2'-Azobis-2-(Aminopropan)dihydrochlorid (WakoVA44) zugegeben und weitere 2 Stunden bei 65°C polymerisiert.

Man erhielt eine wässrige Dispersion von einem Feststoffgehalt von 39,9 %, einer Viskosität von 1900 mPas und einem LD-Wert (bei 39,9 % Feststoffgehalt gemessen) von < 0,5.

### Beispiel 9

In ein mit Ankerrührer (200 Upm) und Stickstoffeinleitung ausgestattetes 2-1-Glasgefäß wurden 550,9 g Wasser, 2 g Natriumdihydrogenphosphat-Dihydrat, 6 g Polyvinylpyrrolidon (K-Wert 90, bestimmt mit 1%iger wässriger Lösung), 25 g einer 40%iger Luviquat FC 370 Lösung (Copolymer aus Vinylpyrrolidon und Vinyl-2-methylimmidazolium methylsulfat : 7:3, K-Wert ca. 40 bestimmt mit 1%iger wässriger Lösung) sowie 180 g Polyethylenglykol der Molmasse 1500 eingewogen und durch Rühren zu einer homogenen Lösung verarbeitet. Man gab 180 g N-Vinylformamid, 44,4 g N-Vinyl-2-methylimmidazolium Methylsulfat (45% wässrige Lösung), und 0,6 g Triallylamin zu und stellte danach durch Zusatz von 25%iger wässriger Natronlauge den pH-Wert der Lösung auf 6,75 ein. Man leitete permanent Stickstoff durch die Reaktionsmischung und gab 1,0 g 2,2'-Azobis-2-(Aminopropan)dihydrochlorid (WakoV50) zu und erhitzte das Reaktionsgemisch zur Polymerisation auf eine Temperatur von 55°C. Die Polymerisationszeit betrug 4 Stunden. Danach wurde 0,24 g 2,2'-Azobis-2-(Aminopropan)dihydrochlorid (WakoVA44) zugegeben und weitere 2 Stunden bei 65°C polymerisiert.

Man erhielt eine wässrige Dispersion von einem Feststoffgehalt von 39,9 %, einer Viskosität von 1450 mPas und einem LD-Wert (bei 39,9 % Feststoffgehalt gemessen) von < 0,5.

### Beispiel 10

In ein mit Ankerrührer (200 Upm) und Stickstoffeinleitung ausgestattetes 2-1-Glasgefäß wurden 550,9 g Wasser, 2 g Natriumdihydrogenphosphat-Dihydrat, 6 g Polyvinylpyrrolidon (K-Wert 90, bestimmt mit 1%iger wässriger Lösung), 25 g einer 40%iger Luviquat FC 905 Lösung (Copolymer aus Vinylpyrrolidon und Vinyl-2-methylimmidazoliummethylsulfat : 5:95, K-Wert ca. 40 bestimmt mit 1%iger wässriger Lösung) sowie 180 g Polyethylenglykol der Molmasse 1500 eingewogen und durch Rühren zu einer homogenen Lösung verarbeitet. Man gab 180 g N-Vinylformamid, 44,4 g N-Vinyl-2-methylimmidazolium Methylsulfat (45% wässrige Lösung), und 0,6 g Triallylamin zu und stellte danach durch Zusatz von 25%iger wässriger Natronlauge den pH-Wert der Lösung auf 6,75 ein. Man leitete permanent Stickstoff durch die Reaktionsmischung und gab 1,0 g 2,2'-Azobis-2-(Aminopropan)dihydrochlorid (WakoV50) zu und erhitzte das Reaktionsgemisch zur Polymerisation auf eine Temperatur von 55°C. Die Polymerisationszeit betrug 4 Stunden. Danach wurde 0,24 g 2,2'-Azobis-2-(Aminopropan)-dihydrochlorid (WakoVA44) zugegeben und weitere 2 Stunden bei 65°C polymerisiert.

Man erhielt eine wässrige Dispersion von einem Feststoffgehalt von 39,9 %, einer Viskosität von 1100 m Pas und einem LD-Wert (bei 39,9 % Feststoffgehalt gemessen) von < 0,5.

### Beispiel 11 (Zulauffahrweise)

In ein mit Ankerrührer (200 Upm) und Stickstoffeinleitung ausgestattetes 2-1-Glasgefäß wurden 417,9 g Wasser, 2 g Natriumdihydrogenphosphat-Dihydrat, 6 g Polyvinylpyrrolidon (K-Wert 90, bestimmt mit 1 %iger wässriger Lösung), 10 g Polyvinylpyrrolidon (K-Wert 17, bestimmt mit 1%iger wässriger Lösung) sowie 180 g Polyethylenglykol der Molmasse 1500 eingewogen und durch Rühren zu einer homogenen Lösung verarbeitet. Man gab 160 g N-Vinylformamid, 88,9 g N-Vinyl-2-methylimmidazolium Methylsulfat (45% wässrige Lösung), und 1,0 g Triallylamin zu und stellte danach durch Zusatz von 25%iger wässriger Natronlauge den pH-Wert der Lösung auf 6,75 ein. Man leitete permanent Stickstoff durch die Reaktionsmischung und erhitzte das Reaktionsgemisch zur Polymerisation auf eine Temperatur von 55°C. 1,0 g 2,2'-Azobis-2-(Aminopropan)dihydrochlorid (WakoV50) in 100 ml Wasser wurde innerhalb von 3 Stunden zugegeben. Danach wurde weiterhin 3 Stunden polymerisiert. Danach wurde noch 0,3 g 2,2'-Azobis-2-(Aminopropan)dihydrochlorid (WakoV50) in 33 ml Wasser in 15 Minuten zugegeben und weitere 2 Stunden bei 75°C polymerisiert. Man erhielt eine wässrige Dispersion von einem Feststoffgehalt von 39,9 %, einer Viskosität von 3400 mPas und einem LD-Wert (bei 39,9 % Feststoffgehalt gemessen) von < 0,5 %.

### Beispiel 12

In ein mit Ankerrührer (200 Upm) und Stickstoffeinleitung ausgestattetes 2-I-Glasgefäß wurden 583 g Wasser, 2 g Natriumdihydrogenphosphat-Dihydrat, 6 g Polyvinylpyrrolidon (K-Wert 90, bestimmt mit 1%iger wässriger Lösung), 6 g Polyvinylpyrrolidon (K-Wert 17, bestimmt mit 1%iger wässriger Lösung) sowie 130 g Polyethylenglykol der Molmasse 1500 und 50 g Polyethylenglykol der Molmasse 4000 eingewogen und durch Rühren zu einer homogenen Lösung verarbeitet. Man gab 160 g N-Vinylformamid, 61,5 g Diallyldimethylammonium chlorid (65% wässrige Lösung), und 0,2 g Triallylamin zu und stellte danach durch Zusatz von 25%iger wässriger Natronlauge den pH-Wert der Lösung auf 6,75 ein. Man leitete permanent Stickstoff durch die Reaktionsmischung und gab 1,5g 2,2'-Azobis-2-(Aminopropan)dihydrochlorid (WakoV50) zu und erhitzte das Reaktionsgemisch zur Polymerisation auf eine Temperatur von 55°C. Die Polymerisationszeit betrug 4 Stunden. Danach wurde 0,24 g 2,2'-Azobis-2-(Aminopropan)-dihydrochlorid (WakoVA44) zugegeben und weitere 4 Stunden bei 65°C polymerisiert.

Man erhielt eine wässrige Dispersion von einem Feststoffgehalt von 39,5 %, einer Viskosität von 4600 mPas und einem LD-Wert (bei 39,5 % Feststoffgehalt gemessen) von < 0,5.

### Beispiel 13

In ein mit Ankerrührer (200 Upm) und Stickstoffeinleitung ausgestattetes 2-I-Glasgefäß wurden 433 g Wasser, 2 g Natriumdihydrogenphosphat-Dihydrat, 6 g Polyvinylpyrrolidon (K-Wert 90, bestimmt mit 1%iger wässriger Lösung), 6 g Polyvinylpyrrolidon (K-Wert 17, bestimmt mit 1 %iger wässriger Lösung) sowie 130 g Polyethylenglykol der Molmasse 1500 eingewogen und durch Rühren zu einer homogenen Lösung verarbeitet. Man gab 160 g N-Vinylformamid, 61,5 g Diallyldiemthylammonium chlorid (65% wässrige Lösung), und 0,2 g Triallylamin zu und stellte danach durch Zusatz von 25%iger wässriger Natronlauge den pH-Wert der Lösung auf 6,75 ein. Man leitete permanent Stickstoff durch die Reaktionsmischung und gab 1,5g 2,2'-Azobis-2-(Aminopropan)dihydrochlorid (WakoV50) zu und erhitzte das Reaktionsgemisch zur Polymerisation auf eine Temperatur von 55°C. Die Polymerisationszeit betrug 4 Stunden. Während die Polymerisation wurde nach 2 Stunden 50 g Polyethylenglykol der Molmasse 1500 in 150 g Wasser innerhalb 2 Stunden zugegeben. Danach wurde 0,24g 2,2'-Azobis-2-(Aminopropan)-dihydrochlorid (WakoVA44) zugegeben und weitere 4 Stunden bei 75°C polymerisiert. Man erhielt eine wässrige Dispersion von einem Feststoffgehalt von 39,5 %, einer Viskosität von 2600 mPas und einem LD-Wert (bei 39,5 % Feststoffgehalt gemessen) von < 0,5 %.

### Beispiel 14

In ein mit Ankerrührer (200 Upm) und Stickstoffeinleitung ausgestattetes 2-I-Glasgefäß wurden 575,7 g Wasser, 2 g Natriumdihydrogenphosphat-Dihydrat, 6 g Polyvinylpyrrolidon (K-Wert 90, bestimmt mit 1%iger wässriger Lösung), 10 g Polyvinylpyrrolidon (K-Wert 17, bestimmt mit 1 %iger wässriger Lösung) sowie 180 g Polyethylenglykol der Molmasse 1500 eingewogen und durch Rühren zu einer homogenen Lösung verarbeitet. Man gab 30 g N-Vinylformamid, 7,4 g N-Vinyl-2-methylimmidazolium Methylsulfat (45% wässrige Lösung), und 0,6 g Triallylamin zu und stellte danach durch Zusatz von 25%iger wässriger Natronlauge den pH-Wert der Lösung auf 6,75 ein. Man leitete permanent Stickstoff durch die Reaktionsmischung und gab 1 g 2,2'-Azobis-2-(Aminopropan)dihydrochlorid (WakoV50) zu und erhitzte das Reaktionsgemisch zur Polymerisation auf eine Temperatur von 50°C. Die Polymerisationszeit betrug 6 Stunden. Während der Polymerisation wurde weitere 150 g N-Vinylformamid, 37 g N-Vinyl-2-methylimmidazolium Methylsulfat (45% wässrige Lösung) in der erste 3 Stunden der Polymerisationszeit zugelaufen. Danach wurde 0,4 g 2,2'-Azobis-2-(Aminopropan)dihydrochlorid (WakoVA44) zugegeben und weitere 2 Stunden bei 65°C polymerisiert.

Man erhielt eine wässrige Dispersion von einem Feststoffgehalt von 39,9 %, einer Viskosität von 6500 mPas und einem LD-Wert (bei 39,9 % Feststoffgehalt gemessen) von < 0,5.

### Beispiel 15

In ein mit Ankerrührer (200 upm) und Stickstoffeinleitung ausgestattetes 2-I-Glasgefäß wurden 550,9 g Wasser, 2 g Natriumdihydrogenphosphat-Dihydrat, 8 g Polyethylenglykol der Molmasse 35000, und 180 g Polyethylenglykol der Molmasse 1500 eingewogen und durch Rühren zu einer homogenen Lösung verarbeitet. Man gab 180 g N-Vinylformamid, 44,4 g N-Vinyl-2-methylimmidazolium Methylsulfat (45% wässrige Lösung), und 0,6 g Triallylamin zu und stellte danach durch Zusatz von 25%iger wässriger Natronlauge den pH-Wert der Lösung auf 6,75 ein. Man leitete permanent Stickstoff durch die Reaktionsmischung und gab 1,0 g 2,2'-Azobis-2-(Aminopropan)dihydrochlorid (WakoV50) zu und erhitzte das Reaktionsgemisch zur Polymerisation auf eine Temperatur von 55°C. Die Polymerisationszeit betrug 4 Stunden. Danach wurde 0,24 g 2,2'-Azobis-2-(Aminopropan)dihydrochlorid (WakoVA44) zugegeben und weitere 2 Stunden bei 65°C polymerisiert.

Man erhielt eine wässrige Dispersion von einem Feststoffgehalt von 39,9 %, einer Viskosität von 1900 mPas und einem LD-Wert (bei 39,9 % Feststoffgehalt gemessen) von < 0,5.

### Beispiel 16

In ein mit Ankerrührer, Stickstoffeinleitung, Destillationsbrücke und Vakuumregulierung ausgestatteten 2-1-Glasgefäß wurden 800 g Wasser, 5 g Natriumdihydrogenphosphat-Dihydrat, 150 g Polyvinylpyrrolidon (K-Wert 30, bestimmt mit 1%iger wässriger Lösung) sowie 150 g Polyethylenglykol der Molmasse 1500 eingewogen und durch Rühren zu einer homogenen Lösung verarbeitet. Man gab 400 g N-Vinylformamid, 155 g Diallyldimethylammoniumchlorid (65 % wässrige Lösung) und 2,0 g Pentaerytrittriallylether zu und stellte danach durch Zusatz von 25%iger wässriger Natronlauge den pH-Wert der Lösung auf 6,5 ein. Man leitete permanent Stickstoff durch die Reaktionsmischung und gab eine Lösung von 2,5g 2,2'-Azobis-2-(Aminopropan)-dihydro-chlorid in 100 g Wasser zu und erhitzte das Reaktionsgemisch zur Polymerisation auf eine Temperatur von 50°C. Die Polymerisation wurde bei dieser Temperatur und einem Druck von 130 mbar durchgeführt, wobei die entstehende Polymerisationswärme durch Siedekühlung abgeführt wurde. Die Polymerisationszeit betrug 3 Stunden. Innerhalb dieser Zeit wurde so viel Wasser abdestilliert, dass man eine wässrige Dispersion mit einem Feststoffgehalt von 44 % erhielt. Sie hatte eine Viskosität von 4800 mPas und einen LD-Wert von < 0,5 % (gemessen bei 44 % Feststoffgehalt).
Verwendete Abkürzungen
VFA Vinylformamid
QVI quarternisiertes Vinylimidazol
Pluriol E INCI Polyethylenglykol
Kollidon 90 F INCI PVP
Kollidon 17 PF INCI PVP

Herstellbeispiele von (Meth)Acrylamid-haltigen Dispersionen
Abkürzungen
PVP Polyvinylpyrrolidon (Luviskol K 30)
EDTA Ethylendiamintetraessigsäure
PEG Polyethylenglykol
AM Acrylamid
DADMAC Diallyldimethylammoniumchlorid
AS Acrylsäure
DMA3*MeCl mit CH₃Cl quarternisiertes Dimethylaminoethylacrylat
QVI mit Dimethylsulfat quaternisiertes Vinylimidazol
V-50^{™} 2,2'-Azobis(2-methylpropionamid)dihydrochlorid
V A-044^{™} 2,2'-Azobis[2-(2-imidazolin-2-yl)propan]dihydrochlorid

### Beispiel (M)A 1

90 g einer 30 gew.-%igen wässrigen PVP-Lösung und 0.1 g einer 40 gew.-%igen wässrigen Lösung von EDTA wurden in 275 g Wasser gelöst und 120 g PEG (Pluriol^{™} E 1500) wurden hinzugefügt. Der pH-Wert wurde mit Triethanolamin auf 6.75 eingestellt und die Emulsion mit Stickstoffgas für 10 Minuten gespült.
Dann wurde eine Mischung aus 348 g einer 50 gew.-%igen wässrigen Lösung von AM, 133.1 g einer 45 gew.-%igen wässrigen Lösung von QVI während 10 Minuten hinzugegeben.
Danach wurden 0.23 g des Radikalstarters V-50^{™} hinzugegeben und die Reaktionsmischung unter Stickstoffatmosphäre auf 50°C erwärmt. Nachdem die Mischung bei dieser Temperatur für 4 Stunden gerührt worden war, wurden 0,25 g des Radikalstarters VA-044^{™} zugegeben und die Mischung für weitere 2 Stunden bei 60°C gerührt und dann auf Raumtemperatur abgekühlt.
Man erhielt eine weisse Dispersion mit einer Viskosität von 670 mPa*s.

### Beispiel (M)A 2

135 g einer 30 gew.-%igen wässrigen PVP-Lösung und 0.1 g einer 40 gew.-%igen wässrigen Lösung von EDTA wurden in 255 g Wasser gegeben und 105 g PEG (Pluriol^{™} E 1500) wurden hinzugefügt. Der pH-Wert wurde mit Triethanolamin auf 6.75 eingestellt und die Emulsion mit Stickstoffgas für 10 Minuten gespült.
Dann wurde eine Mischung aus 348 g einer 50 gew.-%igen wässrigen Lösung von AM und 72.5 g einer 60.7 gew.-%igen wässrigen Lösung von DADMAC während 10 Minuten hinzugegeben.
Danach wurden 0.1 g des Radikalstarters V-50^{™} in 50 g Wasser während 2 Stunden kontinuierlich hinzugegeben und die Reaktionsmischung unter Stickstoffatmosphäre auf 60°C erwärmt. Nachdem die Mischung bei dieser Temperatur für 2 Stunden gerührt worden war, wurden 0.1 g des Radikalstarters VA-044^{™} zugegeben und die Mischung für eine Stunde bei 70°C gerührt und dann auf Raumtemperatur abgekühlt.
Man erhielt eine weisse Dispersion mit einer Viskosität von 1520 mPa*s.

### Beispiel (M)A 3

90 g einer 30 gew.-%igen wässrigen PVP-Lösung und 0.1 g einer 40 gew.-%igen wässrigen Lösung von EDTA wurden in 275 g Wasser gegeben und 120 g PEG (Pluriol^{™} E 1500) wurden hinzugefügt. Der pH-Wert wurde mit Triethanolamin auf 6.75 eingestellt und die Emulsion mit Stickstoffgas für 10 Minuten gespült.
Dann wurde eine Mischung aus 348 g einer 50 gew.-%igen wässrigen Lösung von AM und 133.1 g einer 45 gew.-%igen wässrigen Lösung von QVI während 10 Minuten hinzugegeben. Danach wurden 0,1 g des Radikalstarters V-50^{™} hinzugegeben und die Reaktionsmischung unter Stickstoffatmosphäre auf 60°C erwärmt. Nachdem die Mischung bei dieser Temperatur für 4 Stunden gerührt worden war, wurden 0,1 g des Radikalstarters VA-044^{™} zugegeben und die Mischung für eine Stunde bei 60°C gerührt und dann auf Raumtemperatur abgekühlt.
Man erhielt eine weisse Dispersion mit einer Viskosität von 410 mPa*s.

### Beispiel (M)A 4

90 g einer 30 gew.%igen wässrigen PVP-Lösung und 0.1 g einer 40 gew.-%igen wässrigen Lösung von EDTA und 30 g NaCl wurden in 275 g Wasser gelöst und 120 g PEG (Pluriol^{™} E 1500) wurden hinzugefügt. Der pH-Wert wurde mit Triethanolamin auf 6.75 eingestellt und die Emulsion mit Stickstoffgas für 10 Minuten gespült.

Dann wurde eine Mischung aus 313.5 g einer 50 gew.-%igen wässrigen Lösung von AM, 133.1 g einer 45 gew.-%igen wässrigen Lösung von QVI und 17.7 g AS während 10 Minuten hinzugegeben.
Danach wurden 0,1 g des Radikalstarters V-50™ hinzugegeben und die Reaktionsmischung unter Stickstoffatmosphäre auf 60°C erwärmt. Nachdem die Mischung bei dieser Temperatur für 4 Stunden gerührt worden war, wurden 0,1 g des Radikalstarters VA-044^{™} zugegeben und die Mischung für eine weitere Stunde bei 60°C gerührt und dann auf Raumtemperatur abgekühlt. Man erhielt eine weisse Dispersion mit einer Viskosität von 550 mPa*s.

### Beispiel (M)A 5

90 g einer 30 gew.-%igen wässrigen PVP-Lösung und 0.1 g einer 40 gew.-%igen wässrigen Lösung von EDTA wurden in 275 g Wasser gelöst und 120 g PEG (Pluriol^{™} E 1500) wurden hinzugefügt. Der pH-Wert wurde mit Triethanolamin auf 6.75 eingestellt und die Emulsion mit Stickstoffgas für 10 Minuten gespült.
Dann wurde eine Mischung aus 348 g einer 50 gew.-%igen wässrigen Lösung von AM, 133.1 g einer 45 gew.%igen wässrigen Lösung von QVI und 0.373 g Triallylamin während 10 Minuten hinzugegeben.
Danach wurden 0,1 g des Radikalstarters V-50^{™} hinzugegeben und die Reaktionsmischung unter Stickstoffatmosphäre auf 60°C erwärmt. Nachdem die Mischung bei dieser Temperatur für 4.25 Stunden gerührt worden war, wurden 0,1 g des Radikalstarters VA-044^{™} zugegeben und die Mischung für eine weitere Stunde bei 60°C gerührt und dann auf Raumtemperatur abgekühlt.
Man erhielt eine weisse Dispersion mit einer Viskosität von 650 mPa*s.

### Beispiel (M)A 6

135 g einer 30 gew.-%igen wässrigen PVP-Lösung und 0.1 g einer 40 gew.-%igen wässrigen Lösung von EDTA wurden in 167 g Wasser gelöst und 135 g PEG (Pluriol^{™} E 1500) wurden hinzugefügt. Der pH-Wert wurde mit Triethanolamin auf 6.75 eingestellt und die Emulsion mit Stickstoffgas für 10 Minuten gespült.
Dann wurde eine Mischung aus 348 g einer 50 gew.-%igen wässrigen Lösung von AM, 56.66 g einer 80 gew.-%igen wässrigen Lösung von DMA3*MeCl während 10 Minuten hinzugegeben. Danach wurden 0,1 g des Radikalstarters V-50^{™} hinzugegeben und die Reaktionsmischung unter Stickstoffatmosphäre auf 60°C erwärmt. Nachdem die Mischung bei dieser Temperatur für 3 Stunden gerührt worden war, wurden 0,1 g des Radikalstarters VA-044^{™} zugegeben und die Mischung für eine weitere Stunde bei 60°C gerührt und dann auf Raumtemperatur abgekühlt. Man erhielt eine weisse Dispersion mit einer Viskosität von 2260 mPa*s.

Die Brookfield-Viskositätsmessung wurde bei 25°C, mit Spindel 4 und 12 Umdrehungen gemessen.

Die Bestimmung der Kämmkraftabnahme wurde wie folgt durchgeführt:

Bestimmung Blindwert Nasskämmbarkeit: Die gewaschenen Haare wurden über Nacht im Klimaraum getrocknet. Vor der Messung wurden sie zweimal mit Texapon NSO insgesamt 1 Minute shampooniert und 1 Minute ausgespült, damit sie definiert nass, d.h. gequollen sind. Vor Beginn der Messung wurde die Tresse so vorgekämmt, bis keine Verhakungen der Haare mehr vorhanden sind und somit bei wiederholtem Messkämmen eine konstante Kraftaufwendung erforderlich ist. Anschließend wurde die Tresse an der Halterung fixiert und mit der feinzinkigen Seite des Kammes in die feinzinkige Seite des Prüfkammes eingekämmt. Das Einlegen der Haare in den Prüfkamm erfolgte bei jeder Meßung gleichmäßig und spannungsfrei. Die Messung wurde gestartet und mittels Software (EGRANUDO-Programm, Fa. Frank) ausgewertet. Die Einzelmessung wurde 5 bis 10 mal wiederholt. Der errechnete Mittelwert wurde notiert.

Bestimmung Messwert Nasskämmbarkeit: Nach der Bestimmung des Blindwertes wurden die Haare je nach gewünschter Anwendung behandelt. Die Messung der Kämmkraft erfolgt analog der Blindwertbestimmung.

### Auswertung:

### Kämmkraftabnahme naß [%] =100 - (Meßwert *100/ Blindwert)

### Bestimmung Blindwert Trockenkämmbarkeit:

Die gewaschenen Haare werden über Nacht im Klimaraum getrocknet. Vor Beginn der Messung wird die Tresse so vorgekämmt, bis keine Verhakungen der Haare mehr vorhanden sind und somit bei wiederholtem Messkämmen eine konstante Kraftaufwendung erforderlich ist. Anschließend wird die Tresse an der Halterung fixiert und in die feinzinkige Seite des Prüfkamms eingekämmt. Das Einlegen der Haare in den Prüfkamm hat bei jeder Messung gleichmäßig und spannungsfrei zu erfolgen. Die Messung wird gestartet und mittels Software (mtt-win, Fa. DI-ASTRON) ausgewertet. Die Einzelmessung wird 5-10 mal wiederholt. Der errechnete Mittelwert wird zusammen mit der Standardabweichung notiert.

### Bestimmung Messwert Trockenkämmbarkeit:

Nach der Bestimmung des Blindwertes werden die Haare je nach gewünschter Anwendung behandelt und über Nacht getrocknet. Die Messung der Kämmkraft erfolgt analog der Blindwertbestimmung.

### Auswertung:

### Kämmkraftabnahme trocken [%] = 100- (Meßwert*100/ Blindwert)

Die Ergebnisse der Beispiele 1 bis 3 sowie Vergleichsbeispiele V1 bis V3 sind in Tabelle 1 zusammengestellt:

**Tabelle 1:**

| Nr. | VFA Monomer a) [Gew.-%] | QVI Monomer e) [Gew.-%] | Triallylamin Monomer d) [Gew.-%] | Pluriol E 1500 Polymeres Fällungsagenz c) [Gew.%] | Polymeres Dispergiermittel b) [Gew.-%] | | Dispersion Feststoffgehalt [Gew.-%]# | Viskosität [mPas] | | Kämmkraftabnahme [Note] | | Kämmkraftabnahme [in %] | |
|---|---|---|---|---|---|---|---|---|---|---|---|---|---|
| | | | | | Kollidon 90 | Kollidon 17 | | Tel quel | | Naß | Trocken | Naß | Trocken |
| 1 | 18 | 2 | 0,06 | 18 | 3 | 5 | 46,06 | 650 | | 1- | 1 | 65 | 92 |
| 2 | 16 | 4 | 0,1 | 18 | 3 | 5 | 46,1 | 5900 | | 1-2 | 1 | 59 | 95 |
| 3 | 18 | 2 | 0,04 | 18 | 6 | 5 | 49,04 | 8600 | | 1- | 1 | 71 | 95 |
| V1 | 18 | 2 | 0,06 | - | - | - | 20,06 | >75000 | | 2-3 | -* | 24 | - |
| V2 | 16 | 4 | 0,1 | - | - | - | 20,1 | >75000 | | 2 | -* | 32 | |
| V3 | 18 | 2 | - | 18 | 5 | 5 | 49 | 2600 | | 2- | -* | 28 | - |

| | | | | | | | | | | | | | |
|---|---|---|---|---|---|---|---|---|---|---|---|---|---|
| # Wasser wird zugefügt zur 100 Gew. % | | | | | | | | | | | | | |
| * nicht gemessen weil Naßkämmbarkeit zu niedrig. | | | | | | | | | | | | | |

Shampooformulierungen mit Polymeren aus Beispielen (M)A 1 bis 6

| | |
|---|---|
| Rezeptur: 35.70g | Texapon NSO |
| 12.50g | Tego Betain L7 |
| 0.50g | Polymer |
| 0.10g | Euxyl K 100 |
| ad 100g | Wasser |

| Dispersion [Bezeichnung laut Beispielen] | Feststoff-gehalt Dispersion [Gew.-%] | Monomere [Bezeichnung laut Beispielen] | Monomer-Verhältnis [mol-%] | Nasskämmbarkeit unbehandelt von Hand (Note 1-3) | Nasskämmbarkeit behandelt von Hand (Note 1-3) | Kämmkraftabnahme der Nasskämmbarkeit [%] |
|---|---|---|---|---|---|---|
| (M)A 1 | 24,2 | AM/QVI | 90/10 | 2-3 | 1-2 | 37 |
| (M)A 2 | 23,8 | AM / DADMAC | 90/10 | 2-3 | 1-2 | 35 |
| (M)A 3 | 23,5 | AM/QVI | 90/10 | 2-3 | 1-2 | 39 |
| (M)A 4 | 23,5 | AM/AS/QVI | 80/10/10 | 2-3 | 1-2 | 43 |
| (M)A 5 | 23,5 | AM/QVI | 90/10 | 2-3 | 1-2 | 43 |
| (M)A 6 | 26,6 | AM / DMA3.CH₃Cl | 90/10 | 2-3 | 1-2 | 40 |
| (M)A 5 | 23,5 | AM / QVI | 90/10 | 2-3 | 1-2 | 43 |

Die erfindungsgemäßen Dispersionen (Beispiele 1 bis 3) zeigen exzellente haarskosmetische Eigenschaften. Sie sind mit hohem Feststoffgehalt bei erwünschter niedriger Viskosität herstellbar. Die entsprechenden Dispersionen, hergestellt ohne Vernetzer (Vergleichsbeispiel V-3) zeigen unbefriedigende haarkosmetische Eigenschaften. Die Herstellung in Gegenwart eines Vernetzer ist zwingend notwendig zum Erreichen der anwendungstechnischen Eigenschaften. Polymerisate, die ohne polymeres Dispergiermittel und polymeres Fällungsagens hergestellt werden (Vergleichsbeispiel V-1) sind aufgrund ihrer hohen Lösungsviskosität großtechnisch nicht zugänglich. Zudem sind deren haarkosmetischen Eigenschaften unbefriedigend im Vergleich zu denen der erfindungsgemäßen Dispersionen. Für die Herstellung von anwendungstechnisch als exzellent eingestuften Polymerisaten ist daher die Gegenwart von eines polymeren Fällungsagens (insbesondere PEG) und eines geeigneten polymeren Dispergiermittels erforderlich.

### Beispiele für kosmetische Zubereitungen (alle Angaben in Gew.-%)

In allen Formulierungen wurden die Dispersionen erhalten nach Beispielen 1,3, 4, 7 und 11 eingesetzt.

### Beispiel 1: Flüssiges Makeup

| | |
|---|---|
| A | |
| 1,70 | Glyceryl Stearate |
| 1,70 | Cetyl Alcohol |
| 1,70 | Ceteareth-6 |
| 1,70 | Ceteareth-25 |
| 5,20 | Caprylic/Capric Triglyceride |
| 5,20 | Mineral Oil |

| | |
|---|---|
| B | |
| q.s. | Konservierungsmittel |
| 4,30 | Propylene Glycol |
| 2,50 | erfindungsgemäße Dispersion |
| 59,50 | dest. Wasser |

| | |
|---|---|
| C | |
| q.s. | Parfumöl |

| | |
|---|---|
| D | |
| 2,00 | Iron Oxides |
| 12,00 | Titanium Dioxide |

### Herstellung:

Phase A und Phase B getrennt voneinunder auf 80°C erwärmen. Dann Phase B in Phase A mit einem Rührer mischen. Alles auf 40°C abkühlen lassen und Phase C und Phase D zugeben. Wiederholt homogenisieren.

### Bespiel 2: Ölfreies Makeuu

| | |
|---|---|
| A | |
| 0,35 | Veegum |
| 5,00 | Butylene Glykol |
| 0,15 | Xanthan Gum |

| | |
|---|---|
| B | |
| 53,0 | dest. Wasser |
| q.s. | Konservierungsmittel |
| 0,2 | Polysorbate-20 |
| 1,6 | Tetrahydroxypropyl Ethylenediamine |

| | |
|---|---|
| C | |
| 1,0 | Silica |
| 2,0 | Nylone-12 |
| 4,15 | Mica |
| 6,0 | Titanium Dioxide |
| 1,85 | Iron Oxides |

| | |
|---|---|
| D | |
| 4,0 | Stearic Acid |
| 1,5 | Glyceryl Stearate |
| 7,0 | Benzyl Laurate |
| 5,0 | Isoeicosane |
| q.s. | Konservierungsmittel |

| | |
|---|---|
| E | |
| 1,0 | dest. Wasser |
| 0,5 | Panthenol |
| 0,1 | Imidazolidinyl Urea |
| 5,0 | erfindungsgemäße Disperison |

### Herstellung:

Phase A mit Butylene Glykol benetzen, in Phase B hineingeben und gut mischen. Phase AB auf 75°C erwärmen. Phase C Einsatzstoffe pulverisieren, in Phase AB hineingeben und gut homogenisieren. Einsatzstoffe von Phase D mischen, auf 80°C erwärmen und zu Phase ABC geben. Einige Zeit mischen, bis alles homogen ist. Alles in ein Gefäß mit Propellermischer übertragen. Einsatzstoffe von Phase E mischen, in Phase ABCD hineingeben und gut vermischen.

### Besipiel 3: Eyeliner

| | |
|---|---|
| A | |
| 40,6 | dest. Wasser |
| 0,2 | Disodium EDTA |
| q.s. | Konservierungsmittel |

| | |
|---|---|
| B | |
| 0,6 | Xanthan Gum |
| 0,4 | Veegum |
| 3,0 | Butylene Glycol |
| 0,2 | Polysorbate-20 |

| | |
|---|---|
| C | |
| 15,0 | Iron oxide / Al Powder / Silica (z.B. Sicopearl Fantastico Gold^{™} von BASF) |

| | |
|---|---|
| D | |
| 10,0 | Dest. Wasser |
| 30,0 | erfindungsgemäße Dispersion |

### Herstellung:

Phase B vormischen. Mit einem Propellermischer Phase B in Phase A hineinmischen, wobei man den Verdicker quellen lässt. Phase C mit Phase D benetzen, alles in Phases AB zugeben und gut mischen.

### Besipiel 4: Schimmerndes Gel

| | |
|---|---|
| A | |
| 32,6 | Dest. Wasser |
| 0,1 | Disodium EDTA |
| 25,0 | Carbomer (2%ige wässrige Lösung) |
| 0,3 | Konservierungsmittel |

| | |
|---|---|
| B | |
| 0,5 | Dest. Wasser |
| 0,5 | Triethanolamine |

| | |
|---|---|
| C | |
| 10,0 | Dest. Wasser |
| 9,0 | erfindungsgemäße Dispersion |
| 1,0 | Polyquatemium-46 |
| 5,0 | Iron Oxide |

| | |
|---|---|
| D | |
| 15,0 | Dest. Wasser |
| 1,0 | D-Panthenol 50 P (Panthenol und Propylene Glycol) |

### Herstellung:

Mit einem Propellermischer die Einsatzstoffe von Phase A in der angegebenen Reihenfolge gut mischen. Dann Phase B in Phase A heineingeben. Langsam rühren bis alles homogen ist. Phase C gut homogenisieren, bis die Pigmente gut verteilt sind. Phase C und Phase D zu Phase AB geben und gut mischen.

### Beispiel 5: Wasserfester Mascara

| | |
|---|---|
| A | |
| 46,7 | Dest. Wasser |
| 3,0 | Lutrol E 400 (PEG-8) |
| 0,5 | Xanthan Gum |
| q.s. | Konservierungsmittel |
| 0,1 | lmidazolidinyl Urea |
| 1,3 | Tetrahydroxypropyl Ethylenediamine |

| | |
|---|---|
| B | |
| 8,0 | Carnauba Wax |
| 4,0 | Beeswax |
| 4,0 | Isoeicosane |
| 4,0 | Polyisobutene |
| 5,0 | Stearic Acid |
| 1,0 | Glyceryl Stearate |
| q.s. | Konservierungsmittel |
| 2,0 | Benzyl Laurate |

| | |
|---|---|
| C | |
| 10,0 | Iron oxide / Al Powder / Silica (z.B. Sicopearl Fantastico Gold^{™} von BASF) |

| | |
|---|---|
| E | |
| 8,0 | Polyurethane-1 |
| 2,0 | erfindungsgemäße Dispersion |

### Herstellung:

Phase A und Phase B getrennt voneinander auf 85ºC erwärmen. Temperatur halten und Phase C zu Phase A geben und homogenisieren, bis die Pigmente gleichmäßig verteilt sind. Phase B zu Phases AC geben und für 2-3 Minuten homogenisieren. Dann Phase E zugeben und langsam rühren. Alles auf Raumtemperatur abkühlen lassen.

### Besipiel 6: Sonnenschutz-Gel

| | |
|---|---|
| Phase A | |
| 1,00 | PEG-40 Hydrogenated Castor Oil |
| 8,00 | Octyl Methoxycinnamate (Uvinul MC 80^{™} von BASF) |
| 5,00 | Octocrylene (Uvinul N 539^{™} von BASF) |
| 0,80 | Octyl Triazone (Uvinul T 150^{™} von BASF) |
| 2,00 | Butyl Methoxydibenzoylmethane (Uvinul BMBM^{™} von BASF) |
| 2,00 | Tocopheryl Acetate |
| q.s. | Parfümöl |

| | |
|---|---|
| Phase B | |
| 2,50 | erfindungsgemäße Dispersion |
| 0,30 | Acrylates/C10-30 Alkyl Acrylate Crosspolymer |
| 0,20 | Carbomer |
| 5,00 | Glycerin |
| 0,20 | Disodium EDTA |
| q.s. | Konservierungsmittel |
| 72,80 | dest. Wasser |

| | |
|---|---|
| Phase C | |
| 0,20 | Sodium Hydroxide |

### Herstellung:

Die Komponenten der Phase A mischen. Phase B quellen lassen und unter Homogenisieren in Phase A einrühren. Mit Phase C neutralisieren und erneut homogenisieren.

### Besipiel 7: Sonnenschutzemulsion mit TiO₂ und ZnO₂

| | |
|---|---|
| Phase A | |
| 6,00 | PEG-7 Hydrogenated Castor Oil |
| 2,00 | PEG-45/Dodecyl Glycol Copolymer |
| 3,00 | Isopropyl Myristate |
| 8,00 | Jojoba (Buxus Chinensis) Oil |
| 4,00 | Octyl Methoxycinnamate (Uvinul MC 80) |
| 2,00 | 4-Methylbenzylidene Camphor (Uvinul MBC 95) |
| 3,00 | Titanium Dioxide, Dimethicone |
| 1,00 | Dimethicone |
| 5,00 | Zinc Oxide, Dimethicone |

| | |
|---|---|
| Phase B | |
| 2,00 | erfindungsgemäße Dispersion |
| 0,20 | Disodium EDTA |
| 5,00 | Glycerin |
| q.s. | Konservierungsmittel |
| 58,80 | dest. Wasser |

| | |
|---|---|
| Phase C | |
| q.s. | Parfümöl |

### Herstellung:

Die Phasen A und B getrennt auf ca. 85°C erwärmen. Phase B in Phase A einrühren und homogenisieren. Abkühlen auf ca. 40°C, Phase C hinzugeben und nochmals kurz homogenisieren.

### Besipiel 8: Sun Protection Lotion

| | |
|---|---|
| Phase A | |
| 6,00 | Octyl Methoxycinnamate (Uvinul MC 80^{™} von BASF) |
| 2,50 | 4-Methylbenzylidene Camphor (Uvinul MBC 95^{™} von BASF) |
| 1,00 | Octyl Triazone (Uvinul T 150^{™} von BASF) |
| 2,00 | Butyl Methoxydibenzoylmethane (Uvinul BMBM^{™} von BASF) |
| 2,00 | PVP/Hexadecene Copolymer |
| 5,00 | PPG-3 Myristyl Ether |
| 0,50 | Dimethicone |
| 0,10 | BHT, Ascorbyl Palmitate, Citric Acid, Glyceryl Stearate, Propylene Glycol |
| 2,00 | Cetyl Alcohol |
| 2,00 | Potassium Cetyl Phosphate |

| | |
|---|---|
| Phase B | |
| 2,50 | erfindungsgemäße Dispersion |
| 5,00 | Propylene Glycol |
| 0,20 | Disodium EDTA |
| q.s. | Konservierungsmittel |
| 63,92 | dest. Wasser |
| Phase C | |
| 5,00 | Mineral Oil |
| 0,20 | Carbomer |
| Phase D | |
| 0,08 | Sodium Hydroxide |

| | |
|---|---|
| Phase E | |
| q.s. | Parfümöl |

### Herstellung:

Die Phasen A und B getrennt auf ca. 80°C erwärmen. Phase B unter Homogenisieren in Phase A einrühren, kurz nachhomogenisieren. Phase C anschlämmen, in Phase AB einrühren, mit Phase D neutralisieren und nachhomogenisieren. Abkühlen auf ca. 40°C, Phase E zugeben, nochmals homogenisieren.

### Beispiel 9: Abziehbare Gesichtsmaske

| | |
|---|---|
| Phase A | |
| 57,10 | dest. Wasser |
| 6,00 | Polyvinyl Alcohol |
| 5,00 | Propylene Glycol |

| | |
|---|---|
| Phase B | |
| 20,00 | Alcohol |
| 4,00 | PEG-32 |
| q.s | Parfümöl |

| | |
|---|---|
| Phase C | |
| 5,00 | Polyquaternium-44 |
| 2,70 | erfindungsgemäße Dispersion |
| 0,20 | Allantoin |

### Herstellung:

Phase A auf mind. 90°C erwärmen und rühren bis gelöst. Phase B bei 50°C lösen und in Phase A einrühren. Bei ca. 35°C den Ethanolverlust ausgleichen. Phase C zugeben und unterrühren.

### Beispiel 10: Gesichtsmaske

| | |
|---|---|
| Phase A | |
| 3,00 | Ceteareth-6 |
| 1,50 | Ceteareth-25 |
| 5,00 | Cetearyl Alcohol |
| 6,00 | Cetearyl Octanoate |
| 6,00 | Mineral Oil |
| 0,20 | Bisabolol |
| 3,00 | Glyceryl Stearate |

| | |
|---|---|
| Phase B | |
| 2,00 | Propylene Glycol |
| 5,00 | Panthenol |
| 2,80 | erfindungsgemäße Dispersion |
| q.s. | Konservierungsmittel |
| 65,00 | dest. Wasser |

| | |
|---|---|
| Phase C | |
| q.s. | Parfümöl |
| 0,50 | Tocopheryl Acetate |

### Herstellung:

Phase A und B getrennt auf ca. 80°C erwärmen. Phase B in Phase A unter Homogenisieren einrühren, kurz nachhomogenisieren. Abkühlen auf ca. 40°C, Phase C hinzugeben, nochmals homogenisieren.

### Beispiel 11: Körperlotion-Schaum

| | |
|---|---|
| Phase A | |
| 1,50 | Ceteareth-25 |
| 1,50 | Ceteareth-6 |
| 4,00 | Cetearyl Alcohol |
| 10,00 | Cetearyl Octanoate |
| 1,00 | Dimethicone |

| | |
|---|---|
| Phase B | |
| 3,00 | erfindungsgemäße Dispersion |
| 2,00 | Panthenol |
| 2,50 | Propylene Glycol |
| q.s. | Konservierungsmittel |
| 74,50 | dest. Wasser |

| | |
|---|---|
| Phase C | |
| q.s. | Parfümöl |

### Herstellung:

Die Phasen A und B getrennt auf ca. 80°C erwärmen. Phase B in Phase A einrühren und homogenisieren. Abkühlen auf ca. 40°C, Phase C hinzugeben und nochmals kurz homogenisieren. Abfüllung: 90% Wirkstoff und 10% Propan/Butan bei 3,5bar (20°C).

### Beispiel 12: Gesichtswasser für trockene und empfindliche Haut

| | |
|---|---|
| Phase A | |
| 2,50 | PEG-40 Hydrogenated Castor Oil |
| q.s. | Parfümöl |
| 0,40 | Bisabolol |

| | |
|---|---|
| Phase B | |
| 3,00 | Glycerin |
| 1,00 | Hydroxyethyl Cetyldimonium Phosphate |
| 5,00 | Whitch Hazel (Hamamelis Virginiana) Distillate |
| 0,50 | Panthenol |
| 0,50 | erfindungsgemäße Dispersion |
| q.s. | Konservierungsmittel |
| 87,60 | dest. Wasser |

### Herstellung:

Phase A klar lösen. Phase B in Phase A einrühren.

### Beispiel 13: Gesichtswaschpaste mit Peelingeffekt

| | |
|---|---|
| Phase A | |
| 70,00 | dest. Wasser |
| 3,00 | erfindungsgemäße Dispersion |
| 1,50 | Carbomer |
| q.s. | Konservierungsmittel |

| | |
|---|---|
| Phase B | |
| q.s. | Parfümöl |
| 7,00 | Potassium Cocoyl Hydrolyzed Protein |
| 4,00 | Cocamidopropyl Betaine |

| | |
|---|---|
| Phase C | |
| 1,50 | Triethanolamine |
| Phase D | |
| 13,00 | Polyethylene (Luwax A^{™} von BASF) |

### Herstellung:

Phase A quellen lassen. Phase B klar lösen. Phase B in Phase A einrühren. Mit Phase C neutralisieren. Danach Phase D einrühren.

### Gesichtsseife

| | |
|---|---|
| Phase A | |
| Potassium | Cocoate |
| Disodium | Cocoamphodiacetate |
| 2.0 | Lauramide DEA |
| Glycol | Stearate |
| 2.0 | erfindungsgemäße Dispersion |
| 50.0 | dest. Wasser |
| q.s. | Citric Acid |

| | |
|---|---|
| Phase B | |
| q.s. | Konservierungsmittel |
| q.s. | Parfumöl |

### Herstellung:

Phase A unter Rühren auf 70°C erwärmen, bis alles homogen ist. pH-Wert auf 7.0 - 7.5 mit Citric Acid. Alles auf 50°C abkühlen lassen und Phase B zugeben.

### Beispiel 14: Gesichtsreinigungsmilch Typ O/W

| | |
|---|---|
| Phase A | |
| 1,50 | Ceteareth-6 |
| 1,50 | Ceteareth-25 |
| 2,00 | Glyceryl Stearate |
| 2,00 | Cetyl Alcohol |
| 10,00 | Mineral Oil |

| | |
|---|---|
| Phase B | |
| 5,00 | Propylene Glycol |
| q.s. | Konservierungsmittel |
| 1,0 | erfindungsgemäße Dispersion |
| 66,30 | dest. Wasser |

| | |
|---|---|
| Phase C | |
| 0,20 | Carbomer |
| 10,00 | Cetearyl Octanoate |

| | |
|---|---|
| Phase D | |
| 0,40 | Tetrahydroxypropyl Ethylenediamine |

| | |
|---|---|
| Phase E | |
| q.s. | Parfümöl |
| 0,10 | Bisabolol |

### Herstellung:

Die Phasen A und B getrennt auf ca. 80°C erwärmen. Phase B unter Homogenisieren in Phase A einrühren, kurz nachhomogenisieren. Phase C anschlämmen, in Phase AB einrühren, mit Phase D neutralisieren und nachhomogenisieren. Abkühlen auf ca. 40°C, Phase E zugeben, nochmals homogenisieren.

### Beispiel 15: Transparente Seife

| | |
|---|---|
| 4,20 | Sodium Hydroxide |
| 3,60 | dest. Wasser |
| 2,0 | erfindungsgemäße Dispersion |
| 22,60 | Propylene Glycol |
| 18,70 | Glycerin |
| 5,20 | Cocoamide DEA |
| 10,40 | Cocamine Oxide |
| 4,20 | Sodium Lauryl Sulfate |
| 7,30 | Myristic Acid |
| 16,60 | Stearic Acid |
| 5,20 | Tocopherol |

### Herstellung:

Alle Zutaten mischen. Die Mischung klar schmelzen bei 85°C. Sofort in die Form ausgiessen.

### Beispiel 16: Peeling-Creme, Typ O/W

| | |
|---|---|
| Phase A | |
| 3,00 | Ceteareth-6 |
| 1,50 | Ceteareth-25 |
| 3,00 | Glyceryl Stearate |
| 5,00 | Cetearyl Alcohol, Sodium Cetearyl Sulfate |
| 6,00 | Cetearyl Octanoate |
| 6,00 | Mineral Oil |
| 0,20 | Bisabolol |

| | |
|---|---|
| Phase B | |
| 2,00 | Propylene Glycol |
| 0,10 | Disodium EDTA |
| 3,00 | erfindungsgemäße Dispersion |
| q.s. | Konservierungsmittel |
| 59,70 | dest. Wasser |

| | |
|---|---|
| Phase C | |
| 0,50 | Tocopheryl Acetate |
| q.s. | Parfümöl |

| | |
|---|---|
| Phase D | |
| 10,00 | Polyethylene |

### Herstellung:

Die Phasen A und B getrennt auf ca. 80°C erwärmen. Phase B in Phase A einrühren und homogenisieren. Abkühlen auf ca. 40°C, Phase C hinzugeben und nochmals kurz homogenisieren. Anschließend Phase D unterrühren.

### Beispiel 17: Rasierschaum

| | |
|---|---|
| 6,00 | Ceteareth-25 |
| 5,00 | Poloxamer 407 |
| 52,00 | dest. Wasser |
| 1,00 | Triethanolamine |
| 5,00 | Propylene Glycol |
| 1,00 | PEG-75 Lanolin Oil |
| 5,00 | erfindungsgemäße Dispersion |
| q.s. | Konservierungsmittel |
| q.s. | Parfümöl |
| 25,00 | Sodium Laureth Sulfate |

### Herstellung:

Alles zusammennwiegen, danach rühren bis gelöst. Abfüllung: 90 Teile Wirksubstanz und 10 Teile Propan/Butan-Mischung 25:75.

### Beispiel 18: After Shave Balsam

| | |
|---|---|
| Phase A | |
| 0,25 | Acrylates/C10-30 Alkyl Acrylate Crosspolymer |
| 1,50 | Tocopheryl Acetate |
| 0,20 | Bisabolol |
| 10,00 | Caprylic/Capric Triglyceride |
| q.s. | Parfümöl |
| 1,00 | PEG-40 Hydrogenated Castor Oil |

| | |
|---|---|
| Phase B | |
| 1,00 | Panthenol |
| 15,00 | Alcohol |
| 5,00 | Glycerin |
| 0,05 | Hydroxyethyl Cellulose |
| 1,92 | erfindungsgemäße Dispersion |
| 64,00 | dest. Wasser |

| | |
|---|---|
| Phase C | |
| 0,08 | Sodium Hydroxide |

### Herstellung:

Die Komponenten der Phase A mischen. Phase B unter Homogenisieren in Phase A einrühren, kurz nachhomogenisieren. Mit Phase C neutralisieren und erneut homogenisieren.

### Beispiel 19: Körperpflegecreme

| | |
|---|---|
| Phase A | |
| 2,00 | Ceteareth-6 |
| 2,00 | Ceteareth-25 |
| 2,00 | Cetearyl Alcohol |
| 3,00 | Glyceryl Stearate SE |
| 5,00 | Mineral Oil |
| 4,00 | Jojoba (Buxus Chinensis) Oil |
| 3,00 | Cetearyl Octanoate |
| 1,00 | Dimethicone |
| 3,00 | Mineral Oil, Lanolin Alcohol |

| | |
|---|---|
| Phase B | |
| 5,00 | Propylene Glycol |
| 0,50 | Veegum |
| 1,00 | Panthenol |
| 1,70 | erfindungsgemäße Dispersion |
| 6,00 | Polyquaternium-44 |
| q.s. | Konservierungsmittel |
| 60,80 | dest. Wasser |

| | |
|---|---|
| Phase C | |
| q.s. | Parfümöl |

### Herstellung:

Die Phasen A und B getrennt auf ca. 80°C erwärmen. Phase B homogenisieren. Phase B unter Homogenisieren in Phase A einrühren, kurz nachhomogenisieren. Abkühlen auf ca. 40°C, Phase C hinzugeben und nochmals kurz homogenisieren.

### Beispiel 20: Zahnpasta

| | |
|---|---|
| Phase A | |
| 34,79 | dest. Wasser |
| 3,00 | erfindungsgemäße Dispersion |
| 0,30 | Konservierungsmittel |
| 20,00 | Glycerin |
| 0,76 | Sodium Monofluorophosphate |

| | |
|---|---|
| Phase B | |
| 1,20 | Sodium Carboxymethylcellulose |

| | |
|---|---|
| Phase C | |
| 0,80 | Aromaöl |
| 0,06 | Saccharin |
| 0,10 | Konservierungsmittel |
| 0,05 | Bisabolol |
| 1,00 | Panthenol |
| 0,50 | Tocopheryl Acetate |
| 2,80 | Silica |
| 1,00 | Sodium Lauryl Sulfate |
| 7,90 | Dicalciumphosphate Anhydrate |
| 25,29 | Dicalciumphosphate Dihydrate |
| 0,45 | Titanium Dioxide |

### Herstellung:

Phase A lösen. Phase B in Phase A einstreuen und lösen. Phase C zugeben und unter Vakuum bei RT ca. 45 Min. rühren lassen.

### Beispiel 21: Mundwasser

| | |
|---|---|
| Phase A | |
| 2,00 | Aromaöl |
| 4,00 | PEG-40 Hydrogenated Castor Oil |
| 1,00 | Bisabolol |
| 30,00 | Alcohol |

| | |
|---|---|
| Phase B | |
| 0,20 | Saccharin |
| 5,00 | Glycerin |
| q.s. | Konservierungsmittel |
| 5,00 | Poloxamer 407 |
| 0,5 | erfindungsgemäße Dispersion |
| 52,30 | dest. Wasser |

### Herstellung:

Phase A und Phase B getrennt klar lösen. Phase B in Phase A einrühren.

### Beispiel 22: Prothesenhaftmittel

| | |
|---|---|
| Phase A | |
| 0,20 | Bisabolol |
| 1,00 | Beta-Carotene |
| q.s. | Aromaöl |
| 20,00 | Cetearyl Octanoate |
| 5,00 | Silica |
| 33,80 | Mineral Oil |

| | |
|---|---|
| Phase B | |
| 5,00 | erfindungsgemäße Dispersion |
| 35,00 | PVP (20%ige Lösung in Wasser) |

### Herstellung:

Phase A gut mischen. Phase B in Phase A einrühren.

### Beispiel 23: Hautpflegecreme, Typ O/W

| | |
|---|---|
| Phase A | |
| 8,00 | Cetearyl Alcohol |
| 2,00 | Ceteareth-6 |
| 2,00 | Ceteareth-25 |
| 10,00 | Mineral Oil |
| 5,00 | CetearylOctanoate |
| 5,00 | Dimethicone |

| | |
|---|---|
| Phase B | |
| 3,00 | erfindungsgemäße Dispersion |
| 2,00 | Panthenol, Propylene Glycol |
| q.s. | Konservierungsmittel |
| 63,00 | dest. Wasser |

| | |
|---|---|
| Phase C | |
| q.s. | Parfümöl |

### Herstellung:

Phase A und B getrennt auf ca. 80 C erwärmen. Phase B in Phase A unter Homogenisieren einrühren, kurz nachhomogenisieren. Abkühlen auf ca. 40 C, Phase C hinzugeben, nochmals homogenisieren.

### Beispiel 24: Hautpflegecreme, Typ W/O

| | |
|---|---|
| Phase A | |
| 6,00 | PEG-7 Hydrogenated Castor Oil |
| 8,00 | Cetearyl Octanoate |
| 5,00 | Isopropyl Myristate |
| 15,00 | MineralOil |
| 2,00 | PEG-45/Dodecyl Glycol Copolymer |
| 0,50 | Magnesium Stearate |
| 0,50 | Aluminum Stearate |

| | |
|---|---|
| Phase B | |
| 3,00 | Glycerin |
| 3,30 | erfindungsgemäße Dispersion |
| 0,70 | Magnesium Sulfate |
| 2,00 | Panthenol |
| q.s. | Konservierungsmittel |
| 48,00 | dest. Wasser |

| | |
|---|---|
| Phase C | |
| 1,00 | Tocopherol |
| 5,00 | Tocopheryl Acetate |
| q.s. | Parfümöl |

### Herstellung:

Die Phasen A und B getrennt auf ca. 80°C erwärmen. Phase B in Phase A einrühren und homogenisieren. Abkühlen auf ca. 40°C, Phase C hinzugeben und nochmals kurz homogenisieren.

### Beispiel 25: Lippenpflegecreme

| | |
|---|---|
| Phase A | |
| 10,00 | Cetearyl Octanoate |
| 5,00 | Polybutene |

| | |
|---|---|
| Phase B | |
| 0,10 | Carbomer |

| | |
|---|---|
| Phase C | |
| 2,00 | Ceteareth-6 |
| 2,00 | Ceteareth-25 |
| 2,00 | Glyceryl Stearate |
| 2,00 | Cetyl Alcohol |
| 1,00 | Dimethicone |
| 1,00 | Benzophenone-3 |
| 0,20 | Bisabolol |
| 6,00 | Mineral Oil |

| | |
|---|---|
| Phase D | |
| 8,00 | erfindungsgemäße Dispersion |
| 3,00 | Panthenol |
| 3,00 | Propylene Glycol |
| q.s. | Konservierungsmittel |
| 54,00 | dest. Wasser |

| | |
|---|---|
| Phase E | |
| 0,10 | Triethanolamine |

| | |
|---|---|
| Phase F | |
| 0,50 | Tocopheryl Acetate |
| 0,10 | Tocopherol |
| q.s. | Parfümöl |

### Herstellung:

Phase A klar lösen. Phase B dazugeben und homogenisieren. Phase C addieren und schmelzen bei 80 C. Phase D auf 80 C erwärmen. Phase D zu Phase ABC geben und homogenisieren. Abkühlen auf ca. 40 C, Phase E und Phase F zugeben, nochmals homogenisieren.

### Beispiel 26: Glänzender Lippenstift

| | |
|---|---|
| Phase A | |
| 5,30 | Candelilla (Euphorbia Cerifera) Wax |
| 1,10 | Bees Wax |
| 1,10 | Microcrystalline Wax |
| 2,00 | Cetyl Palmitate |
| 3,30 | Mineral Oil |
| 2,40 | Castor Oil, Glyceryl Ricinoleate, Octyldodecanol, Carnauba, Candelilla Wax, |
| 0,40 | Bisabolol |
| 16,00 | Cetearyl Octanoate |
| 2,00 | Hydrogenated Coco-Glycerides |
| q.s. | Konservierungsmittel |
| 1,00 | erfindungsgemäße Dispersion |
| 60,10 | Castor (Ricinus Communis) Oil |
| 0,50 | Tocopheryl Acetate |

| | |
|---|---|
| Phase B | |
| 0,80 | C. I. 14720:1, Acid Red 14 Aluminum Lake |

| | |
|---|---|
| Phase C | |
| 4,00 | Mica, Titanium Dioxide |

### Herstellung:

Die Komponenten der Phase A einwiegen und aufschmelzen. Phase B homogen einarbeiten. Phase C zugeben und unterrühren. Unter Rühren auf Raumtemperatur abkühlen.

### Beispiel 26: Duschgel

| | |
|---|---|
| 50,00 | Sodium Laureth Sulfate, Magnesium Laureth Sulfate, Sodium Laureth-8 Sulfate, |
| Magnesium | Laureth-8 |
| 1,00 | Cocoamide DEA |
| 4,00 | erfindungsgemäße Dispersion |
| 2,00 | Sodium Laureth Sulfate, Glycol Distearate, Cocamide MEA, Laureth-10 |
| q.s. | Konservierungsmittel |
| q.s. | Parfümöl |
| 2,00 | Sodium Chloride |
| 41,00 | Aqua dem. |

### Herstellung:

Alles zusammenwiegen, rühren bis gelöst.

| | |
|---|---|
| Beispiel 27: Duschgel | |
| 30,00 | Sodium Laureth Sulfate |
| 6,00 | Sodium Cocoamphodiacetate |
| 6,00 | Cocamidopropyl Betaine |
| 3,00 | Sodium Laureth Sulfate, Glycol Distearate, Cocamide MEA, Laureth-10 |
| 7,70 | Polyquaternium-44 |
| 1,50 | erfindungsgemäße Dispersion |
| 1,00 | Panthenol |
| q.s. | Konservierungsmittel |
| q.s. | Parfümöl |
| q.s. | Citric Acid |
| 0,50 | Sodium Chloride |
| 44,30 | Aqua dem. |

### Herstellung:

Die Komponenten der Phase A einwiegen und lösen. Den pH-Wert auf 6-7 einstellen.

### Beispiel 28: Klares Duschgel

| | |
|---|---|
| 40,00 | Sodium Laureth Sulfate |
| 5,00 | Decyl Glucoside |
| 5,00 | Cocamidopropyl Betaine |
| 0,50 | Polyquaternium-10 |
| 2,20 | erfindungsgemäße Dispersion |
| 1,00 | Panthenol |
| q.s. | Parfümöl |
| q.s. | Konservierungsmittel |
| q.s. | Citric Acid |
| 2,00 | Sodium Chloride |
| 44,30 | Aqua dem. |

### Herstellung:

Die Komponenten der Phase A einwiegen und klar lösen.

### Beispiel 29: Duschbad

| | |
|---|---|
| A | |
| 40,00 | Sodium Laureth Sulfate |
| 5,00 | Sodium C12-15 Pareth-15 Sulfonate |
| 5,00 | Decyl Glucoside |
| q.s. | Parfümöl |
| 0,10 | Phytantriol |

| | |
|---|---|
| B | |
| 43,60 | Aqua dem. |
| 0,1 | Guarhydroxypropyl Trimoniumchloride |
| 2,20 | erfindungsgemäße Dispersion |
| 1,00 | Panthenol |
| q.s. | Konservierungsmittel |
| 1,00 | Laureth-3 |
| q.s. | Citric Acid |
| 2,00 | Sodium Chloride |

### Herstellung:

Die Komponenten der Phase A mischen. Die Komponenten der Phase B nacheinander zugeben und mischen. Den pH-Wert auf 6-7 einstellen.

### Beispiel 30: Flüssigseife

| | |
|---|---|
| A | |
| 44,06 | Aqua dem. |
| 0,34 | Aminomethyl Propanol |
| 3,40 | Acrylates Copolymer |

| | |
|---|---|
| B | |
| 40,00 | Sodium Laureth Sulfate |
| 10,00 | Cocamidopropyl Betaine |
| 0,20 | erfindungsgemäße Dispersion |
| q.s. | Parfümöl |
| q.s. | Konservierungsmittel |
| 2,00 | Sodium Chloride |

### Herstellung:

Die Komponenten der Phase A einwiegen und klar lösen. Die Komponenten der Phase B nacheinander zugeben und mischen.

### Beispiel 31: Flüssiges Fussbad

| | |
|---|---|
| A | |
| 1,00 | Nonoxynol-14 |
| 0,10 | Bisabolol |
| 1,00 | Pine (Pinus Sylvestris) Oil |

| | |
|---|---|
| B | |
| 5,00 | PEG-8 |
| 1,20 | erfindungsgemäße Dispersion |
| 0,50 | Triclosan |
| 30,00 | Sodium Laureth Sulfate |
| 3,00 | Polyquaternium-16 |
| 58,20 | Aqua dem. |
| q.s. | C. I. 19140 + C. I. 42 051 |

### Herstellung:

Phase A solubilisieren. Phase B mischen.

### Beispiel 32: Erfrischungsgel

| | |
|---|---|
| A | |
| 0,60 | Carbomer |
| 45,40 | Aqua dem. |

| | |
|---|---|
| B | |
| 0,50 | Bisabolol |
| 0,50 | Farnesol |
| q.s. | Parfümöl |
| 5,00 | PEG-40 Hydrogenated Castor Oil |
| 0,50 | erfindungsgemäße Dispersion |
| 1,00 | Tetrahydroxypropyl Ethylenediamine |
| 1,50 | Menthol |
| 45,00 | Alcohol |
| q.s. | C. 1. 74 180, Direct Blue 86 |

### Herstellung:

Phase A quellen lassen. Phase B lösen. Phase B in Phase A einrühren.

### Beispiel 33: Roll-on Antiperspirant

| | |
|---|---|
| A | |
| 0,40 | Hydroxyethylcellulose |
| 50,00 | Aqua dem. |

| | |
|---|---|
| B | |
| 25,00 | Alcohol |
| 0,10 | Bisabolol |
| 0,30 | Farnesol |
| 2,00 | PEG-40 Hydrogenated Castor Oil |
| q.s. | Parfümöl |

| | |
|---|---|
| C | |
| 5,00 | Aluminum Chlorohydrate |
| 3,00 | Propylene Glycol |
| 3,00 | Dimethicone Copolyol |
| 3,00 | Polyquaternium-16 |
| 1,20 | erfindungsgemäße Dispersion |
| 7,00 | Aqua dem. |

### Herstellung:

Phase A quellen lassen. Phase B und C getrennt lösen. Phase A und B in Phase C einrühren.

### Beispiel 34: Transparenter Deostift

| | |
|---|---|
| 5,00 | Sodium Stearate |
| 0,50 | Triclosan |
| 3,00 | Ceteareth-25 |
| 20,00 | Glycerin |
| 0,50 | erfindungsgemäße Dispersion |
| q.s. | Parfümöl |
| 60,00 | Propylene Glycol |
| 0,20 | Bisabolol |
| 10,80 | Aqua dem. |

### Herstellung:

Phase A zusammenwiegen, schmelzen und homogenisieen. Anschließend in die Form gießen.

### Beispiel 35: Wasserlösliches Badeöl

| | |
|---|---|
| 15,00 | Cetearyl Octanoate |
| 15,00 | Caprylic/Capric Triglyceride |
| 1,00 | Panthenol, Propylene Glycol |
| 0,10 | Bisabolol |
| 2,00 | Tocopheryl Acetate |
| 2,00 | Retinyl Palmitate |
| 0,10 | Tocopherol |
| 37,00 | PEG-7-Glyceryl-Cocoate |
| 0,40 | erfindungsgemäße Dispersion |
| 3,80 | Aqua dem. |
| q.s. | Parfümöl |
| 23,60 | PEG-40 Hydrogenated Castor Oil |

### Herstellung:

Mischen und rühren bis alles klar gelöst ist.

### Beispiel 36: Tagespflege-Aerosol

| | |
|---|---|
| A | |
| 4,00 | Ethylhexyl Methoxycinnamate |
| 1,50 | Octocrylene |
| 9,00 | Caprylic/Capric Triglyceride |
| 5,00 | Simmondsia Chinensis (Jojoba) Seed Oil |
| 1,50 | Cyclomethicone |
| 3,00 | Hydrogenated Coco-Glycerides |
| 1,00 | PVP/Hexadecene Copolymer |
| 1,00 | Ceteareth-6, Stearyl Alcohol |

| | |
|---|---|
| B | |
| 5,00 | Zinc Oxide |

| | |
|---|---|
| C | |
| 2,00 | Ceteareth-25 |
| 1,20 | Panthenol |
| 0,20 | Sodium Ascorbyl Phosphate |
| 0,30 | Imidazolidinyl Urea |
| 0,10 | Disodium EDTA |
| 1,50 | erfindungsgemäße Dispersion |
| 62,67 | Aqua dem. |

| | |
|---|---|
| D | |
| 0,50 | Tocopheryl Acetate |
| 0,20 | Bisabolol |
| 0,33 | Caprylic/Capric Triglyceride, Retinol |
| q.s. | Parfümöl |

### Herstellung:

Phase A auf 80°C erwärmen. Phase A klar lösen. Phase B einarbeiten und homogenisieren. Phase C zugeben, erhitzen auf 80°C, aufschmelzen und homogenisieren. Unter Rühren auf ca. 40°C abkühlen, Phase D hinzugeben und kurz homogenisieren. 90% Wirkstofflösung : 10% Propan/Butan mit 3,5bar (20°C) abfüllen.

### Beispiel 37: Feuchtigskeitscreme

| | |
|---|---|
| A | |
| 3,00 | Vitis Vinifera (Grape) Seed Oil |
| 1,00 | Cyclopentasiloxane, Cyclohexasiloxane |
| 1,50 | Cyclomethicone |
| 2,00 | Soybean (Glycine Soja) Oil |
| 2,00 | Ethylhexyl Methoxycinnamate |
| 1,00 | Uvinul A Plus (BASF) |
| 1,00 | Hydrogenated Lecithin |
| 1,00 | Cholesterol |
| 2,00 | PEG-40 Hydrogenated Castor Oil |
| 5,00 | Cetearyl Octanoate |
| 5,00 | Caprylic/Capric Triglyceride |

| | |
|---|---|
| B | |
| 3,00 | Caprylic/Capric Triglyceride, Acrylates Copolymer |

| | |
|---|---|
| C | |
| 3,00 | erfindungsgemäße Dispersion |
| 0,50 | Cocotrimonium Methosulfate |
| 2,00 | Panthenol, Propylene Glycol |
| 3,00 | Glycerin |
| 0,10 | Disodium EDTA |
| 60,30 | Aqua dem. |

| | |
|---|---|
| D | |
| 0,30 | Perfume |
| 0,30 | DMDM Hydantoin |
| 1,00 | Tocopheryl Acetate |
| 2,00 | Tocopherol |

### Herstellung:

Phase A auf 80°C erwärmen. Phase B in Phase A einrühren. Phase C auf ca. 80°C erwärmen und unter Homogenisieren in Phase A+B einrühren. Unter Rühren auf ca. 40°C abkühlen, Phase D hinzugeben und kurz homogenisieren.

### Beispiel 38: Aerosolhaarschaum

| | |
|---|---|
| A | |
| 2,00 | Cocotrimonium Methosulfate |
| 0,20 | Parfümöl |

| | |
|---|---|
| B | |
| 63,90 | Aqua dem. |
| 6,70 | erfindungsgemäße Dispersion |
| 0,50 | Acrylates Copolymer |
| 0,10 | Aminomethyl Propanol |
| 0,20 | Ceteareth-25 |
| 0,20 | Trimethylsilylamodimethicone, Trideceth-10, Cetrimonium Chloride |
| 0,10 | PEG-25 PABA |
| 0,20 | Hydroxyethylcellulose |
| 0,20 | PEG-8 |
| 0,20 | Panthenol |
| 15,00 | Alcohol |

| | |
|---|---|
| C | |
| 10,00 | Propan/Butan 3,5 bar (20°C) |

### Herstellung:

Phasen A und B mischen und mit Treibgas abfüllen.

### Beispiel 39: Pumpmousse

| | |
|---|---|
| A | |
| 2,00 | Cocotrimonium Methosulfate |
| q.s. | Parfümöl |

| | |
|---|---|
| C | |
| 86,30 | Aqua dem. |
| 7,00 | Polyquaternium-46 |
| 3,00 | erfindungsgemäße Dispersion |
| 0,50 | PEG-8 |
| 1,00 | Panthenol |
| q.s. | Konservierungsmittel |
| 0,20 | PEG-25 PABA |

### Herstellung:

Die Komponenten der Phase A mischen. Die Komponenten der Phase B nacheinander zugeben und klar lösen.

### Beispiel 40: Aerosolschaum

| | |
|---|---|
| 15,00 | erfindungsgemäße Dispersion |
| 5,00 | PVPNA Copolymer |
| 0,50 | Hydroxyethyl Cetyldimonium Phosphate |
| 0,20 | Ceteareth-25 |
| 0,40 | Parfümöl PC 910.781/Cremophor |
| 68,90 | Aqua dem. |
| q.s. | Konservierungsmittel |
| 10,00 | Propan/Butan 3,5 bar (20°C) |

### Herstellung:

Alles zusammen wiegen, rühren bis gelöst, dann abfüllen.

### Beispiel 41: Farbstyling-Mousse

| | |
|---|---|
| A | |
| 2,00 | Cocotrimonium Methosulfate |
| q.s. | Parfümöl |

| | |
|---|---|
| B | |
| 6,70 | erfindungsgemäße Dispersion |
| 0,50 | Acrylates Copolymer |
| 0,10 | Aminomethyl Propanol |
| 0,20 | Ceteareth-25 |
| 0,20 | Panthenol |
| 0,20 | Hydroxyethylcellulose |
| 10,00 | Alcohol |
| 69,97 | Aqua dem. |
| 0,08 | C.l. 12245, Basic Red 76 |
| 0,05 | C.l. 42510, Basic Violet 14 |

| | |
|---|---|
| C | |
| 10,00 | Propan/Butan 3,5 bar (20°C) |

### Herstellung:

Alles zusammen wiegen, rühren bis gelöst, dann abfüllen.
Nur für dunkelbondes und braunes Haar geeignet!

### Beispiel 42: Aerosolhaarschaum

| | |
|---|---|
| A | |
| 0,20 | Parfümöl |
| 2,00 | Cocotrimonium Methosulfate |

| | |
|---|---|
| B | |
| 69,90 | Aqua dem. |
| 14,70 | Polyurethane-1 |
| 2,00 | erfindungsgemäße Dispersion |
| 0,50 | PEG-25 PABA |
| 0,20 | Amodimethicone, Tallowtrimonium Chloride, Nonoxynol-10 |
| q.s. | Konservierungsmittel |
| 0,50 | Ceteareth-25 |

| | |
|---|---|
| C | |
| 10,00 | Propan/Butan 3,5 bar (20°C) |

### Herstellung:

Phase A mischen. Die Komponenten der Phase B nacheinander zugeben und lösen. Mit Phase C abfüllen.

### Beispiel 43: Pumphaarschaum

| | |
|---|---|
| A | |
| 1,50 | Cocotrimonium Methosulfate |
| q.s. | Parfümöl |

| | |
|---|---|
| B | |
| 2,00 | erfindungsgemäße Dispersion |
| 94,04 | Aqua dem. |

| | |
|---|---|
| C | |
| 0,46 | Aminomethyl Propanol |
| 4,00 | PEG/PPG-25/25 Dimethicone/Acrylates Copolymer |
| q.s. | Konservierungsmittel |

### Herstellung:

Phase A mischen. Phase B in Phase A einrühren. Phase C zugeben und rühren bis gelöst.

### Beispiel 44: Haarstylinggel

| | |
|---|---|
| A | |
| 0,50 | Carbomer |
| 87,60 | Aqua dem. |

| | |
|---|---|
| B | |
| 0,70 | Triethanolamine |

| | |
|---|---|
| C | |
| 6,00 | erfindungsgemäße Dispersion |
| 5,00 | PVP (Luviskol K30 oder Luviskol K90) |
| q.s. | Parfümöl |
| q.s. | PEG-40 Hydrogenated Castor Oil |
| q.s. | Konservierungsmittel |
| 0,10 | Tocopheryl Acetate |

### Herstellung:

Phase A quellen lassen und mit Phase B neutralisieren. Phase C lösen und in Phase A+B einrühren.

### Beispiel 45: Haarstylinggel

| | |
|---|---|
| A | |
| 0,50 | Carbomer |
| 87,60 | Aqua dem. |

| | |
|---|---|
| B | |
| 0,90 | Tetrahydroxypropyl Ethylenediamine |

| | |
|---|---|
| C | |
| 2,00 | erfindungsgemäße Dispersion |
| 9,00 | VPNA Copolymer (Luviskol VA64W; BASF) |
| q.s. | Parfümöl |
| q.s. | PEG-40 Hydrogenated Castor Oil |
| q.s. | Konservierungsmittel |
| 0,10 | Propylene Glycol |

### Herstellung:

Phase A quellen lassen und mit Phase B neutralisieren. Phase C lösen und in Phase A+B einrühren.

### Beispiel 46: Haarstylinggel

| | |
|---|---|
| 2,00 | erfindungsgemäße Dispersion |
| 6,00 | Corn Starch Modified (Amaze, National Starch) |
| 0,50 | Chitosan |
| q.s. | Parfümöl |
| q.s. | PEG-40 Hydrogenated Castor Oil |
| 0,10 | PEG-14 Dimethicone |
| 0,10 | Konservierungsmittel |
| 91,40 | Aqua dem. |

### Herstellung:

Alle Komponenten mischen bis sie homogen sind.

### Beispiel 47: Haarstylinggel

| | |
|---|---|
| 8,00 | erfindungsgemäße Dispersion |
| 5,00 | VP/DMAPA Acrylates Copolymer (ISP: Styleze CC-10) |
| 0,05 | Aminomethyl Propanol |
| 84,85 | Aqua dem. |
| q.s. | Parfümöl |
| q.s. | PEG-40 Hydrogenated Castor Oil |
| 0,10 | Dimethicone Copolyol |
| 0,10 | Konservierungsmittel |
| 2,00 | Hydroxypropylcellulose |

### Herstellung:

Alle Komponenten mischen bis sie homogen sind.

### Beispiel 48: Haarstylinggel

| | |
|---|---|
| 6,00 | erfindungsgemäße Dispersion |
| 1,00 | VP/Acrylates/Lauryl Methacrylate Copolymer (ISP: Styleze 2000) |
| 0,26 | Aminomethyl Propanol |
| 90,64 | Aqua dem |
| q.s. | Parfümöl |
| q.s. | PEG-40 Hydrogenated Castor Oil |
| 0,10 | Sorbitol |
| 0,10 | Konservierungsmittel |
| 2,00 | Hydroxypropyl Guar (Rhodia Inc., N-Hance Hydroxypropylguar) |

### Herstellung:

Alle Komponenten mischen bis sie homogen sind.

### Beispiel 49: Haargel

| | |
|---|---|
| A | |
| 0,50 | Carbomer |
| 90,01 | Aqua dem. |

| | |
|---|---|
| B | |
| 0,70 | Triethanolamine |

| | |
|---|---|
| C | |
| 6,00 | erfindungsgemäße Dispersion |
| 2,00 | Acrylates/C1-2 Succinates/Hydroxyacrylates Copolymer (Rohm&Haas, Allianz LT-120) |
| 0,19 | Aminomethyl Propanol |
| q.s. | Parfümöl |
| q.s. | PEG-40 Hydrogenated Castor Oil |
| 0,10 | PEG-8 |
| 0,10 | Konservierungsmittel |
| 0,50 | Hydroxyethylcellulose |

### Herstellung:

Phase A quellen lassen und mit Phase B neutralisieren. Phase C lösen und in Phase A+B einrühren.

### Beispiel 50: Haargel

| | |
|---|---|
| 7,00 | erfindungsgemäße Dispersion |
| 7,00 | Methacrylic Acid/Sodium Acrylamidomethyl Propane Sulfonate Copolymer (Ondeo Nalco, Fixomer A30) |
| 0,70 | Triethanolamine |
| q.s. | Parfümöl |
| q.s. | PEG-40 Hydrogenated Castor Oil |
| 0,10 | Panthenol |
| 0,10 | Konservierungsmittel |
| 84,90 | Aqua dem. |
| 1,00 | Polyacrylamide / C13-14 Isoparaffin / Laureth-7 (Seppic, Sepigel 305) |

### Herstellung:

Alle Komponenten mischen bis sie homogen sind.

### Beispiel 51: Haargel

| | |
|---|---|
| A | |
| 0,50 | Carbomer |
| 90,50 | Aqua dem. |

| | |
|---|---|
| B | |
| 0,70 | Triethanolamine |

| | |
|---|---|
| C | |
| 7,00 | erfindungsgemäße Dispersion |
| 1,00 | Polyvinylformamide |
| q.s. | Parfümöl |
| q.s. | PEG-40 Hydrogenated Castor Oil |
| 0,10 | Konservierungsmittel |
| 0,10 | Ethylhexyl Methoxycinnamate |
| 0,10 | PEG-14 Dimethicone |

### Herstellung:

Phase A quellen lassen und mit Phase B neutralisieren. Phase C lösen und in Phase A+B einrühren.

### Beispiel 52: Aquawax

| | |
|---|---|
| 10,00 | erfindsungsgemäßes Copolymer |
| q.s. | Parfümöl |
| q.s. | PEG-40 Hydrogenated Castor Oil |
| 0,10 | Diethyl Phtalate |
| 0,10 | Cetearyl Ethylhexanoate |
| 0,10 | PEG-7 Glyceryl Cocoate |
| 0,10 | Konservierungsmittel |
| 87,70 | Aqua dem. |
| 2,00 | Caprylic/Capric Triglyceryde, Acrylates Copolymer |

### Herstellung:

Alles mischen und homogenisieren. 15 Minuten nachrühren.

### Beispiel 53: Rinse-off Conditioner and Repair Treatment

| | |
|---|---|
| A | |
| 0,20 | CetearylOctanoate |
| 0,10 | Phytantriol |
| 2,00 | PEG-40 Hydrogenated Castor Oil |

| | |
|---|---|
| B | |
| q.s. | Parfümöl |
| 2,00 | Cocotrimonium Methosulfate |

| | |
|---|---|
| C | |
| 77,70 | Aqua dem. |

| | |
|---|---|
| D | |
| 2,00 | Polyquaternium-16 |
| 5,00 | erfindungsgemäße Dispersion |
| 1,00 | Dimethicone Copolyol |
| q.s. | Konservierungsmittel |
| 10,00 | Alcohol |
| q.s. | Citric Acid |

### Herstellung:

Die Phasen A und B getrennt mischen. Phase C in Phase B einrühren.

### Beispiel 54: Haarkur

| | |
|---|---|
| A | |
| 2,00 | Ceteareth-6, Stearyl Alcohol |
| 1,00 | Ceteareth-25 |
| 6,00 | Cetearyl Alcohol |
| 6,00 | Cetearyl Octanoate |
| 0,30 | Phytantriol |

| | |
|---|---|
| B | |
| 5,00 | erfindungsgemäße Dispersion |
| 0,70 | Guar Hydroxylpropytrimonium Chloride |
| 5,00 | Propylene Glycol |
| 2,00 | Panthenol |
| 0,30 | Imidazolidinyl Urea |
| 69,00 | Aqua dem. |

| | |
|---|---|
| C | |
| 2,00 | Cosi Silk Soluble |
| 0,20 | Perfume |
| 0,50 | Phenoxyethanol |

### Herstellung:

Die Phasen A und B getrennt auf ca. 80°C erwärmen. Phase B homogenisieren.

### Beispiel 55: Haar-Cocktail

| | |
|---|---|
| A | |
| 0,40 | Acrylates/C10-30 Alkyl Acrylate Crosspolymer |
| 2,00 | Dimethicone |
| 3,00 | Cyclomethicone, Dimethiconol |
| 2,00 | Phenyl Trimethicone |
| 2,00 | Amodimethicone, Cetrimonium Chloride, Trideceth-10 |
| 0,50 | Dimethicone Copolyol |
| 1,00 | Macadamia (Ternifolia) Nut Oil |
| 0,50 | Tocopheryl Acetate |
| 1,00 | PEG-40 Hydrogenated Castor Oil |
| q.s. | Parfümöl |

| | |
|---|---|
| B | |
| 82,84 | Aqua dem. |
| 0,30 | erfindungsgemäße Dispersion |
| 0,46 | Aminomethyl Propanol |
| 4,00 | PEG/PPG-25/25 Dimethicone/Acrylates Copolymer |

### Herstellung:

Die Komponenten der Phase A mischen. Phase B lösen. Phase B unter Homogenisieren in Phase A einrühren.

### Beispiel 56: Dauerwelle

Well-Lösung

| | |
|---|---|
| A | |
| 73,95 | Aqua dem. |
| 0,20 | Cocamidopropyl Betaine |
| 0,20 | Polysorbate 20 |
| 1,25 | erfindungsgemäße Dispersion |
| 0,20 | Disodium EDTA |
| 0,20 | Hydroxyethylcellulose |

| | |
|---|---|
| B | |
| 8,00 | Thioglycolic Acid |

| | |
|---|---|
| C | |
| 11,00 | Ammonium Hydroxide |

| | |
|---|---|
| D | |
| 5,00 | Ammonium Carbonate |

### Herstellung:

Die Komponenten der Phase A einwiegen und klar lösen. Phase B in Phase A einrühren.

### Fixierung:

| | |
|---|---|
| A | |
| 1,00 | PEG-40 Hydrogenated Castor Oil |
| 0,20 | Parfümöl |
| 93,60 | Aqua dem. |

| | |
|---|---|
| B | |
| 0,20 | Cocamidopropyl Betaine |
| 0,20 | Ceteareth-25 |
| 2,50 | erfindungsgemäße Dispersion |
| q.s. | Konservierungsmittel |

| | |
|---|---|
| C | |
| 2,30 | Hydrogen Peroxid |

| | |
|---|---|
| D | |
| q.s. | Phosphoric Acid |

### Herstellung:

Phase A solubilisieren. Die Komponenten der Phase B nacheinander zugeben und klar lösen.

### Beispiel 57: dunkelbraune Permanenthaarfarbe (Oxidationshaarfarbe)

| | |
|---|---|
| A | |
| 50,90 | Aqua dem. |
| 0,20 | Sodium Sulfite |
| 0,05 | Disodium EDTA |
| 0,20 | p-Phenylenediamine |
| 0,30 | Resorcinol |
| 0,20 | 4-Amino-2-hydroxytoluene |
| 0,10 | m-Aminophenol |
| 1,50 | Oleyl Alcohol |
| 4,50 | Propyleneglycol |
| 2,30 | Sodium C12-15 Pareth-15 Sulfonate |
| 20,00 | Oleic Acid |

| | |
|---|---|
| B | |
| 1,00 | erfindungsgemäße Dispersion |
| 13,70 | Ammonium Hydroxide |
| 6,00 | i-Propanol |
| q.s. | Perfume |

### Herstellung:

Phase A solubilisieren. Die Komponenten der Phase B nacheinander zugeben und mischen.

| | |
|---|---|
| Entwickleremulsion (pH: 3-4) | |
| 3,00 | Hexadecylalcohol |
| 2,00 | erfindungsgemäße Dispersion |
| 1,00 | Ceteareth-20 |
| 1,00 | Sodium C12-15 Pareth-15 Sulfonate |
| 6,00 | Hydrogen Peroxide |
| 0,50 | Phosphoric Acid |
| 0,01 | Acetanilid |
| 86,49 | Aqua dem. |

### Herstellung:

Die Komponenten nacheinander zugeben und mischen.

### Beispiel 58: hellbraune Semi-Permanethaarfarbe

| | |
|---|---|
| 10,00 | Cocodiethanolamide |
| 4,00 | Natriumdodecyl-benzoylsulfonat, 50%ig |
| 1,00 | erfindungsgemäße Dispersion |
| 6,00 | C9-11 Pareth-3 |
| 2,50 | Sodium Lauryl Sulfate |
| 0,4 | 2-Nitro-p-Phenylendiamine |
| 0,20 | HC Red No.3 |
| 0,20 | HC Yellow No.2 |
| 75,70 | Aqua dem. |

### Herstellung:

Die Komponenten nacheinander zugeben und mischen.

### Beispiel 59: Klares Conditioning Shampoo

| | |
|---|---|
| A | |
| 15,00 | Cocamidopropyl Betaine |
| 10,00 | Disodium Cocoamphodiacetate |
| 5,00 | Polysorbate 20 |
| 5,00 | Decyl Glucoside |
| q.s. | Perfume |
| q.s. | Preservative |
| 0,1-1,0 | erfindungsgemäße Dispersion |
| 2,00 | Laureth-3 |
| add 100 | Aqua dem. |
| q.s. | Citric Acid |

| | |
|---|---|
| B | |
| 3,00 | PEG-150 Distearate |

### Herstellung:

Komponenten der Phase A einwiegen und lösen. pH-Wert auf 6-7 einstellen. Phase B zugeben und auf 50°C erwärmen. Auf Raumtemperatur abkühlen lassen unter Rühren.

### Beispiel 60: Shampoo

| | |
|---|---|
| 30,00 | Sodium Laureth Sulfate |
| 6,00 | Sodium Cocoamphoacetate |
| 6,00 | Cocamidopropyl Betaine |
| 3,00 | Sodium Laureth Sulfate, Glycol |

Distearate, Cocamide MEA, Laureth-10

| | |
|---|---|
| 0,1-1,0 | erfindungsgemäße Dispersion |
| 2,00 | Dimethicone |
| q.s. | Perfume |
| q.s. | Preservative |
| q.s. | Citric Acid |
| 1,00 | Sodium Chloride |
| add 100 | Aqua dem. |

### Herstellung:

Komponenten einwiegen und lösen. pH-Wert auf 6-7 einstellen.

### Beispiel 61: Shampoo

| | |
|---|---|
| 30,00 | Sodium Laureth Sulfate |
| 6,00 | Sodium Cocoamphoacetate |
| 6,00 | Cocamidopropyl Betaine |
| 3,00 | Sodium Laureth Sulfate, Glycol Distearate, Cocamide MEA, Laureth-10 |
| 0,1-1,0 | erfindungsgemäße Dispersion |
| 2,00 | Amodimethicone |
| q.s. | Perfume |
| q.s. | Preservative |
| q.s. | Citric Acid |
| 1,00 | Sodium Chloride |
| add 100 | Aqua dem. |

### Herstellung:

Komponenten einwiegen und lösen. pH-Wert auf 6-7 einstellen.

### Beispiel 62: Shampoo

| | |
|---|---|
| 40,00 | Sodium Laureth Sulfate |
| 10,00 | Cocamidopropyl Betaine |
| 3,00 | Sodium Laureth Sulfate, Glycol Distearate, Cocamide MEA, Laureth-10 |
| 0,1-1,0 | erfindungsgemäße Dispersion |
| 2,00 | Dow Corning 3052 |
| q.s. | Perfume |
| q.s. | Preservative |
| q.s. | Citric Acid |
| 2,00 | Cocamido DEA |
| add 100 | Aqua dem. |

### Herstellung:

Komponenten einwiegen und lösen. pH-Wert auf 6-7 einstellen.

### Beispiel 63: Antischuppen-Shampoo

| | |
|---|---|
| 40,00 | Sodium Laureth Sulfate |
| 10,00 | Cocamidopropyl Betaine |
| 10,00 | Disodium Laureth Sulfosuccinate |
| 2,50 | Sodium Laureth Sulfate, Glycol Distearate,Cocamide MEA, Laureth-10 |
| 0,1-1,0 | erfindungsgemäße Dispersion |
| 0,50 | Climbazole |
| q.s. | Perfume |
| q.s. | Preservative |
| 0,50 | Sodium Chloride |
| add 100 | Aqua dem. |

### Herstellung:

Komponenten einwiegen und lösen. pH-Wert auf 6-7 einstellen.

### Beispiel 64: Shampoo

| | |
|---|---|
| 25,00 | Sodium Laureth Sulfate |
| 5,00 | Cocamidopropyl Betaine |
| 2,50 | Sodium Laureth Sulfate, Glycol Distearate,Cocamide MEA, Laureth-10 |
| 0,1-1,0 | erfindungsgemäße Dispersion |
| q.s. | Perfume |
| q.s. | Preservative |
| 2,00 | Cocamido DEA |
| add 100 | Aqua dem. |

### Herstellung:

Komponenten einwiegen und lösen. pH-Wert auf 6-7 einstellen.

### Beispiel 65: Shampoo

| | |
|---|---|
| 20,00 | Ammonium Laureth Sulfate |
| 15,00 | Ammonium Lauryl Sulfate |
| 5,00 | Cocamidopropyl Betaine |
| 2,50 | Sodium Laureth Sulfate, Glycol Distearate,Cocamide MEA, Laureth-10 |
| 0,1-1,0 | erfindungsgemäße Dispersion |
| q.s. | Perfume |
| q.s. | Preservative |
| 0,50 | Sodium Chloride |
| add 100 | Aqua dem. |

### Herstellung:

Komponenten einwiegen und lösen. pH-Wert auf 6-7 einstellen.

### Beispiel 66: Klares Duschgel

| | |
|---|---|
| 40,00 | Sodium Laureth Sulfate |
| 5,00 | Decyl Glucoside |
| 5,00 | Cocamidopropyl Betaine |
| 0,1-1,0 | erfindungsgemäße Dispersion |
| 1,00 | Panthenol |
| q.s. | Perfume |
| q.s. | Preservative |
| q.s. | Citric Acid |
| 2,00 | Sodium Chloride |
| add 100 | Aqua dem. |

### Herstellung:

Komponenten einwiegen und lösen. pH-Wert auf 6-7 einstellen.

### Beispiel 67: Shampoo

| | |
|---|---|
| 12,00 | Sodium Laureth Sulfate |
| 1,50 | Decyl Glucoside |
| 2,50 | Cocamidopropyl Betaine |
| 5,00 | Coco-Glucoside Glyceryl Oleate |
| 2,00 | Sodium Laureth Sulfate, Glycol Distearate,Cocamide MEA,Laureth-10 |
| 0,1-1,0 | erfindungsgemäße Dispersion |
| q.s. | Preservative |
| q.s. | Sunset Yellow C. I. 15 985 |
| q.s. | Perfume |
| 1,00 | Sodium Chloride |
| add 100 | Aqua dem. |

### Herstellung:

Komponenten einwiegen und lösen. pH-Wert auf 6-7 einstellen.

### Beispiel 68: Shampoo

| | |
|---|---|
| A | |
| 40,00 | Sodium Laureth Sulfate |
| 5,00 | Sodium C12-15 Pareth-15 Sulfonate |
| 5,00 | Decyl Glucoside |
| q.s. | Perfume |
| 0,10 | Phytantriol |

| | |
|---|---|
| B | |
| add 100 | Aqua dem. |
| 0,1-1,0 | erfindungsgemäße Dispersion |
| 1,00 | Panthenol |
| q.s. | Preservative |
| 1,00 | Laureth-3 |
| q.s. | Citric Acid |
| 2,00 | Sodium Chloride |

### Herstellung:

Komponenten der Phase A einwiegen und lösen. pH-Wert auf 6-7 einstellen. Phase B zugeben und mischen.

## Patentansprüche

1. Wässrige Dispersion erhältlich durch radikalische Polymerisation von
a) mindestens einem N-Vinylhaltigen Monomer und/oder mindestens einem (Meth)acrylamidmonomer
b) mindestens einem polymeren Dispergiermittel
c) mindestens einem polymeren Fällungsagens
d) mindestens einem Vernetzer
e) gegebenenfalls weiteren Monomeren
f) gegebenenfalls mindestens einem Regler
g) gegebenenfalls einer Puffersubstanz
wobei das Gewichtsverhältnis von b) zu c) im Bereich von 1:50 bis 1:0,02 liegt.

2. Dispersion nach Anspruch 1, **dadurch gekennzeichnet, dass** das Gewichtsverhältnis von b) zu c) im Bereich von 1:20 bis 1: 0,05 liegt.

3. Dispersion nach den Ansprüchen 1 oder 2, **dadurch gekennzeichnet, dass** als Monomer a)
N-Vinylamide und/oder
N-Vinyllactame und/oder
(Meth)acrylamidmonomere ausgewählt aus der Gruppe bestehend aus Acrylamid,
2-Acrylamidoglykolsäure,
N-(Tris(hydroxymethyl)-methyl)acrylamid,
N-Hydroxymethylacrylamid,
N-Methylacrylamid,
N-Isopropylacrylamid,
2-Acrylamido-2-methyl-1-propansulfonsäure
Methacrylamid,
**N-Ethyl-methacrylamid,**
N-Hydroxymethyl-methacrylamid,
N-(2-hydroxypropyl)-methacrylamid,
N-Methyl-methacrylamid,
N-Isobutoxy-methylacrylamid,
N-Methoxymethylmethacrylamid
eingesetzt werden.

4. Dispersion nach den Ansprüchen 1 bis 3, **dadurch gekennzeichnet, dass** Monomer a) ausgewählt ist aus der Gruppe bestehend aus Acrylamid, Methacrylamid, N-Hydroxymethylacrylamid, N-(2-hydroxypropyl)-methacrylamid, N-Hydroxymethylmethacrylamid, N-Isopropylacrylamid.

5. Dispersion nach einem der Ansprüche 1 bis 4, **dadurch gekennzeichnet, dass** das polymere Dispergiermittel b) ausgewählt ist aus der Gruppe bestehend aus Polyvinylacetat, Polyalkylenglykolen, insbesondere Polyethylenglykole, Polyvinylalkohol, Polyvinylpyridin, Polyethylenimin, Polyvinylimidazol, Polyvinylsuccinimid und Polydiallyldimethylammoniumchlorid, Polyvinylpyrrolidon, Polymerisaten, die mindestens 5 Gew.% an Vinylpyrrolidon-Einheiten enthalten, Polymerisaten, die mindestens 50 Gew.% an Vinylalkohol-Einheiten enthalten, Oligosacchariden, Polysacchariden, oxidativ, hydrolytisch oder enzymatisch abgebauten Polysacchariden, chemisch modifizierten Oligo- oder Polysacchariden wie insbesondere Carboxymethylcellulose, wasserlösliche Stärke und Stärkederivate, Stärkeester, Stärkexanthanogenate, Stärkeacetate, Dextran, und deren Mischungen.

6. Dispersion nach einem der Ansprüche 1 bis 5, **dadurch gekennzeichnet, dass** als polymeres Dispergiermittel b) Polymerisate, die mindestens 5 Gew.% an Vinylpyrrolidon-Einheiten enthalten und/oder Polyvinylpyrrolidon eingesetzt werden.

7. Dispersion nach einem der Ansprüche 1 bis 6, **dadurch gekennzeichnet, dass** als polymeres Fällungsagens c) eine wasserlösliche polyetherhaltige Verbindung eingesetzt wird.

8. Dispersion nach einem der Ansprüche 1 bis 7, **dadurch gekennzeichnet, dass** als polymeres Fällungsagens c) eine wasserlösliche polyetherhaltige Verbindung der folgenden Formel (Ib) eingesetzt wird: 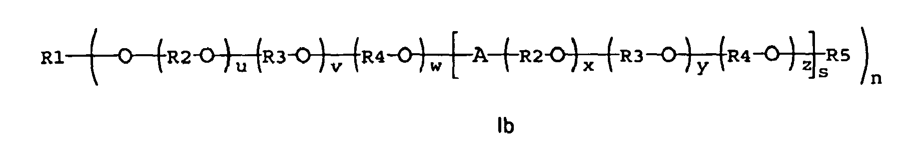 in der die Variablen unabhängig voneinander folgende Bedeutung haben:
R¹ Wasserstoff, C₁-C₂₄-Alkyl, R⁶-C(=O)-, R⁶-NH-C(=O)-, Polyalkoholrest;
R⁵ Wasserstoff, C₁-C₂₄-Alkyl, R⁶-C(=O)-, R⁶-NH-C(=O)-;
R² bis R⁴ -(CH₂)₂-, -(CH₂)₃-, -(CH₂)₄-, -CH₂-CH(R⁶)-, -CH₂-CHOR⁷-CH₂-;
R⁶ C₁-C₂₄-Alkyl;
R⁷ Wasserstoff, C₁-C₂₄-Alkyl, R⁶-C(=O)-, R⁶-NH-C(=O)-;
A -C(=O)-O, -C(=O)-B-C(=O)-O, -CH₂-CH(-OH)-B-CH(-OH)-CH₂-O, -C(=O)-NH-B-NH-C(=O)-O; 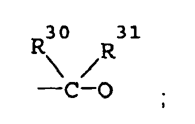
B -(CH₂)ₜ-, Arylen, ggf. substituiert;
R³⁰, R³¹ Wasserstoff, C₁-C₂₄-Alkyl, C₁-C₂₄-Hydroxyalkyl, Benzyl oder Phenyl;
n 1 wenn R¹ kein Polyakoholrest ist oder
n 1 bis 1000 wenn R¹ ein Polyalkoholrest ist
s = 0 bis 1000; t = 1 bis 12; u =1 bis 5000;v = 0 bis 5000; w =0 bis 5000;x= 0 bis 5000;
y= 0 bis 5000; z= 0 bis 5000.

9. Dispersion nach einem der Ansprüche 1 bis 8, **dadurch gekennzeichnet, dass** als polymeres Fällungsagens c) Polyalkylenglykole eingesetzt werden.

10. Dispersion der Ansprüche 1 bis 9, **dadurch gekennzeichnet, dass** als polymeres Fällungsagens c) Polyethylenglykol (PEG) eingesetzt wird.

11. Dispersion nach einem der Ansprüche 1 bis 10, **dadurch gekennzeichnet, dass** als polymeres Fällungsagens c) eine Verbindung mit einem Molekulargewicht von 300 bis 100 000, bevorzugt 1000 bis 30 000, insbesondere 1000 bis 10 000, eingesetzt wird.

12. Dispersion nach einem der Ansprüche 1 bis 11, **dadurch gekennzeichnet, dass** das Gewichtsverhältnis der Summe von b) und c) zur Summe der restlichen Monomeren im Bereich von 1 : 10 bis 1 : 0,1 liegt.

13. Dispersion nach einem der Ansprüche 1 bis 12, **dadurch gekennzeichnet, dass** als weiteres Monomer e) ein kationisches und/oder ein quaternisierbares Monomer eingesetzt wird.

14. Dispersion nach Anspruch 13, **dadurch gekennzeichnet, dass** als weiteres Monomer e) ein Diallylamin der allgemeinen Formel (II) eingesetzt wird, worin R⁴ für C₁-C₂₄-Alkyl steht

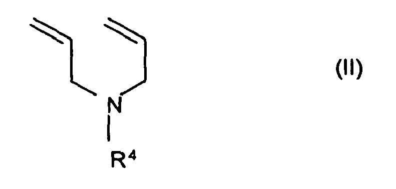

15. Dispersion nach Anspruch 13, **dadurch gekennzeichnet, dass** als weiteres Monomer e) ein N-Vinylimidazolderivat der allgemeinen Formel (I) eingesetzt wird, worin R¹ bis R³ für Wasserstoff, C₁-C₄-Alkyl oder Phenyl steht.

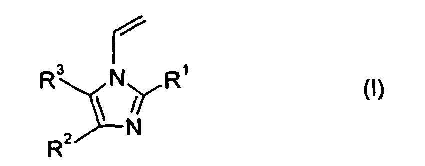

16. Dispersion nach einem der Ansprüche 1 bis 15, **dadurch gekennzeichnet, dass** als polymeres Dispergiermittel b) Polymerisate, die mindestens 5 Gew.% an Vinylpyrrolidon-Einheiten enthalten und/oder Polyvinylpyrrolidon eingesetzt werden und als Fällungsagens c) Polyethylenglykol eingesetzt wird.

17. Wässrige Lösung, erhältlich durch Verdünnen der Dispersion nach einem der Ansprüche 1 bis 16 mit Wasser.

18. Verfahren zur Herstellung von wässrigen Dispersionen, wobei
a) mindestens ein N-Vinylhaltiges Monomer und/oder mindestens ein (Meth)acrylamidmonomer
b) mindestens ein polymeres Dispergiermittel
**c) mindestens ein Fällungsagens**
d) mindestens ein Vernetzer
e) gegebenenfalls weitere Monomere
g) gegebenenfalls einer Puffersubstanz
in Gegenwart mindestens eines Reglers f) umgesetzt werden und das Gewichtsverhältnis von b) zu c) im Bereich von 1:50 bis 1:0,02 liegt.

19. Verfahren nach Anspruch 18, **dadurch gekennzeichnet, dass** als Regler f) ein multifunktionaler Regler eingesetzt wird.

20. Verfahren nach Anspruch 18, **dadurch gekennzeichnet, dass** man die erhaltene Dispersion einer Hydrolyse unterzieht.

21. Verfahren nach Anspruch 20, **dadurch gekennzeichnet, dass** man die Hydrolyse bis zu einem Gehalt von Aminen im Polymerisat < 20 mol % bezogen auf die Komponente (a) durchführt.

## Claims

1. An aqueous dispersion obtainable by free-radical polymerization of
a) at least one N-vinyl-containing monomer and/or at least one (meth)acrylamide monomer
b) at least one polymeric dispersant
c) at least one polymeric precipitation agent
d) at least one crosslinker
e) if appropriate further monomers
f) if appropriate at least one regulator
g) if appropriate a buffer substance
where the weight ratio of b) to c) is in the range from 1:50 to 1:0.02.

2. The dispersion according to claim 1, wherein the weight ratio of b) to c) is in the range from 1:20 to 1:0.05.

3. The dispersion according to claim 1 or 2, wherein
N-vinylamides and/or
N-vinyllactams and/or
(meth)acrylamide monomers chosen from the group consisting of acrylamide,
2-acrylamidoglycolic acid,
N-(tris(hydroxymethyl)methyl)acrylamide,
N-hydroxymethylacrylamide,
N-methylacrylamide,
N-isopropylacrylamide,
2-acrylamido-2-methyl-1-propanesulfonic acid methacrylamide,
N-ethyl-methacrylamide,
N-hydroxymethylmethacrylamide,
N-(2-hydroxypropyl)methacrylamide,
N-methylmethacrylamide,
N-isobutoxymethylacrylamide,
N-methoxymethylmethacrylamide
are used as monomer a).

4. The dispersion according to claims 1 to 3, wherein monomer a) is chosen from the group consisting of acrylamide, methacrylamide, N-hydroxymethylacrylamide, N-(2-hydroxypropyl)methacrylamide, N-hydroxymethylmethacrylamide, N-isopropylacrylamide.

5. The dispersion according to any of claims 1 to 4, wherein the polymeric dispersant b) is chosen from the group consisting of polyvinyl acetate, polyalkylene glycols, in particular polyethylene glycols, polyvinyl alcohol, polyvinylpyridine, polyethyleneimine, polyvinylimidazole, polyvinylsuccinimide and polydiallyldimethylammonium chloride, polyvinylpyrrolidone, polymers which comprise at least 5% by weight of vinylpyrrolidone units, polymers which comprise at least 50% by weight of vinyl alcohol units, oligosaccharides, polysaccharides, oxidatively, hydrolytically or enzymatically degraded polysaccharides, chemically modified oligo- or polysaccharides, such as, in particular, carboxymethylcellulose, water-soluble starch and starch derivatives, starch esters, starch xanthanogenates, starch acetates, dextran, and mixtures thereof.

6. The dispersion according to any of claims 1 to 5, wherein polymers which comprise at least 5% by weight of vinylpyrrolidone units and/or polyvinylpyrrolidone are used as polymeric dispersant b) .

7. The dispersion according to any of claims 1 to 6, wherein a water-soluble polyether-containing compound is used as polymeric precipitation agent c).

8. The dispersion according to any of claims 1 to 7, wherein a water-soluble polyether-containing compound of the following formula (Ib) is used as polymeric precipitation agent c): 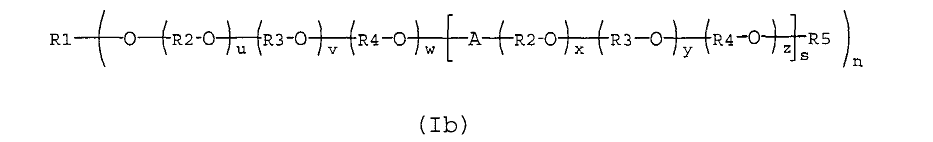 in which the variables, independently of one another, have the following meanings:
R¹ is hydrogen, C₁-C₂₄-alkyl, R⁶-C (=O) -, R⁶⁻NH-C(=O)-, polyalcohol radical;
R⁵ is hydrogen, C₁-C₂₄-alkyl, R⁶-C (=O) -, R⁶⁻NH-C (=O) -;
R² to R⁴ are - (CH₂)₂-, - (CH₂)₃-, - (CH₂)₄-, -CH₂₋CH(R⁶) -, -CH₂-CHOR⁷-CH₂-;
R⁶ is C₁-C₂₄-alkyl;
R⁷ is hydrogen, C₁-C₂₄-alkyl, R⁶-C (=O) -, R⁶⁻NH-C (=O) -;
A is -C (=O) -O, -C(=0)-B-C(=0)-0, -CH₂-CH(-OH) -B-CH(-OH) -CH₂-O, -C(=O)-NH-B-NH-C(=O)-O; 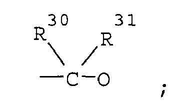
B is -(CH₂)ₜ-, arylene, optionally substituted;
R³⁰, R³¹ are hydrogen, C₁-C₂₄-alkyl, C₁-C₂₄₋hydroxyalkyl, benzyl or phenyl;
n is 1 when R¹ is not a polyalcohol radical or
n is 1 to 1000 when R¹ is a polyalcohol radical
s = 0 to 1000; t = 1 to 12; u =1 to 5000; v = 0 to 5000; w =0 to 5000; x = 0 to 5000;
y = 0 to 5000; z = 0 to 5000.

9. The dispersion according to any of claims 1 to 8,
wherein polyalkylene glycols are used as polymeric precipitation agent c).

10. The dispersion of claims 1 to 9, wherein polyethylene glycol (PEG) is used as polymeric precipitation agent c).

11. The dispersion according to any of claims 1 to 10, wherein a compound with a molecular weight of from 300 to 100 000, preferably 1000 to 30 000, in particular 1000 to 10 000, is used as polymeric precipitation agent c).

12. The dispersion according to any of claims 1 to 11,
wherein the weight ratio of the sum of b) and c) to the sum of the remaining monomers is in the range from 1 : 10 to 1 : 0.1.

13. The dispersion according to any of claims 1 to 12,
wherein a cationic and/or a quaternizable monomer is used as further monomer e).

14. The dispersion according to claim 13, wherein a diallylamine of the formula (II), in which R⁴ is C₁₋C₂₄-alkyl is used as further monomer e)

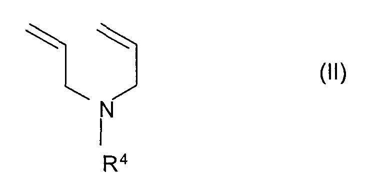

15. The dispersion according to claim 13, wherein an N-vinylimidazole derivative of the formula (I) in which R¹ to R³ is hydrogen, C₁-C₄-alkyl or phenyl is used as further monomer e).

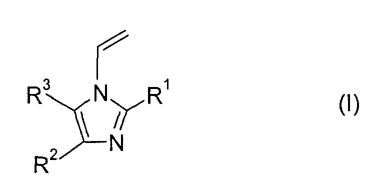

16. The dispersion according to any of claims 1 to 15, wherein polymers which comprise at least 5% by weight of vinylpyrrolidone units and/or polyvinylpyrrolidone are used as polymeric dispersant b), and polyethylene glycol is used as precipitation agent c).

17. An aqueous solution obtainable by diluting the dispersion according to any of claims 1 to 16 with water.

18. A process for the preparation of aqueous dispersions where
a) at least one N-vinyl-containing monomer and/or at least one (meth)acrylamide monomer
b) at least one polymeric dispersant
c) at least one precipitation agent
d) at least one crosslinker
e) if appropriate further monomers
g) if appropriate a buffer substance
are reacted in the presence of at least one regulator f) and the weight ratio of b) to c) is in the range from 1:50 to 1:0.02.

19. The process according to claim 18, wherein a multifunctional regulator is used as regulator f).

20. The process according to claim 18, wherein the resulting dispersion is subjected to hydrolysis.

21. The process according to claim 20, wherein the hydrolysis is carried out up to a content of amines in the polymer of < 20 mol%, based on component (a) .

## Revendications

1. Dispersion aqueuse que l'on peut obtenir par polymérisation radicalaire de :
a) au moins un monomère contenant du N-vinyle et/ou au moins un monomère de (méth) acrylamide,
b) au moins un agent dispersant polymère,
c) au moins un agent de précipitation polymère,
d) au moins un agent de réticulation,
e) éventuellement d'autres monomères,
f) éventuellement au moins un régulateur,
g) éventuellement une substance tampon,
dans laquelle le rapport pondéral de b) à c) se situe dans la plage de 1:50 à 1:0,02.

2. Dispersion selon la revendication 1, **caractérisée en ce que** le rapport pondéral de b) à c) se situe dans la plage de 1:20 à 1:0,05.

3. Dispersion selon la revendication 1 ou 2,
**caractérisée en ce que** l'on utilise comme monomère a) les substances suivantes :
des N-vinylamides et/ou
des N-vinyllactames et/ou
des monomères de (méth)acrylamide choisis dans le groupe constitué des substances suivantes :
l'acrylamide,
l'acide 2-acrylamidoglycolique,
le N-(tris(hydroxyméthyl)méthyl)acrylamide,
le N-hydroxyméthylacrylamide,
le N-méthylacrylamide,
le N-isopropylacrylamide,
l'acide 2-acrylamido-2-méthyl-1-propanesulfonique
le méthacrylamide,
le N-éthyl-méthacrylamide,
le N-hydroxyméthyl-méthacrylamide,
le N-(2-hydroxypropyl)-méthacrylamide,
le N-méthyl-méthacrylamide,
le N-isobutoxy-méthylacrylamide, et
le N-méthoxyméthylméthacrylamide.

4. Dispersion selon les revendications 1 à 3, **caractérisée en ce que** le monomère a) est choisi dans le groupe constitué de l'acrylamide, du méthacrylamide, du N-hydroxyméthylacrylamide, du N-(2-hydroxypropyl)-méthacrylamide, du N-hydroxyméthylméthacrylamide et du N-isopropylacrylamide.

5. Dispersion selon l'une quelconque des revendications 1 à 4, **caractérisée en ce que** l'agent dispersant polymère b) est choisi dans le groupe constitué du poly(acétate de vinyle), des polyalkylèneglycols, en particulier les polyéthylèneglycols, de l'alcool polyvinylique, de la poly(vinylpyridine), de la poly(éthylèneimine), du poly(vinylimidazole), du poly(vinylsuccinimide) et du poly(chlorure de diallyldiméthylammonium), de la polyvinylpyrrolidone, de polymères qui contiennent au moins 5% en poids d'unités vinylpyrrolidone, de polymères qui contiennent au moins 50% en poids d'unités alcool vinylique, des oligosaccharides, des polysaccharides, des polysaccharides dégradés par voie oxydante, hydrolytique ou enzymatique, des oligosaccharides ou des polysaccharides modifiés chimiquement, tout particulièrement la carboxyméthylcellulose, des amidons hydrosolubles et des dérivés d'amidon, des esters d'amidon, des xanthogénates d'amidon, des acétates d'amidon, du dextrane et de leurs mélanges.

6. Dispersion selon l'une quelconque des revendications 1 à 5, **caractérisée en ce que** l'on utilise comme agent dispersant polymère b) des polymères qui contiennent au moins 5% en poids d'unités vinylpyrrolidone et/ou de la poly(vinylpyrrolidone).

7. Dispersion selon l'une quelconque des revendications 1 à 6, **caractérisée en ce que** l'on utilise comme agent de précipitation polymère c) un composé hydrosoluble contenant des polyéthers.

8. Dispersion selon l'une quelconque des revendications 1 à 7, **caractérisée en ce que** l'on utilise comme agent de précipitation polymère c) un composé hydrosoluble contenant des polyéthers de formule (Ib) suivante : 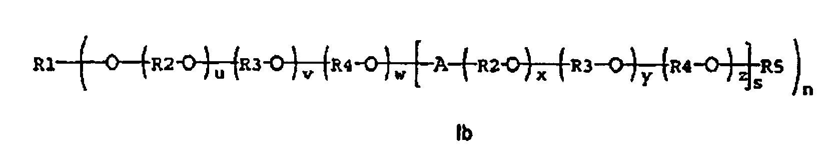 dans laquelle les variables ont, indépendamment les unes des autres, les significations suivantes :
R¹ représente de l'hydrogène, un alkyle en C₁-C₂₄, R⁶-C (=O) -, R⁶-NH-C(=O)-, un radical polyol;
R⁵ représente de l'hydrogène, un alkyle en C₁-C₂₄, R⁶-C(=O)-, R⁶-NH-C(=O)-;
R² à R⁴ représentent -(CH₂)_{**2**}-, -(CH₂)₃-, -(CH₂)₄-, -CH₂-CH(CH(R⁶)-, -CH₂-CHOR⁷ -CH₂-;
R⁶ représente un alkyle en C₁-C₂₄;
R⁷ représente de l'hydrogène, un alkyle en C₁-C₂₄, R⁶-C(=O)-, R⁶-NH-C(=O)-;
A représente -C(=O)-O, -C(=O)-B-C(=O)-O, -CH₂-CH(-OH)-B-CH(-OH)-CH₂-O,-C(=O)-NH-B-NH-C(=O)-O; 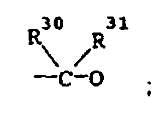
B représente -(CH₂)ₜ-, un arylène, éventuellement substitué;
R³⁰, R³¹ représentent de l'hydrogène, un alkyle en C₁-C₂₄, un hydroxyalkyle en C₁-C₂₄, un benzyle ou un phényle;
n est 1 lorsque R¹ n'est pas un radical polyol ou
n vaut 1 à 1 000 lorsque R¹ est un radical polyol;
s = 0 à 1 000; t = 1 à 12; u = 1 à 5 000; v = 0 à 5 000; w = 0 à 5 000; x = 0 à 5 000;
y = 0 à 5 000; z = 0 à 5 000.

9. Dispersion selon l'une quelconque des revendications 1 à 8, **caractérisée en ce que** l'on utilise des poly(alkylèneglycols) comme agent de précipitation polymère c).

10. Dispersion selon les revendications 1 à 9, **caractérisée en ce que** l'on utilise du poly(éthylèneglycol) (PEG) comme agent de précipitation polymère c).

11. Dispersion selon l'une quelconque des revendications 1 à 10, **caractérisée en ce que** l'on utilise comme agent de précipitation polymère c) un composé ayant un poids moléculaire de 300 à 100 000, de préférence, de 1 000 à 30 000, en particulier, de 1 000 à 10 000.

12. Dispersion selon l'une quelconque des revendications 1 à 11, **caractérisée en ce que** le rapport pondéral de la somme de b) et c) à la somme des monomères restants se situe dans la plage de 1:10 à 1:0,1.

13. Dispersion selon l'une quelconque des revendications 1 à 12, **caractérisée en ce que** l'on utilise comme autre monomère e) un monomère cationique et/ou un monomère quaternisable.

14. Dispersion selon la revendication 13, **caractérisée en ce que** l'on utilise comme autre monomère e) une diallylamine de formule générale (II) : 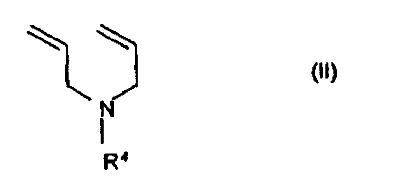 dans laquelle R⁴ représente un alkyle en C₁-C₂₄.

15. Dispersion selon la revendication 13, **caractérisée en ce que** l'on utilise comme autre monomère e) un dérivé de N-vinylimidazole de formule générale (I) : 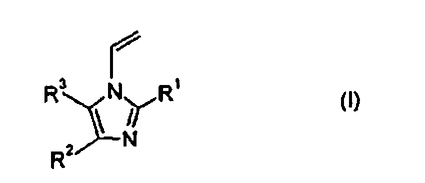 dans laquelle R¹ à R³ représentent de l'hydrogène, un alkyle en C₁-C₄ ou un phényle.

16. Dispersion selon l'une quelconque des revendications 1 à 15, **caractérisée en ce que** l'on utilise comme agent de dispersion polymère b) des polymères qui contiennent au moins 5% en poids d'unités vinylpyrrolidone et/ou de la polyvinylpyrrolidone et comme agent de précipitation c) du poly(éthylèneglycol).

17. Solution aqueuse que l'on peut obtenir par dilution de la dispersion selon l'une quelconque des revendications 1 à 16 avec de l'eau.

18. Procédé de fabrication de dispersions aqueuses, dans lequel on fait réagir :
a) au moins un monomère contenant du N-vinyle et/ou au moins un monomère de (méth)acrylamide,
b) au moins un agent de dispersion polymère,
c) au moins un agent de précipitation,
d) au moins un agent de réticulation,
e) éventuellement d'autres monomères,
g) éventuellement une substance tampon,
en présence d'au moins un régulateur f) et le rapport pondéral de b) à c) se situe dans la plage de 1:50 à 1:0,02.

19. Procédé selon la revendication 18, **caractérisé en ce que** l'on utilise un régulateur multifonctionnel comme régulateur f).

20. Procédé selon la revendication 18, **caractérisé en ce que** l'on soumet la dispersion obtenue à une hydrolyse.

21. Procédé selon la revendication 20, **caractérisé en ce que** l'on effectue l'hydrolyse jusqu'à une teneur en amines dans le polymère < 20% en mole par rapport au composant (a).
